# EUROPEAN PATENT SPECIFICATION

(11) **EP 4 616 840 B1**
(45) Date of publication and mention of the grant of the patent: **09.12.2026**
(21) Application number: 25191960.1
(22) Date of filing: 09.12.2021
(51) Int. Cl.: A61Q 19/00, A61Q 15/00, A61Q 5/00, A61K 8/34, A61K 8/35

(54) **COMPOSITIONS COMPRISING (BIO)-ALKANEDIOLS WITH ANTIMICROBIALS FOR PRODUCT PROTECTION**
ZUSAMMENSETZUNGEN MIT (BIO)-ALKANDIOLEN MIT ANTIMIKROBIELLEN MITTELN ZUM PRODUKTSCHUTZ
COMPOSITIONS COMPRENANT DES (BIO)-ALCANEDIOLS ET DES AGENTS ANTIMICROBIENS POUR LA PROTECTION DE PRODUITS

(30) Priority: 09.12.2020 WO PCT/EP2020/085171
(43) Date of publication of application: 17.09.2025
(62) Document number(s) of the earlier application(s) in accordance with Art. 76 EPC: 21830679.3 / 4 103 288
(73) Proprietor: Symrise AG, 37603 Holzminden (DE)
(72) Inventor: LANGE, Sabine, 37603 Holzminden (DE); BUGDAHN, Nikolas, 37603 Holzminden (DE); KOCH, Christin, 32760 Detmold (DE)
(74) Representative: Global IP Europe Patentanwaltskanzlei

(56) References cited:
- EP-B1- 2 774 481
- WO-A1-2020/043269
- WO-A1-2020/094244
- WO-A1-2020/160905
- US-A1- 2011 218 250
- US-A1- 2016 374 352

## Description

### Technical field

The present invention refers to a composition comprising or consisting of a synergistic combination of an effective amount of 2,3-octanediol and an effective amount of 4-hydroxyacetophenone. In addition, the present invention refers to a composition comprising or consisting of a synergistic combination of an effective amount of a mixture comprising 1,2-octanediol and 2,3-octanediol and an effective amount of 4-hydroxyacetophenone. Furthermore, the present invention relates to food, cosmetic or pharmaceutical preparations or homecare products comprising said composition. Additionally, the present invention refers to the use of said composition for suppressing or inhibiting microorganism growth in food, a cosmetic or a pharmaceutical preparation or homecare product. Additionally, the present invention relates to the use of 2,3-octanediol or a mixture comprising 1,2-octanediol and 2,3-octanediol in an effective amount for boosting the efficacy of 4-hydroxyacetophenone. Finally, the present invention relates to a method for suppressing or inhibiting of microorganism growth in food, a cosmetic or a pharmaceutical preparation or homecare product.

### Background Art

Food, cosmetics or pharmaceuticals or homecare products provide an optimal medium for microbial contaminants: With proteins, sugar, vitamins, oils and water, they contain everything microorganisms need to grow. In particular, cosmetic or pharmaceutical formulations with a higher water content and/or great surfaces, such as emulsions or wet-wipes, or formulations with natural ingredients, which are contaminated with bacteria innately, are susceptible against microbial growth. The neutral pH-value and the storage in a warm and humid bathroom contribute to an ideal climate for bacteria and moulds. Via hand and mouth, a lot of microorganisms get into the product. Sometimes the product is already contaminated during the production process, for example from contaminated raw materials. Most of the microorganisms are harmless to the consumer. However, microbial contaminants can cause dangerous infections. In addition, some moulds and bacteria produce toxins which can cause allergic reactions and skin irritations.

In order to minimize the microbial contamination, preservatives are used. Preservatives are essential in the production of cosmetics, because they kill or inhibit the growth of microorganisms. Without sufficient preservation, this in turn can lead to product spoilage, which in cosmetic products may manifest as changes in smell, discoloration, mould growth, gas formation, the separation of emulsions or changes in viscosity, rendering the product unacceptable to the consumer.

Preservatives are therefore very important to keep cosmetics from spoiling and to inhibit the growth of potentially dangerous microorganisms. However, not all substances that can be used as a preservative are harmless to the consumer. Parabens for example have a hormonal effect; benzoic acid can cause pseudo allergic reactions. The German Cosmetics Regulation lists all preservatives allowed in cosmetic products.

In addition, there are a couple of natural materials that contain substances that are said to have an antimicrobial effect. These include for example propolis, cinnamon bark, clove oil, allspice oil, nutmeg oil, ylang ylang, rose oil and thyme. However, these substances have not been adequately researched for use as a preservative in the cosmetics industry.

At the moment, there are no legally binding limit values for germ contents in cosmetics. According to a recommendation of the Scientific Committee on Consumer Safety of the European Commission, cosmetics should contain no more than 100 or 1.000 colony-forming units per gram, depending on the type of product. Certain pathogens such as Staphylococcus or *E. coli* are not allowed to be detectable at all.

Thus, in the cosmetic, pharmaceutical or homecare products industry and also in the food industry there is an ongoing need for agents with antimicrobial properties for product protection.

It is true that a multiplicity of antimicrobial active compounds are already used in the technical fields concerned, but alternatives continue to be sought in order to be able to carry out targeted special treatments and/or reduce side effects. However, in this context when searching for alternative agents having an antimicrobial and in particular a preservative action it must be taken into account that the substances used in the cosmetic, pharmaceutical and/or food sector must be
·toxicologically acceptable;
·well tolerated by the skin;
·stable (especially in the customary cosmetic and/or pharmaceutical formulations);
·substantially and preferably completely odourless; and
·able to be prepared inexpensively (i.e., using standard methods and/or starting from standard precursors) and of natural origin or based on green synthesis.

The search for suitable (active) substances that possess one or more of the said properties to an adequate extent is made more difficult for those skilled in the art because there is no clear dependence between the chemical structure of a substance, on the one hand, and its biological activity towards specific microorganisms (germs) and its stability, on the other hand. Furthermore, there is no predictable relationship between the antimicrobial action, toxicological acceptability, tolerance by the skin and the stability of a substance.

Furthermore, the antimicrobial component should have antimicrobial activity characteristics, which fulfil diverse criteria. They should
·provide a broad-spectrum antimicrobial activity against gram positive and gram-negative bacteria, yeast, mould and fungi;
·show a particularly strong activity against Candida and Aspergillus; and
·be active in different cosmetic or pharmaceutical formulations, in particular at different pH values.

The addition of biocides, especially classical preservative agents like formaldehyde donors, according to biocide regulation is increasingly undesired, in particular with regard to natural cosmetics.

Sterilisation of the formulation is less desired due to a possible degradation of ingredients of the formulation on heat treatment and additional process costs involved.

Thus, there is still an ongoing need to develop novel compositions that are microbial safe and microbial stable during shelf life. The cosmetic or pharmaceutical industry is permanently searching for ingredients which can improve this aspect. In particular, there is continued interest in the market to improve both the potential and the range of efficiency of such antimicrobials, either by achieving a higher performance of an antimicrobial with the same amounts, or, vice versa, by achieving the same performance with lower amounts of an antimicrobial, so that the overall content of antimicrobials can be reduced in the ready-for-use formulation.

It is therefore an object of the present invention to provide a composition, which develop a synergistic action against a multiplicity of bacteria, yeast, mould and fungi, especially in a cosmetic or pharmaceutical preparation or homecare product, and its use by which the activity or growth of microorganism in a cosmetic or pharmaceutical preparation or homecare product can be suppressed or inhibited.

Furthermore, it is an object of the present invention, to provide a food, cosmetic or pharmaceutical preparation or homecare product comprising said composition.

Furthermore, it is an object of the present invention, to provide a substance, by which the antimicrobial efficacy of an antimicrobial component can be boosted.

Furthermore, it is an object of the present invention, to provide a method for suppressing or inhibiting of microorganisms' growth in food, a cosmetic or a pharmaceutical preparation or homecare product.

EP 2 774 481 B1 and WO 2020/043269 A1 (see Tables X, XIII, XIV, XVII, XIX, XXII, XXVII, XXXI, XXXII, XXXIV to XXXVII, XXXIX, XXXXII, XXXXIV to XXXXVI, LVII and LX) disclose cosmetic compositions such as anti-dandruff shampoo, hair conditioner, after shave, etc. comprising:
(a) pentylene glycol (1,2-pentanediol), decylene glycol (1,2-decanediol) and/or 1,2-hexanediol and 1,2-octanediol (caprylyl glycol); and
(b) hydroxyphenyl propamidobenzoic acid, denatured alcohol, hydroxyacetophenone, climbazole, bisabolol, phenoxyethanol, methylparaben, ethylparaben, propylparaben, butylparaben, isobutylparaben and/or hydroxyacetophenone and/or one of the aforementioned alkanediols as antimicrobial compounds.

WO 2020/160905 A1 (see Tables 6, 7, 23 to 39, 43 to 52, 55, 57, 58, 61 to 67 and 69 to 73) discloses cosmetic compositions comprising:
(a) 1,2-decanediol (decylene glycol), 1,2-pentanediol (pentylene glycol), 1,2-hexanediol and/or 1,2-octanediol (caprylyl glycol); and
(b) propanediol caprylate, azelaic acid, glyceryl caprylate, bisabolol, hydroxyacetophenone, phenoxyethanol, sodium benzoate, hydroxyphenyl propamidobenzoic acid, zinc pyrithione, climbazole, disodium EDTA, triethyl citrate, piroctone olamine and/or ethanol as antimicrobial agents.

Compositions 2, 5 and 6 in Table 2, compositions 9 and 12 to 15 in Table 3 and compositions 18 and 21 to 24 in Table 4 of WO 2020/094244 A1 disclose bar soaps comprising:
(a) decylene glycol (1,2-decanediol),1,2-hexanediol and 1,2-octanediol; and
(b) phenoxyethanol, 2-benzylheptanol, lauryl alcohol, methylbenzyl alcohol, phenylpropanol, o-cymen-5-ol and/or climbazole as antimicrobial agents.

US 2016/374352 A1 relates to antimicrobial compositions for skin disinfection. Paragraphs [0171], [0172], [0174] to [0189], [0194] to [0196], etc. give examples of such compositions comprising:
(a) 1,2-octanediol; and
(b) various antimicrobial components.

US 2011/218250 A1 discloses a composition comprising 1,2-(ethylhexyl)glycerol ether and 1,2-octanediol (see claim 1). The composition further comprises at least one diol other than 1,2-octanediol. Claim 6 identifies this further diol as being one diol selected from the group consisting of 1,2-butanediol, 1,2-pentanediol, 1,2-hexanediol, 1,2-heptanediol and mixtures thereof.

The invention is based on the surprising finding that the concomitantly use of an effective amount of 2,3-octanediol or a mixture comprising 1,2-octanediol and 2,3-octanediol with the antimicrobial component 4-hydroxyacetophenone results in a synergistic antimicrobial efficacy action against a multiplicity of microorganisms such as bacteria, yeast, mould and fungi. The use of such a composition in food, a cosmetic or pharmaceutic preparation or homecare product in turn results in microbial stable formulations which can be stored. Surprisingly, the synergistic antimicrobial efficacy is directed also against species of the genus Candida, such as *Candida albicans* and Aspergillus, such as *Aspergillus brasiliensis,* which both are known to be combated only with great difficulty.

### Summary of the invention

In order to accomplish the above problem, the present invention provides a composition, comprising or consisting of
(a1) an effective amount of 2,3-octanediol;
(b1) an effective amount of 4-hydroxacetophenone; and
(c1) optionally at least one active substance for a food, a cosmetic or pharmaceutical composition or a homecare product and/or additive.

Alternatively, the present invention provides a composition, comprising or consisting of
(a) an effective amount of a mixture comprising 1,2-octanediol and 2,3-octanediol;
(b) an effective amount of 4-hydroxyacetophenone; and
(c) optionally at least one active substance for a food, a cosmetic or pharmaceutical composition or a homecare product and/or additive.

In a further aspect, the present invention provides for food, cosmetic or pharmaceutical preparations or homecare products comprising said composition according to the present invention.

In a further aspect, the present invention provides for the use of said composition according to the present for suppressing or inhibiting microorganism growth in food, a cosmetic or pharmaceutical preparation or homecare products.

In a still further aspect, the present invention provides for the use of 2,3-octanediol or a mixture comprising 1,2-octanediol and 2,3-octanediol for boosting the efficacy of 4-hydroxyacetophenone.

In a final aspect, the present invention provides a method for suppressing or inhibiting of microorganism growth in food, a cosmetic or a pharmaceutical preparation or a homecare product.

### Description of Figures

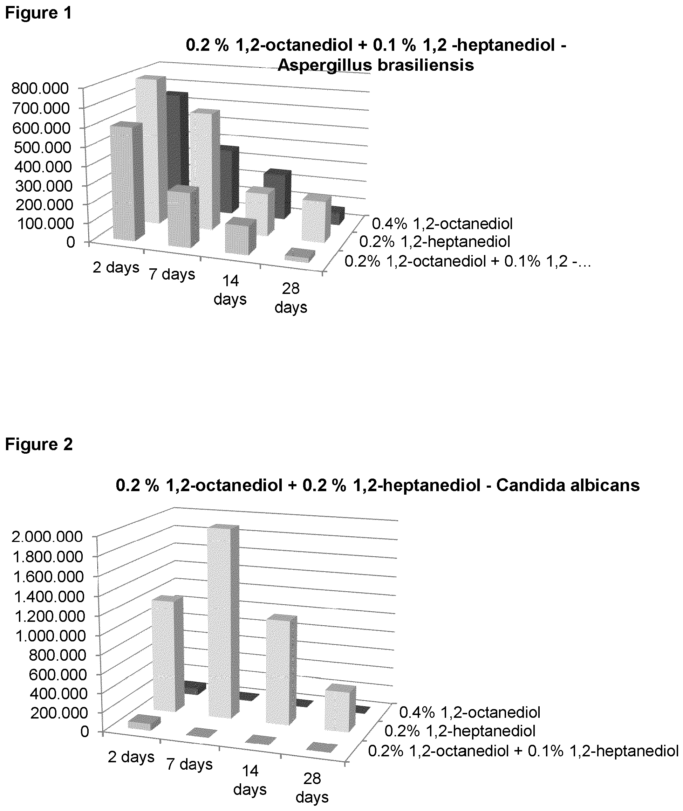

Figure 1 is a diagram visualizing the synergistic antimicrobial efficacy of 1,2-heptanediol and 1,2-octanediol against *Aspergillus brasiliensis* (not in accordance with the present invention).
Figure 2 is a diagram visualizing the synergistic antimicrobial efficacy of 1,2-heptanediol and 1,2-octanediol against *Candida albicans* (not in accordance with the present invention).

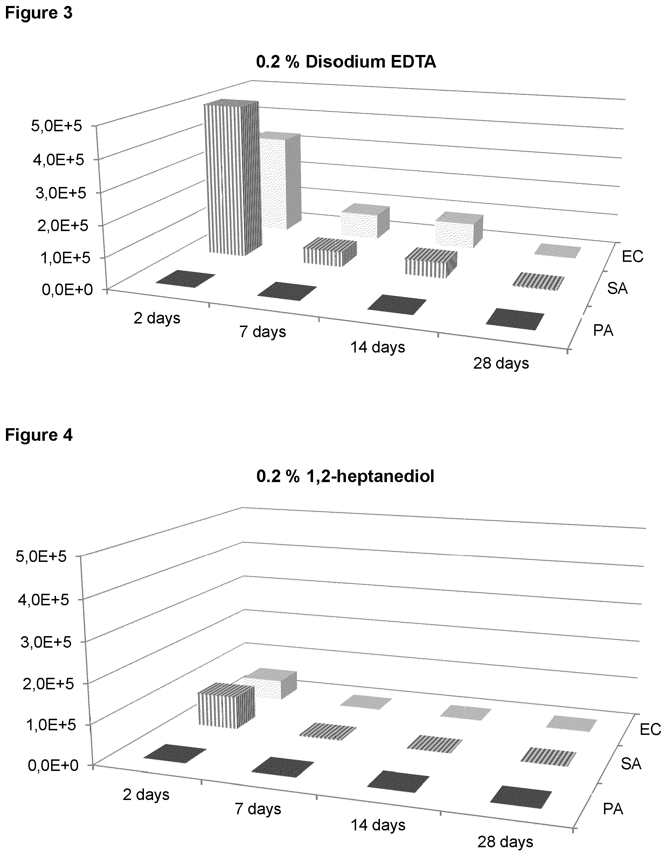

Figure 3 is a diagram visualizing the antimicrobial efficacy of 0.2 % Disodium EDTA against bacteria (not in accordance with the present invention).
Figure 4 is a diagram visualizing the antimicrobial efficacy of 0.2 % 1,2-heptanediol against bacteria (not in accordance with the present invention).

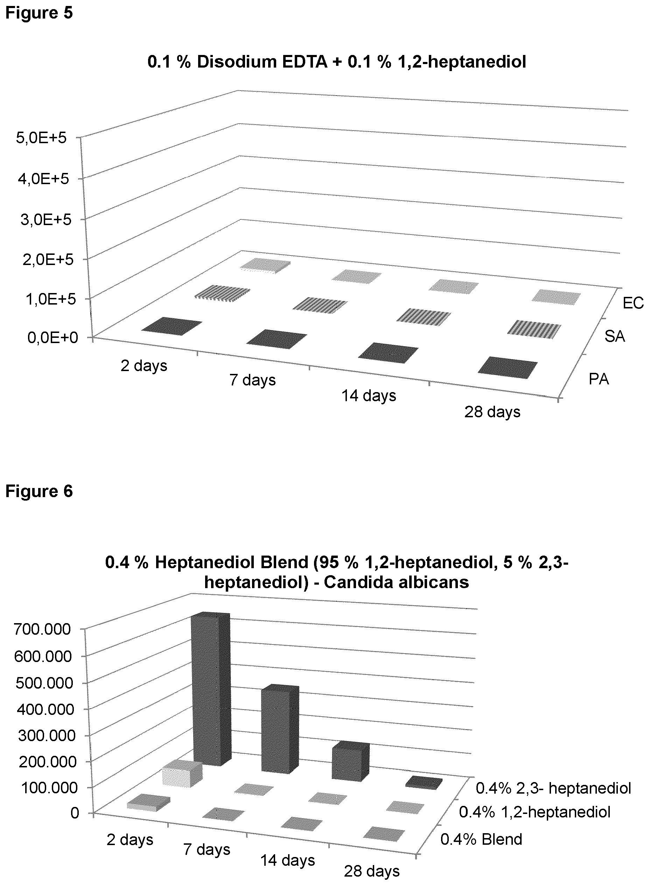

Figure 5 is a diagram visualizing the synergistic antimicrobial efficacy of 0.1 % Disodium EDTA plus 0.1 % 1,2-heptanediol against bacteria (not in accordance with the present invention).
Figure 6 is a diagram visualizing the synergistic antimicrobial efficacy of 1,2-heptanediol and 2,3-heptanediol against *Candida albicans* (not in accordance with the present invention).

### Detailed description of the invention

The present invention is specified in the appended claims. The invention itself, and its preferred variants, other objects and advantages, are however also apparent from the following detailed description in conjunction with the accompanying examples and figures.

The present invention relates to a composition, comprising or consisting of:
(a1) an effective amount of 2,3-octanediol;
(b1) an effective amount of 4-hydroxyacetophenone; and
(c1) optionally at least one active substance for a food, a cosmetic or pharmaceutical composition or a homecare product and/or additive.

Alternatively, the present invention relates to a composition, comprising or consisting of:
(a2) an effective amount of a mixture of comprising 1,2-octanediol and 2,3-octanediol;
(b2) an effective amount of 4-hydroxyacetophenone; and
(c2) optionally at least one active substance for a food, a cosmetic or pharmaceutical composition or a homecare product and/or additive.

The term "comprising" means that the named components in the composition are essential, but other components may be added and is still embraced by the present invention.

The term "consisting of" as used according to the present invention means that the total amount of components (a) to (c) adds up to 100 % by weight, based on the total weight of the composition, and signifies that the subject matter is closed-ended and can only include the limitations that are expressly recited.

Whenever reference is made to "comprising" it is intended to cover both meanings as alternatives, that is the meaning can be either "comprising" or "consisting of", unless the context dictates otherwise.

The term "at least one ..." means that the composition according to the present invention can comprise either one or a mixture of two, three, four, five, six or even more different of the respective components following said term.

The term "optionally" means that the subsequently described component may but need not to be present in the composition, and that the description includes variants, were the compound is included or variants, were the compound is absent.

The term "synergistic effect" is an effect achieved when two different chemical substances or biological structures interact resulting in acumulative overall effect that is greater than the sum of individual effects of any of them. In the present invention, the synergistic effect relates to a synergistic antimicrobial effect.

Antimicrobial effect in the context of the present invention means that the microorganisms' growth is reduced or even inhibited, so that the number of living microorganisms is reduced.

Alkanediols are glycols, i.e. any of a class of organic compounds belonging to the alcohol family; in the molecule of a glycol, two hydroxyl (-OH) groups are attached to different carbon atoms of a carbon chain.

The component (a) in the composition according to the present invention is either 2,3-octanediol (a1) or a mixture comprising 1,2-octanediol and 2,3-octanediol (a2).

1,2-octanediol belongs to the category of alkanediols and is a straight chain alkanediol with the general formula: 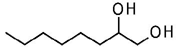 It has a carbon chain with 8 carbon atoms and the two functional OH groups of the alkanediol are in alpha,beta position and chemically bonded to the C1 and C2 carbon atoms in the alkanediol chain.

2,3-octanediol belongs to the category of alkanediols and is a straight chain alkanediol with the general formula: 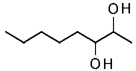 **It has a carbon** chain with 8 carbon atoms and the two functional OH groups of the alkanediol are in beta,gamma position and chemically bonded to the C2 and C3 carbon atoms in the alkanediol chain.

Straight chain 1,2-alkanediols have been used for more than 15 years as multifunctional actives. Short chain 1,2-alkanediols are amphiphilic compounds and thus, like 1,2-pentanediol and 1,2-hexanediol, are soluble both in water and cosmetic oils. In contrast, 1,2-octanediol is a solid and tends to precipitate or recrystallize in oily solutions as well as in water at ≥ 0.5 %. On the other hand, 1,2-decanediol is a solid and soluble only in cosmetic oils. Apart from moisturizing, some 1,2-alkanediols are used as viscosity modifiers.

The term "alkanediol" within the context of the present invention also includes its constitutional isomers or position isomers. Constitutional isomers are compounds that have the same molecular formula and different connectivity. Position isomers, a particular form of constitutional isomerism, are structural isomers that can be viewed as differing only on the position of a functional group on a parent structure, which in this case is the position of the two alcohol functions.

Exemplary 1,2-alkanediols are those as represented by the following formulae:

| | |
|---|---|
| 1,2-pentanediol | 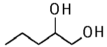 |
| 1,2-hexanediol | 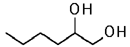 |
| 1,2-heptanediol | 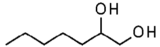 |
| 1,2-octanediol | 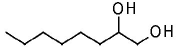 |
| 1,2-nonanediol | 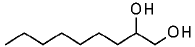 |
| 1,2-decanediol | 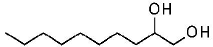 |
| 1,2-undecanediol | 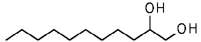 |
| 1,2-dodecanediol | 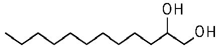 |
| 1,2-tridecanediol | 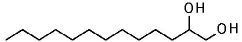 |
| 1,2-tetradecanediol | 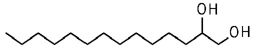 |

Examplary 2,3-alkanediols are those as represented by the following formulae:

| | |
|---|---|
| 2,3-pentanediol | 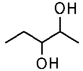 |
| 2,3-hexanediol | 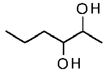 |
| 2,3-heptanediol | 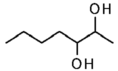 |
| 2,3-octanediol | 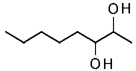 |
| 2,3-nonanediol | 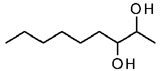 |
| 2,3-decanediol | 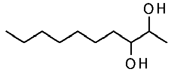 |
| 2,3-undecanediol | 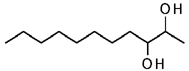 |
| 2,3-dodecanediol | 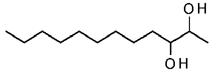 |
| 2,3-tridecanediol | 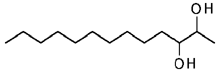 |
| 2,3-tetradecanediol | 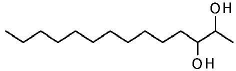 |

2,3-octanediol is liquid at a purity of 90 to 99 %.

The liquid 2,3-octanediol can be easier incorporated into semi-finished products or final products.

In a preferred variant, the composition according to the present invention comprises ▪ a mixture comprising 1,2-octanediol and 2,3-octanediol;

The above specified homo alkanediol mixture according to the present invention, comprising a 1,2-alkanediol and a 2,3-alkanediol are characterized by a synergistically antimicrobial efficacy against a broad spectrum of microorganism, i.e. against bacteria, yeast and fungi/mould when used in combination with the antimicrobial component4-hydroxyacetophenone, compared to their respective single alkanediol substances as it is demonstrated by the preservation efficacy test (PET) in the following examples. Surprisingly, the synergistic antimicrobial effect can even be demonstrated for Candida, such as *Candida albicans* and Aspergillus, such as *Aspergillus brasiliensis,* which both are known to be combated only with great difficulty.

The alkanediol mixture comprising 1,2-octanediol and 2,3-octanediol in combination with 4-hydroxyacetophenone is particularly advantageous.

With the use of the above-described alkanediol mixture including 1,2-octanediol and the corresponding 2,3-octanediol in combination with4-hydroxyacetophenone, the microbial growth in food, or cosmetic or pharmaceutical compositions or homecare products can be considerably inhibited, and, thus, microbial load in such formulations can be reduced.

With the above-described antimicrobial efficacy of the mixture including 1,2-octanediol and the corresponding2,3-alkanediol, microbial load in food, or cosmetic or pharmaceutical compositions or homecare products can be considerably reduced.

Hence, the storage stability of such formulations can be considerably improved, and, thus, the shelf live can be prolonged. Moreover, the risk of spoilage during individual customer use is significantly reduced.

Additionally, with the admixture of the corresponding liquid 2,3-alkanediol, even in small amounts, to the solid1,2-octanediol, the solid 1,2-octanediol can be solved, resulting in a liquid alkanediol mixture. The 2,3-alkanediol serves as solvent for the solid 1,2-alkanediol. Such liquid mixtures have the benefit that firstly the availability of the solid 1,2-alkanediol in the mixture is improved and secondly the incorporation of the solid 1,2-alkanediol in semi-finished products or final products is facilitated. This effect is particularly favorably for emulsions, in which lipophilic 1,2-alkanediols having a carbon chain of 8 or more carbon atoms, when used alone, tend to migrate into the oil phase. With the admixture of the corresponding liquid 2,3-alkanediol the 1,2-alkanediol remains in the water phase where usually microbial contamination takes place. This is in particular beneficial for the development of the antimicrobial effect of the 1,2-alkanediol in the water phase.

With the solution of the solid 1,2-octanediol in the liquid 2,3-octanediol, the availability of the strong antimicrobial 1,2-octanediol substance can be likewise improved in the end use.

In addition, the above alkanediol mixture comprising 1,2-octanediol and the corresponding 2,3-alkanediol render possible the cold preparation of cosmetic or pharmaceutical formulations, i.e. no heating during preparation is necessary.

Thus, with the afore-mentioned properties of the specified alkanediol mixtures including 1,2-octanediol and the respective 2,3-octanediol the processability in formulations can be improved.

Hence, a good compromise can be achieved when the active antimicrobial mixture is combined in such a way as to maintain the antimicrobial effect, while improving processability in formulations. The above specified 1,2-alkanediol and corresponding 2,3-alkanediol combination solves this balancing problem over the 1,2-alkanediol substance or 2,3-alkanediol substances alone.

The afore specified homo alkanediol mixture including 1,2-octanediol and the corresponding 2,3-alkanediol in combination with 4-hydroxyacetophenone displays a remarkably synergistic activity and is clearly superior to the individually corresponding 1,2-octanediol or 2,3-octanediol and having the same concentration.

The above specified alkanediol mixture according to the present invention, comprising1,2-octanediol and 2,3-octanediol, is characterized by a synergistically antimicrobial efficacy against a broad spectrum of microorganism, i.e. against bacteria, yeast and fungi/mould when used in combination with 4-hydroxyacetophenone compared to their respective single alkanediol substances as it is demonstrated in the following examples. Surprisingly, the synergistic antimicrobial effect even can be demonstrated for Candida, such as *Candida albicans* and Aspergillus, such as *Aspergillus brasiliensis,* which both are known to be combated only with great difficulty.

The use of the alkanediol mixture as specified before in combination with 4-hydroxyacetophenone results in an enhanced, i.e. synergistically, antimicrobial effect against a broad spectrum of microorganism, i.e. against bacteria, yeast and fungi/mould by inhibiting microorganisms' growth compared to the use of the corresponding single alkanediol substances.

With the use of the above-described alkanediol mixture including 1,2-octanediol and 2,3-octanediol in combination with4-hydroxyacetophenone, the microbial growth in food, or cosmetic or pharmaceutical compositions or homecare products can be considerably inhibited, and, thus, microbial load in such formulations can be reduced.

Hence, the storage stability of such formulations can be considerably improved, and, thus, the shelf life can be prolonged. Moreover, the risk of spoilage during individual customer use is significantly reduced.

The term "liquid alkanediol" within the context of the present invention means an alkanediol component which is liquid at room or ambient temperature and under normal pressure, i.e. standard RTP conditions.

The term "alkanediol" within the context of the present invention also includes its stereoisomers. Stereoisomers are molecules that have the same molecular formula and differ only in how their atoms are arranged in three-dimensional space. In accordance with said

definition, the 1,2-octanediol and 2,3-octanediol as described above in detail encompass the following stereoisomers:
1,2-alkanediol stereoisomers:
   (2S)-octane-1,2-diol,
   (2R)-octane-1,2-diol,
2,3-alkanediol stereoisomers:
   (2S,3S)-octane-2,3-diol,
   (2R,3R)-octane-2,3-diol,
   (2S,3R)-octane-2,3-diol,
   (2R,3S)-octane-2,3-diol,

In the context of the present text, the terms "1,2-diol" or "2,3-diol" includes both the corresponding S-configured enantiomers and also the R-enantiomer as well as arbitrary mixutres of these S- and R-configured enantiomers, i.e. mixtures of racemates of the respective diols.

The 1,2-octanediol and the 2,3-octanediol as defined in detail above are present in the alkanediol mixture of the composition according to the present invention in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

In the composition according to the present invention used in a cosmetic or pharmaceutical composition, the 1,2-octanediol and the 2,3-octanediol are comprised in the alkanediol mixture preferably in a ratio in a range of 98 : 2 to 99.9 : 0.1

In the composition according to the present invention used in a homecare product, the 1,2-octanediol and the 2,3-octanediol are comprised in the alkanediol mixture preferably in a ratio in a range of 95 : 5 to 99.9 : 0.1.

Preferably, the 1,2-octanediol and the 2,3-octanediol are comprised in the alkanediol mixture of the composition according to the present invention in a ratio of ≥ 95 : ≤ 5, more preferred in a ratio of ≥ 96 : ≤ 4; still more preferred in a ratio of ≥ 97 : ≤ 3, and most preferred in a ratio of ≥ 98 : ≤ 2.

Most of all the alkanediol mixture of the composition according to the present invention comprises the 1,2-octanediol and the 2,3-octanediol in a ratio of ≥ 95 : ≤ 5, including the ratios ≥ 95.5 : ≤ 4.5; ≥ 96 : ≤ 4; ≥ 96.5 : ≤ 3.5; ≥ 97 : ≤ 3; ≥ 97.5 : 2,5 and ≥ 98.0 : ≤ 2.0.

Even more preferred the alkanediol mixture of the composition according to the present invention comprises the 1,2-octanediol and the 2,3-octanediol in a ratio of ≥ 98 : ≤ 2, including the ratios of ≥ 98.1 : ≤ 1.9; ≥ 98.2 : ≤ 1.8; ≥ 98.3 : ≤ 1.7; ≥ 98.4 : ≤ 1.6; ≥ 98.5 : ≤ 1.5; ≥ 98.6 : ≤ 1.4; ≥ 98.7 : ≤ 1.3; ≥ 98.8 : ≤ 1.2; ≥ 98.9 : ≤ 1.1; ≥ 99 : ≤ 1.0; ≥ 99.1 : ≤ 0.9; ≥ 99.2 : ≤ 0.8; ≥ 99.3 : ≤ 0.7; ≥ 99.4 : ≤ 0.6; ≥ 99.5 : ≤ 0.5; ≥ 99.6 : ≤ 0.4; ≥ 99.7 : ≤ 0.3; ≥ 99.8 : ≤ 0.2 and ≥ 99.9 : ≤ 0.1.

Alternatively, said ratio ranges for the 1,2-octanediol and 2,3-octanediol are switched, such that the second alkanediol is the main component and the first alkanediol is the secondary component.

According to the present invention, the mixture comprises as first linear alkanediol 1,2-octanediol and as second linear alkanediol the corresponding 2,3- octanediol, in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said mixtures the ratios of first linear alkanediol : second alkanediol or ranges of ratios as described above are also applicable.

According to the present invention, the mixture comprises as first linear alkanediol 2,3-octanediol and as second linear alkanediol the corresponding 1,2- octanediol in a ratio in a range of 50 : 50 to 99.9 : 0.1, preferably in a ratio in a range of 75 : 25 to 99 : 1, more preferred in a ratio in a range of 80 : 20 to98 : 2, still more preferred in a ratio in a range of from 90 : 10 to 95 : 5.

Likewise, for said mixtures the ratios of first linear alkanediol : second alkanediol or ranges of ratios as described above are also applicable.

According to the present invention, in the mixture comprising a combination comprising a 1,2-alkanediol as first linear alkandediol and the corresponding 2,3-alkanediol as second linear alkanediol, such as
1,2-octanediol and 2,3-octanediol,
the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show a synergistic antimicrobial efficacy when used in combination with an antimicrobial component in direct comparison with the corresponding individual substances as it is described and demonstrated in the experimental part.

According to the present invention, in the mixtures comprising as first linear alkanediol a 2,3-alkanediol and as second linear alkanediol the corresponding 1,2-alkanediols, such as 2,3-octanediol and 1,2-octanediol,
wherein the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show a synergistic antimicrobial efficacy when used in combination with 4-hydroxyacetophenone in direct comparison with the corresponding individual substances as it is described and demonstrated in the experimental part..

**The** alkanediols, in particular 1,2-octanediol and 2,3-octanediol, are obtained either by synthesis from petrochemical or other fossil fuel sources by known methods such as olefin bishydroxylation, hydrolysis from epoxide or various chemical transformations or from bioderived feedstock by fermentation or from bio-based natural and renewable feedstock such as biomass by catalytic synthesis as it is described in US 2019/0241491 A1 and US 2020/0189995 A1. **The** alkanediols used according to the present invention comprise either petrochemically derived and biobased natural and renewable feedstock derived alkanediols. Preferably, the alkanediols are from bio-based sources and are thus bio-alkanediols.

**The** compound (b) of the composition according to the present invention relates to 4-hydroxyacetophenone as antimicrobial component, which is commonly used in many cosmetic preparations, in particular skincare, haircare or body care, or in pharmaceutical preparations.

**The** term "antimicrobial component" in the context of the present invention refers to a group of substances, in particular to an agent that kills microorganisms or stops or inhibits their growth. Antimicrobial agents can be grouped according to the microorganisms they act primarily against. For example, antibiotics are used against bacteria, antifungals are used against fungi, antiprotozoals are used against protozoans, and antivirals are used against virus. They can also be classified according to their function. Agents that kill microbes are biocidal or microbicidal, while those that merely inhibit their growth are called biostatic.

The antimicrobial component 4-hydroxylacetophenone according to the present invention act primarily against microorganisms, in particular bacteria, yeast and/or fungi. Specified microorganisms are aerobic mesophilic bacteria or yeast or fungi undesirable in a cosmetic product and recognised as a skin pathogen species that may be harmful for human health or as an indication of hygienic failure in the manufacturing process. Microorganisms considered as specified microorganisms according to the present invention are of the genus Pseudomonas (bacterium), Staphylococcus (bacterium), Escherichia (bacterium), Candida (yeast) and Aspergillus (fungi), and combinations thereof.

More preferably, the microorganisms are selected from the group consisting of *Pseudomonas aeruginosa, Staphylococcus aureus, Escherichia coli, Candida albicans* and *Aspergillus brasiliensis,* and combinations thereof.

The composition according to the present invention comprises one of the following combinations of components (a) and (b):
▪ 2,3-octanediol and 4-Hydroxyacetophenone; or
▪ 1,2-octanediol and 2,3-octanediol and 4-Hydroxyacetophenone

Said combinations achieve synergistic antimicrobial efficacy against bacteria, yeast, and fungi/mould, as described in the following examples.

The above-defined specific combinations can be combined with one or more further components as described later on.

In order to further enhance antimicrobial efficacy, the composition as defined herein, is advantageously combined with at least one further antimicrobial agent which is different from the component (b) of the composition according to the present invention. The combination with a further antimicrobial agent provides reliable protection against microbial degradation and deterioration of the preparation, in particular during storage. Additionally, the further different antimicrobial agent provides reliable protection against other microorganisms as described above, for example Corynebacterium, Anaerococcus, Finegoldia, Moraxella, Porphyromonas, Fusobacterium, Malassezia, Peptoniphilus, Streptococcus, Lactobacillus, Gardnerella, Fannyhessea, Epidermophyton, Trichophyton, Cutibacterium. Hence, the combination with a further different antimicrobial agent allows antimicrobial protection against different groups of microorganisms, and, thus, a broader spectrum of microorganism. Furthermore, with the further different antimicrobial agent the composition according to the present invention can also be provided with a different antimicrobial target, preferably antimicrobials for skin protection.

The further antimicrobial agent according to the present invention is preferably selected from the group consisting of Caprylhydroxamic Acid, o-Cymen-5-ol, Isopropylparaben, Capryloyl Glycine, Phenylpropanol, Tropolone, PCA Ethyl Cocoyl Arginate, 2-Methyl 5-Cyclohexylpentanol, Phenoxyethanol, Disodium EDTA, Methylparaben and its salts, Sodium Benzoate, Benzyl Alcohol, Potassium Sorbate, Benzyl Salicylate, Propylparaben, Sodium Methylparaben, Methylchloroisothiazolinone, Methylisothiazolinone, Ethylhexylglycerin, Butylparaben, Ethylparaben, Sodium Propylparaben, DMDM Hydantoin, Dehydroacetic Acid, Salicylic Acid, Chlorphenesin, Isobutylparaben, Sodium Ethylparaben, Diazolidinyl Urea, Farnesol, Bisabolol, Glyceryl Caprylate, Sodium Phytate or Phytic Acid, Sodium Levulinate or Levulinic Acid and esters and/or ketals thereof, Chlorhexidine, Glyceryl Laurate, Anisic Acid and its salts, Chlorhexidine Digluconate, TEA-salicylate, Phenethyl Alcohol, Glyceryl Caprate, Sorbitan Caprylate, Tetrasodium Glutamate Diacetate, Trisodium Ethylenediamine Disuccinate, Hydroxyethoxyphenyl Butanone, Hydroxyethoxyphenyl Butanol, Itaconic Acid, Octopirox, Propanediol Caprylate, Climbazole, Undecylenoyl Glycine, Thymol, Lactic Acid, Sodium Lactate, 1,3-propanediol, 1,2-pentanediol, 1,2-hexanediol, 1,2-heptanediol, 1,2-nonanediol, 1,2-decanediol, 2,3-pentanediol, 2,3-hexanediol, 2,3-heptanediol, 2,3-nonanediol, and mixtures of two or more the aforesaid antimicrobial components.

In addition, the compoisiton according to the present invention further comprises a antimicrobial agent different to the antimicrobial agents in the above list, which is preferably selected from the group consisting of 2-benzylheptanol, alkyl (C 12-22) trimethyl ammonium bromide and chloride, ascorbic acid and salts thereof, benzalkonium bromide, benzalkonium chloride, benzalkonium saccharinate, benzethonium chloride, Benzoic Acid, camphor, cetylpyridinium chloride, chlorhexidine diacetate, chlorhexidine dihydrochloride, chlorocresol, chloroxylenol, Cyclohexylglycerin, Dimethyl phenylbutanol, Dimethyl phenylpropanol, ethanol, ethyl lauroyl arginate HCl, Ethyl Lauroyl Arginate Laurate, eucalyptol, gluconic acid and salts thereof, glycerin, hexamidine, hexamidine diisethionate, Hexylglycerin, lodopropynyl Butylcarbamate, jasmol, lauryl alcohol, Levulinic Acid and esters and/or ketals thereof, mannitol, menthol, methyl salicylate, Octenidine HCl, polyarginine, Polyglyceryl-10 Laurate, polyglyceryl-2 caprate, Polyglyceryl-3 caprylate, polylysine, Salvia Officinalis (Sage) Oil, silver chloride, silver citrate, sodium caproyl lactylate, Sodium Caproyl/Lauroyl Lactylate, sodium lauroyl lactylate, Sorbic Acid, sorbitol, Tetraselmis extract, triclocarban, triclosan, Triethyl citrate, Xylityl Sesquicaprylate, zinc citrate, zinc pyrithione, Zinc ricinoleate, and mixtures of two or more of the aforesaid antimicrobial agents.

In a more preferred variant, the further different antimicrobial agent is selected from the group consisting of 2-benzylheptanol, Benzoic Acid, Dimethyl phenylbutanol, Dimethyl phenylpropanol, ethyl lauroyl arginate HCl, Ethyl Lauroyl Arginate Laurate, gluconic acid and salts thereof, Glyceryl laurate, jasmol, lauryl alcohol, Levulinic Acid and esters and/or ketals thereof, Mannitol, Octenidine HCl, Polyglyceryl-10 Laurate, polyglyceryl-2 caprate, Polyglyceryl-3 caprylate, Salvia Officinalis (Sage) Oil, sodium caproyl lactylate, Sodium Caproyl/Lauroyl Lactylate, sodium lauroyl lactylate, Sorbic Acid, sorbitol, Tetraselmis extract, Triclosan, Triethyl citrate, Xylityl Sesquicaprylate, Zinc ricinoleate, and mixtures of two or more of the aforesaid antimicrobial agents.

Within the context of the present invention, it is also possible and in some cases advantageous to combine the composition according to the present invention with other customary active substances, adjuvants or additives.

Optionally, other conventional active substances, adjuvants or additives, customarily used for food, cosmetic or pharmaceutical compositions or homecare products, as further described below, may be added as component (c), i.e. in order to obtain a ready-for-use preparation or formulation such as food, cosmetic or pharmaceutical preparation, preparation for animal care or homecare product.

The composition according to the present invention can advantageously be combined with other active agents and/or adjuvants and/or additives or auxiliaries, such as are customarily used in food, cosmetic or pharmaceutical preparations, preparations for animal care of homecare products, such as for example abrasives, anti-acne agents, agents against ageing of the skin, anti-cellulitis agents, anti-dandruff agents, anti-inflammatory agents, irritation-preventing agents, irritation-inhibiting agents, antioxidants, astringents, odour absorbers, perspiration-inhibiting agents, antiseptic agents, anti-statics, binders, buffers, carrier materials, chelating agents, cell stimulants, cleansing agents, depilatory agents, surface-active substances, deodorizing agents, antiperspirants, softeners, emulsifiers, enzymes, enzyme inhibitors, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, dyes, friction-reducing agents, lubricants, moisturizing creams, ointments, opacifying agents, plasticizing agents, covering agents, polish, preservatives, gloss agents, green and synthetic polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, surfactants, UV-absorbing agents, UV filters, primary sun protection factors, secondary sun protection factors, detergents, fabric conditioning agents, suspending agents, skin-tanning agents, actives modulating skin or hair pigmentation, matrix-metalloproteinase inhibitors, skin moisturizing agents, glycosaminoglycan stimulators, TRPV1 antagonists, desquamating agents, anti-cellulite agents or fat enhancing agents, hair growth activators or inhibitors, thickeners, rheology additives, vitamins, oils, waxes, pearlizing waxes, fats, phospholipids, saturated fatty acids, mono- or polyunsaturated fatty acids, α-hydroxy acids, polyhydroxy fatty acids, liquefiers, dyestuffs, colour-protecting agents, pigments, anti-corrosives, fragrances or perfume oils, aromas, flavouring substances, odoriferous substances, polyols, electrolytes, organic solvents, and mixtures of two or more of the aforementioned substances, as further described below.

Of the above cosmetically or pharmaceutically active agents and/or adjuvants and/or additives or auxiliaries agents against ageing of the skin, antioxidants, chelating agents, emulsifiers, surfactants, green and synthetic polymers, skin-cooling agents, rheology additives, oils, fragrances or perfume oils, and polyols are particularly preferred in the preparation of cosmetic and pharmaceutical composition.

The cosmetically or pharmaceutically active agents and/or adjuvants and/or additives can in some instances provide one or more than one benefit or operate via more than one mode of action.

Since dermatological conditions or diseases are often associated with dry skin, scratched skin, skin lesions or even inflammation, the composition according to the present invention advantageously contains preferably anti-inflammatories, antibacterial or antimycotic substances, substances having a reddening-alleviating or itch-alleviating action, lenitive substances, moisturisers and/or cooling agents, osmolytes, keratolytic substances, nurturing substances, anti-inflammatory, antibacterial or antimycotic substances, substances having a reddening-alleviating or itch-alleviating action, lenitive substances, anti-dandruff substances, or other active compounds such as solvents, fragrances antioxidants, preservatives, (metal) chelating agents, penetration enhancers, or mixtures of two or more of afore specified agents, as further described below.

**Anti-ageing** actives: A composition according to the present invention is preferably combined with one or more anti-ageing actives. In the context of the invention, anti-ageing or biogenic agents are, for example antioxidants, matrix-metalloproteinase inhibitors (MMPI), skin moisturizing agents, glycosaminglycan stimulators, anti-inflammatory agents, TRPV1 antagonists and plant extracts.

**Antioxidants:** A composition according to the present invention is preferably combined with one or more antioxidants. Suitable antioxidants encompass amino acids (preferably glycine, histidine, tyrosine, tryptophan) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, Levulinic Acid and esters and/or ketals thereof, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone. If vitamin E and/or derivatives thereof are used as the antioxidant(s), it is advantageous to choose their concentrations from the range from about 0.001 to about 10 % by weight, based on the total weight of the composition. If vitamin A or vitamin A derivatives or carotenes or derivatives thereof are used as the antioxidant(s), it is advantageous to choose their concentrations from the range from about 0.001 to about 10 % by weight based on the total weight of the composition.

Matrix-Metalloproteinase inhibitors (MMPI): A composition according to the present invention is preferably combined with one or more matrix-metalloproteinase inhibitors, especially those inhibiting matrix-metalloproteinases enzymatically cleaving collagen, selected from the group consisting of ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, MGDA (Trisodiumdicarboxymethyl alaninante), galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf, as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

Skin-moisturizing agents: A preferred composition according to the present invention comprises one or more skin-moisturizing agents. Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of glycerol, 1,2-propylene glycol, 1,2-butylene glycol, 1,3-butylene glycol, 1,2-pentanediol, 1,2-hexanediol, 1,2-heptanediol, 1,2-octanediol and 1,2-decanediol.

Glycosaminoglycan stimulators: A preferred composition according to the present invention comprises one or more substances stimulating the synthesis of glycosaminoglycans which are selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

TRPV1 antagonists: A preferred composition according to the present invention comprises one or more TRPV1 antagonists. Suitable compounds which reduce the hypersensitivity of skin nerves based on their action as TRPV1 antagonists, encompass e.g., trans-4-tert-butyl cyclohexanol, or indirect modulators of TRPV1 by an activation of the µ-receptor, e.g., acetyl tetrapeptide-15, are preferred.

Plant extracts: A preferred composition according to the present invention comprises one or more plant extracts. Plant extracts, special highly active plant extract fractions and also highly pure active substances isolated from plant extracts can also be used in the composition according to the present invention. Extracts, fractions and active substances from camomile, *aloe vera, Commiphora* species, *Rubia* species, willows, willow-herb, ginger, marigold, arnica, Glycyrrhiza species, Echinacea species, Rubus species and pure substances such as *inter alia* bisabolol, apigenin, apigenin-7-glucoside, gingerols such as [6]-gingerol, paradols such as [6]-paradol, boswellic acid, phytosterols, glycyrrhizine, glabridin or licochalcone A are particularly preferred.

Anti-inflammatory agents: The composition according to the present invention is preferably combined with anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. More particularly:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occurring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occurring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3->1,4-β-glucan from oats.

When bisabolol is used in the context of the present invention it can be of natural or synthetic origin, and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide (CO₂), hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

**Physiological cooling agents:** The composition according to the present invention can be particularly advantageously combined with one or more physiological cooling agent(s). The use of cooling agents can alleviate itching. Preferred individual cooling agents for use within the framework of the present invention are listed below. The person skilled in the art can add many other cooling agents to this list; the cooling agents listed can also be used in combination with one another: which are preferably selected here from the following list: menthol and menthol derivatives (for example L-menthol, D-menthol, racemic menthol, isomenthol, neoisomenthol, neomenthol) menthylethers (for example (I-menthoxy)-1,2-propanediol, (I-menthoxy)-2-methyl-1,2-propanediol, I-menthyl-methylether), menthone glyceryl acetal, menthone glyceryl ketal or mixtures of both, menthylesters (for example menthylformiate, menthylacetate, menthylisobutyrate, menthyhydroxyisobutyrat, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], menthanecarboxylic acid-N-(4-cyanophenyl)amide or menthanecarboxylic acid-N-(4-cyanomethylphenyl)amide, menthanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates and [(1R,2S,5R)-2-isopropyl-5-methyl-cyclohexyl] 2-(ethylamino)-2-oxo-acetate (X Cool). Cooling agents which are preferred due to their particular synergistic effect are I-menthol, d-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), substituted menthyl-3-carboxamides (such as menthyl-3-carboxylic acid N-ethyl amide), 2-isopropyl-N-2,3-trimethyl butanamide, substituted cyclohexane carboxamides, 3-menthoxypropane-1,2-diol, 2-hydroxyethyl menthyl carbonate, 2-hydroxypropyl menthyl carbonate and isopulegol. Particularly preferred cooling agents are I-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), 3-menthoxypropane-1,2-diol, 2-hydroxyethyl menthyl carbonate and 2-hydroxypropyl menthyl carbonate. Very particularly preferred cooling agents are I-menthol, menthone glycerol acetal (trade name: Frescolat^{®} MGA) and menthyl lactate (preferably I-menthyl lactate, in particular I-menthyl I-lactate (trade name: Frescolat^{®} ML).

Moisturising and/or moisture-retaining substances: Itching occurs with particular intensity when the skin is dry. The use of skin-moisturising and/or moisture-retaining substances can significantly alleviate itching. The composition according to the present invention can therefore advantageously also contain one or more of the following moisturising and/or moisture-retaining substances: sodium lactate, urea, urea derivatives, alcohols, glycerol, diols such as propylene glycol, hexylene glycol, collagen, elastin or hyaluronic acid, diacyl adipates, petrolatum, urocanic acid, lecithin, panthenol, phytantriol, lycopene, (pseudo-)ceramides, glycosphingolipids, cholesterol, phytosterols, chitosan, chondroitin sulphate, lanolin, lanolin esters, amino acids, alpha-hydroxy acids (such as citric acid, lactic acid, malic acid) and their derivatives, mono-, di- and oligosaccharides such as glucose, galactose, fructose, mannose, fructose and lactose, polysugars such as R-glucans, in particular 1,3-1,4-β-glucan from oats, alpha-hydroxy fatty acids, triterpene acids such as betulinic acid or ursolic acid, and algae extracts.

Lenitive substances: The composition according to the present invention can also contain advantageously one or more lenitive substances, wherein any lenitive substances can be used which are suitable or customary in cosmetic or pharmaceutical applications such as alpha-bisabolol, azulene, guaiazulene, 18-beta-glycyrrhetinic acid, allantoin, Aloe vera juice or gel, extracts of Hamamelis virginiana (witch hazel), Echinacea species, Centella asiatica, chamomile, Arnica monatana, Glycyrrhiza species, algae, seaweed and Calendula officinalis, and vegetable oils such as sweet almond oil, baobab oil, olive oil and panthenol, Laureth-9, Trideceth-9 and 4-t-butylcyclohexanol.

Antibacterial or antimycotic active substances: In addition to the antimicrobials as described therein, antibacterial or antimycotic active substances can also particularly advantageously be used in the composition according to the present invention, wherein any antibacterial or antimycotic active substances can be used which are suitable or customary in cosmetic or pharmaceutical, in particular dermatological applications. In addition to the large group of conventional antibiotics, other products which are advantageous here include those relevant to cosmetics such as in particular triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate or combinations of said substances.

Desquamating agents: The composition according to the present invention preferably contains one or more desquamating agents. The expression "desquamating agent" is understood to mean any compound capable of acting:
- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosulphonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine^{®}, prickly pear extracts such as those marketed under the name Exfolactive^{®} by the company SILAB, or Phytosphingosine SLC^{®} (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, chestnut or prickly pear extracts, in particular marketed by SILAB; reducing compounds such as cysteine or cysteine precursors. Desquamating agents which can be used are also nicotinic acid and its esters and nicotinamide, also called vitamin B3 or vitamin PP, and ascorbic acid and its precursors.

**Anti-dandruff substances:** In addition, the composition according to the present invention can also advantageously be used in combination with one or more anti-dandruff substances, including triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate, Propanediol Monocaprylate or combinations of said substances, which are used inter alia against dandruff.

Further suitable anti-dandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

**(Metal) chelating agents:** A combination with one or more (metal) chelating agents can also be advantageous used in the composition according to the present invention, wherein any metal chelating agents can be used which are suitable or customary in cosmetic or pharmaceutical applications. Preferred (metal) chelating agents include α-hydroxy fatty acids, phytic acid, lactoferrin, α-hydroxy acids, such as *inter alia* gluconic acid, glyceric acid, glycolic acid, isocitric acid, citric acid, lactic acid, malic acid, mandelic acid, tartaric acid, as well as humic acids, bile acids, bile extracts, bilirubin, biliverdin or EDTA, EGTA, MGDA (Trisodiumdicarboxymethyl alaninante), and their derivatives. The use of one or more chelating agent(s) improves the stability of the composition according to the present invention.

**Emulsifiers:** In addition, the composition according to the present invention can also advantageously contain one or more emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. **The** preferred emulsifiers are described in more detail as follows:

Partial glycerides: Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Sorbitan esters: Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Polyglycerol esters: Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate, and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Anionic emulsifiers: Typical anionic emulsifiers are aliphatic C12 to C 22 fatty acids, such as palmitic acid, stearic acid or behenic acid for example, and C12 to C22 dicarboxylic acids, such as azelaic acid or sebacic acid for example.

Amphoteric emulsifiers: Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

**Surfactants:** The composition according to the present invention preferably includes one or more anionic and/or amphoteric or zwitterionic surfactants. Typical examples encompass: Almondamidopropylamine Oxide, Almondamidopropyl Betaine, Aminopropyl Laurylglutamine, Ammonium C12-15 Alkyl Sulfate, Ammonium C12-16 Alkyl Sulfate, Ammonium Capryleth Sulfate, Ammonium Cocomonoglyceride Sulfate, Ammonium Coco-Sulfate, Ammonium Cocoyl Isethionate, Ammonium Cocoyl Sarcosinate, Ammonium C12-15 Pareth Sulfate, Ammonium C9-10 Perfluoroalkylsulfonate, Ammonium Dinonyl Sulfosuccinate, Ammonium Dodecylbenzenesulfonate, Ammonium Isostearate, Ammonium Laureth-6 Carboxylate, Ammonium Laureth-8 Carboxylate, Ammonium Laureth Sulfate, Ammonium Laureth-5 Sulfate, Ammonium Laureth-7 Sulfate, Ammonium Laureth-9 Sulfate, Ammonium Laureth-12 Sulfate, Ammonium Lauroyl Sarcosinate, Ammonium Lauryl Sulfate, Ammonium Lauryl Sulfosuccinate, Ammonium Myreth Sulfate, Ammonium Myristyl Sulfate, Ammonium Nonoxynol-4 Sulfate, Ammonium Nonoxynol-30 Sulfate, Ammonium Oleate, Ammonium Palm Kernel Sulfate, Ammonium Stearate, Ammonium Tallate, AMPD-Isostearoyl Hydrolyzed Collagen, AMPD-Rosin Hydrolyzed Collagen, AMP-Isostearoyl Hydrolyzed Collagen, AMP-Isostearoyl Hydrolyzed Keratin, AMP-Isostearoyl Hydrolyzed Soy Protein, AMP-Isostearoyl Hydrolyzed Wheat Protein, Apricotamidopropyl Betaine, Arachidic Acid, Arginine Hexyldecyl Phosphate, Avocadamidopropyl Betaine, Avocado Oil Glycereth-8 Esters, Babassu Acid, Babassuamidopropylamine Oxide, Babassuamidopropyl Betaine, Beeswax Acid, Behenamidopropyl Betaine, Behenamine Oxide, Beheneth-25, Beheneth-30, Behenic Acid, Behenyl Betaine, Bis- Butyldimethicone Polyglyceryl-3, Butoxynol-5 Carboxylic Acid, Butoxynol-19 Carboxylic Acid, Butyldimoniumhydroxypropyl Butylglucosides Chloride, Butyldimoniumhydroxypropyl Laurylglucosides Chloride, Butyl Glucoside, Butylglucoside Caprate, Butylglucosides Hydroxypropyltrimonium Chloride, Butyloctanoic Acid, C18-36 Acid, C20-40 Acid, C30-50 Acid, C16-22 Acid Amide MEA, Calcium Dodecylbenzenesulfonate, Calcium Lauroyl Taurate, C9-16 Alkane/Cycloalkane, C10-14 Alkyl Benzenesulfonic Acid, C12-14 Alkyl Diaminoethylglycine HCL, C9-15 Alkyl Phosphate, Candida Bombicola/Glucose/Methyl Rapeseedate Ferment, Canolamidopropyl Betaine, Capric Acid, Caproic Acid, Caproyl Ethyl Glucoside, Capryl/Capramidopropyl Betaine, Capryleth-4 Carboxylic Acid, Capryleth-6 Carboxylic Acid, Capryleth-9 Carboxylic Acid, Caprylic Acid, Capryloyl Collagen Amino Acids, Capryloyl Glycine, Capryloyl Hydrolyzed Collagen, Capryloyl Hydrolyzed Keratin, Capryloyl Keratin Amino Acids, Capryloyl Silk Amino Acids, Caprylyl/Capryl Glucoside, Caprylyl/Capryl Wheat Bran/Straw Glycosides, Caprylyl Glucoside, Caprylyl Glyceryl Ether, Caprylyl Pyrrolidone, Carnitine, Ceteareth-20, Ceteareth-23, Ceteareth-24, Ceteareth-25, Ceteareth-27, Ceteareth-28, Ceteareth-29, Ceteareth-30, Ceteareth-33, Ceteareth-34, Ceteareth-40, Ceteareth-50, Ceteareth-55, Ceteareth-60, Ceteareth-80, Ceteareth-100, Ceteareth-25 Carboxylic Acid, Ceteareth-2 Phosphate, Ceteareth-4 Phosphate, Ceteareth-5 Phosphate, Ceteareth-10 Phosphate, Ceteth-20, Ceteth-23, Ceteth-24, Ceteth-25, Ceteth-30, Ceteth-40, Ceteth-45, Ceteth-150, Ceteth-8 Phosphate, Ceteth-10 Phosphate, Ceteth-20 Phosphate, Cetoleth-22, Cetoleth-24, Cetoleth-25, Cetoleth-30, Cetyl Betaine, Chrysanthemum Sinense Flower Extract, C12-14 Hydroxyalkyl Hydroxyethyl Beta-Alanine, C12-14 Hydroxyalkyl Hydroxyethyl Sarcosine, Cocamidoethyl Betaine, Cocamidopropylamine Oxide, Cocamidopropyl Betainamide MEA Chloride, Cocamidopropyl Betaine, Cocamidopropyl Hydroxysultaine, Cocamine Oxide, Cocaminobutyric Acid, Cocaminopropionic Acid, Coceth-7 Carboxylic Acid, Coceth-4 Glucoside, Cocoamphodipropionic Acid, Cocobetainamido Amphopropionate, Coco-Betaine, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Coco-Glucoside, Cocoglucosides Hydroxypropyltrimonium Chloride, Coco- Hydroxysultaine, CocoMorpholine Oxide, Coconut Acid, Coconut Oil Glycereth-8 Esters, Coco/Oleamidopropyl Betaine, Coco-Sultaine, Coco/Sunfloweramidopropyl Betaine, Cocoylcholine Methosulfate, Cocoyl Glutamic Acid, Cocoyl Hydrolyzed Collagen, Cocoyl Hydrolyzed Keratin, Cocoyl Hydrolyzed Oat Protein, Cocoyl Hydrolyzed Rice Protein, Cocoyl Hydrolyzed Silk, Cocoyl Hydrolyzed Soy Protein, Cocoyl Hydrolyzed Wheat Protein, Cocoyl Sarcosine, Corn Acid, Cottonseed Acid, Cottonseed Oil Glycereth-8 Esters, C10-16 Pareth-1, C10-16 Pareth-2, C11-13 Pareth-6, C11-13 Pareth-9, C11-13 Pareth-10, C11-15 Pareth-30, C11-15 Pareth-40, C12-13 Pareth-1, C12-13 Pareth- 23, C12-14 Pareth-5, C12-14 Pareth-9, C13-15 Pareth-21, C14-15 Pareth-8, C20-22 Pareth-30, C20- 40 Pareth-40, C20-40 Pareth-95, C22-24 Pareth-33, C30-50 Pareth-40, C9-11 Pareth-6 Carboxylic Acid, C9-11 Pareth-8 Carboxylic Acid, C11-15 Pareth-7 Carboxylic Acid, C12-13 Pareth-5 Carboxylic Acid, C12-13 Pareth-7 Carboxylic Acid, C12-13 Pareth-8 Carboxylic Acid, C12-13 Pareth-12 Carboxylic Acid, C12-15 Pareth-7 Carboxylic Acid, C12-15 Pareth-8 Carboxylic Acid, C12-15 Pareth- 12 Carboxylic Acid, C14-15 Pareth-8 Carboxylic Acid, C6-10 Pareth-4 Phosphate, C12-13 Pareth-2 Phosphate, C12-13 Pareth-10 Phosphate, C12-15 Pareth-6 Phosphate, C12-15 Pareth-8 Phosphate, C12-15 Pareth-10 Phosphate, C12-16 Pareth-6 Phosphate, C4-18 Perfluoroalkylethyl Thiohydroxypropyltrimonium Chloride, Cupuassuamidopropyl Betaine, DEA-C12-13 Alkyl Sulfate, DEA-C12-15 Alkyl Sulfate, DEA-Ceteareth-2 Phosphate, DEA-Cetyl Sulfate, DEA-Cocoamphodipropionate, DEA-C12-13 Pareth-3 Sulfate, DEA-Cyclocarboxypropyloleate, DEA- Dodecylbenzenesulfonate, DEA-Isostearate, DEA-Laureth Sulfate, DEA-Lauryl Sulfate, DEA- Linoleate, DEA-Methyl Myristate Sulfonate, DEA-Myreth Sulfate, DEA-Myristate, DEA-Myristyl Sulfate, DEA-Oleth-5 Phosphate, DEA-Oleth-20 Phosphate, DEA PG-Oleate, Deceth-7 Carboxylic Acid, Deceth-7 Glucoside, Deceth-9 Phosphate, Decylamine Oxide, Decyl Betaine, Decyl Glucoside, Decyltetradeceth-30, Decyltetradecylamine Oxide, Diammonium Lauramido-MEA Sulfosuccinate, Diammonium Lauryl Sulfosuccinate, Diammonium Oleamido PEG-2 Sulfosuccinate, Dibutoxymethane, Di-Cl 2-15 Pareth-2 Phosphate, Di-Cl 2-15 Pareth-4 Phosphate, Di-Cl 2-15 Pareth-6 Phosphate, Di- C12-15 Pareth-8 Phosphate, Di-Cl 2-15 Pareth-10 Phosphate, Didodecyl Butanetetracarboxylate, Diethylamine Laureth Sulfate, Diethylhexyl Sodium Sulfosuccinate, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Dimethicone PEG-7 Phosphate, Dimethicone PEG-10 Phosphate, Dimethicone PEG/PPG-7/4 Phosphate, Dimethicone PEG/PPG-12/4 Phosphate, Dimethicone/Polyglycerin-3 Crosspolymer, Dimethicone Propyl PG- Betaine, Dimyristyl Phosphate, Dioleoylamidoethyl Hydroxyethylmonium Methosulfate, DIPA- Hydrogenated Cocoate, DIPA-Lanolate, DIPA-Myristate, Dipotassium Capryloyl Glutamate, Dipotassium Lauryl Sulfosuccinate, Dipotassium Undecylenoyl Glutamate, Disodium Babassuamido MEA-Sulfosuccinate, Disodium Caproamphodiacetate, Disodium Caproamphodipropionate, Disodium Capryloamphodiacetate, Disodium Capryloamphodipropionate, Disodium Capryloyl Glutamate, Disodium Cetearyl Sulfosuccinate, Disodium Cetyl Phenyl Ether Disulfonate, Disodium Cetyl Sulfosuccinate, Disodium Cocamido MEA-Sulfosuccinate, Disodium Cocamido MIPA PEG-4 Sulfosuccinate, Disodium Cocamido MIPA-Sulfosuccinate, Disodium Cocamido PEG-3 Sulfosuccinate, Disodium Coceth-3 Sulfosuccinate, Disodium Cocoamphocarboxyethylhydroxypropylsulfonate, Disodium Cocoamphodiacetate, Disodium Cocoamphodipropionate, Disodium Coco-Glucoside Sulfosuccinate, Disodium Coco-Sulfosuccinate, Disodium Cocoyl Butyl Gluceth-10 Sulfosuccinate, Disodium Cocoyl Glutamate, Disodium C12-14 Pareth-1 Sulfosuccinate, Disodium C12-14 Pareth-2 Sulfosuccinate, Disodium C12-15 Pareth Sulfosuccinate, Disodium C12-14 Sec-Pareth-3 Sulfosuccinate, Disodium C12-14 Sec-Pareth-5 Sulfosuccinate, Disodium C12-14 Sec-Pareth-7 Sulfosuccinate, Disodium C12-14 Sec-Pareth-9 Sulfosuccinate, Disodium C12-14 Sec-Pareth-12 Sulfosuccinate, Disodium Deceth-5 Sulfosuccinate, Disodium Deceth-6 Sulfosuccinate, Disodium Decyl Phenyl Ether Disulfonate, Disodium Dihydroxyethyl Sulfosuccinylundecylenate, Disodium Ethylene Dicocamide PEG-15 Disulfate, Disodium Hydrogenated Cottonseed Glyceride Sulfosuccinate, Disodium Hydrogenated Tallow Glutamate, Disodium Hydroxydecyl Sorbitol Citrate, Disodium Isodecyl Sulfosuccinate, Disodium Isostearamido MEA-Sulfosuccinate, Disodium Isostearamido MIPA-Sulfosuccinate, Disodium Isostearoamphodiacetate, Disodium Isostearoamphodipropionate, Disodium Isostearyl Sulfosuccinate, Disodium Laneth-5 Sulfosuccinate, Disodium Lauramido MEA-Sulfosuccinate, Disodium Lauramido MIPA Glycol Sulfosuccinate, Disodium Lauramido PEG-2 Sulfosuccinate, Disodium Lauramido PEG-5 Sulfosuccinate, Disodium Laureth-5 Carboxyamphodiacetate, Disodium Laureth-7 Citrate, Disodium Laureth Sulfosuccinate, Disodium Laureth-6 Sulfosuccinate, Disodium Laureth-9 Sulfosuccinate, Disodium Laureth-12 Sulfosuccinate, Disodium Lauriminobishydroxypropylsulfonate, Disodium Lauriminodiacetate, Disodium Lauriminodipropionate, Disodium Lauriminodipropionate Tocopheryl Phosphates, Disodium Lauroamphodiacetate, Disodium Lauroamphodipropionate, Disodium N- Lauroyl Aspartate, Disodium Lauroyl Glutamate, Disodium Lauryl Phenyl Ether Disulfonate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleoamphodipropionate, Disodium Oleth-3 Sulfosuccinate, Disodium Oleyl Phosphate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PEG-12 Dimethicone Sulfosuccinate, Disodium PEG-8 Palm Glycerides Sulfosuccinate, Disodium PPG-2-Isodeceth-7 Carboxyamphodiacetate, Disodium Ricinoleamido MEA-Sulfosuccinate, Disodium Sitostereth-14 Sulfosuccinate, Disodium Soyamphodiacetate, Disodium Stearamido MEA-Sulfosuccinate, Disodium Steariminodipropionate, Disodium Stearoamphodiacetate, Disodium Stearoyl Glutamate, Disodium Stearyl Sulfosuccinamate, Disodium Stearyl Sulfosuccinate, Disodium 2-Sulfolaurate, Disodium 2-Sulfopalmitate, Disodium Tallamido MEA-Sulfosuccinate, Disodium Tallowamido MEA-Sulfosuccinate, Disodium Tallowamphodiacetate, Disodium Tallowiminodipropionate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Undecylenoyl Glutamate, Disodium Wheat Germamido MEA-Sulfosuccinate, Disodium Wheat Germamido PEG-2 Sulfosuccinate, Disodium Wheatgermamphodiacetate, Di-TEA-Cocamide Diacetate, Di-TEA-Oleamido PEG-2 Sulfosuccinate, Di-TEA-Palmitoyl Aspartate, Ditridecyl Sodium Sulfosuccinate, Dodecylbenzene Sulfonic Acid, Erucamidopropyl Hydroxysultaine, Ethylhexeth-3 Carboxylic Acid, Ethyl PEG-15 Cocamine Sulfate, Glyceryl Capryl Ether, Hexyldecanoic Acid, Hydrogenated Coconut Acid, Hydrogenated Laneth-25, Hydrogenated Menhaden Acid, Hydrogenated Palm Acid, Hydrogenated Palm Kernel Amine Oxide, Hydrogenated Tallow Acid, Hydrogenated Tallowamine Oxide, Hydrogenated Tallow Betaine, Hydrogenated Talloweth-25, Hydrogenated Tallowoyl Glutamic Acid, Hydrolyzed Candida Bombicola Extract, Hydroxyceteth-60, Hydroxyethyl Acetomonium PG-Dimethicone, Hydroxyethylbutylamine Laureth Sulfate, Hydroxyethyl Carboxymethyl Cocamidopropylamine, Hydroxyethyl Hydroxypropyl C12-15 Alkoxypropylamine Oxide, Hydroxylauryl/Hydroxymyristyl Betaine, Hydroxystearic Acid, Hydroxysuccinimidyl C10-40 Isoalkyl Acidate, Hydroxysuccinimidyl C21-22 Isoalkyl Acidate, Hydroxysultaines, IPDI/PEG-15 Soyamine Oxide Copolymer, IPDI/PEG-15 Soyethonium Ethosulfate Copolymer, IPDI/PEG-15 Soy Glycinate Copolymer, Isoceteth-30, Isolaureth-4 Phosphate, Isopolyglyceryl-3 Dimethicone, Isopolyglyceryl-3 Dimethiconol, Isopropanolamine Lanolate, Isopropylamine Dodecylbenzenesulfonate, Isostearamidopropylamine Oxide, Isostearamidopropyl Betaine, Isostearamidopropyl Morpholine Oxide, Isosteareth-8, Isosteareth-16, Isosteareth-22, Isosteareth-25, Isosteareth-50, Isostearic Acid, Isostearoyl Hydrolyzed Collagen, Jojoba Oil PEG-150 Esters, Jojoba Wax PEG-80 Esters, Jojoba Wax PEG-120 Esters, Laneth-20, Laneth-25, Laneth-40, Laneth-50, Laneth-60, Laneth-75, Lanolin Acid, Lauramidopropylamine Oxide, Lauramidopropyl Betaine, Lauramidopropyl Hydroxysultaine, Lauramine Oxide, Lauraminopropionic Acid, Laurdimoniumhydroxypropyl Decylglucosides Chloride, Laurdimoniumhydroxypropyl Laurylglucosides Chloride, Laureth-16, Laureth-20, Laureth-21, Laureth-23, Laureth-25, Laureth-30, Laureth-38, Laureth-40, Laureth-3 Carboxylic Acid, Laureth-4 Carboxylic Acid, Laureth-5 Carboxylic Acid, Laureth- 6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, Laureth-6 Citrate, Laureth-7 Citrate, Laureth-1 Phosphate, Laureth-2 Phosphate, Laureth-3 Phosphate, Laureth-4 Phosphate, Laureth-7 Phosphate, Laureth-8 Phosphate, Laureth-7 Tartrate, Laurie Acid, Laurimino Bispropanediol, Lauriminodipropionic Acid, Lauroamphodipropionic Acid, Lauroyl Beta-Alanine, Lauroyl Collagen Amino Acids, Lauroyl Ethyltrimonium Methosulfate, Lauroyl Hydrolyzed Collagen, Lauroyl Hydrolyzed Elastin, Lauroyl Methyl Glucamide, Lauroyl Sarcosine, Lauroyl Silk Amino Acids, Lauryl Betaine, Lauryl Dimethicone/Polyglycerin-3 Crosspolymer, Lauryldimoniumhydroxypropyl Cocoglucosides Chloride, Lauryl Glucoside, Laurylglucosides Hydroxypropyltrimonium Chloride, Lauryl Glycol Hydroxypropyl Ether, Lauryl Hydroxysultaine, Lauryl Malamide, Lauryl Methylglucamide, Lauryl/Myristyl Glycol Hydroxypropyl Ether, Lauryl/Myristyl Wheat Bran/Straw Glycosides, Lauryl Polyglyceryl-3 Polydimethylsiloxyethyl Dimethicone, Lauryl Pyrrolidone, Lauryl Sultaine, Linoleic Acid, Linolenic Acid, Linseed Acid, Lysine Cocoate, Macadamia Seed Oil Glycereth-8 Esters, Magnesium Coceth Sulfate, Magnesium Coco-Sulfate, Magnesium Isododecylbenzenesulfonate, Magnesium Laureth-11 Carboxylate, Magnesium Laureth Sulfate, Magnesium Laureth-5 Sulfate, Magnesium Laureth-8 Sulfate, Magnesium Laureth-16 Sulfate, Magnesium Laureth-3 Sulfosuccinate, Magnesium Lauryl Hydroxypropyl Sulfonate, Magnesium Lauryl Sulfate, Magnesium Methyl Cocoyl Taurate, Magnesium Myreth Sulfate, Magnesium Oleth Sulfate, Magnesium/TEA-Coco-Sulfate, Manicouagan Clay, MEA-Cocoate, MEA-Laureth-6 Carboxylate, MEA- Laureth Sulfate, MEA-Lauryl Sulfate, MEA PPG-6 Laureth-7 Carboxylate, MEA-PPG-8-Steareth-7 Carboxylate, MEA-Undecylenate, Meroxapol 108, Meroxapol 174, Meroxapol 178, Meroxapol 254, Meroxapol 255, Meroxapol 258, Meroxapol 314, Methoxy PEG-450 Amidoglutaroyl Succinimide, Methoxy PEG-450 Amido Hydroxysuccinimidyl Succinamate, Methoxy PEG-450 Maleimide, Methyl Morpholine Oxide, Milkamidopropyl Amine Oxide, Milkamidopropyl Betaine, Minkamidopropylamine Oxide, Minkamidopropyl Betaine, MIPA C12-15 Pareth Sulfate, MIPA-Dodecylbenzenesulfonate, MIPA-Laureth Sulfate, MIPA-Lauryl Sulfate, Mixed Isopropanolamines Lanolate, Mixed Isopropanolamines Lauryl Sulfate, Mixed Isopropanolamines Myristate, Morpholine Oleate, Morpholine Stearate, Myreth-3 Carboxylic Acid, Myreth-5 Carboxylic Acid, Myristalkonium Chloride, Myristamidopropylamine Oxide, Myristamidopropyl Betaine, Myristamidopropyl Dimethylamine Phosphate, Myristamidopropyl Hydroxysultaine, Myristamidopropyl PG-Dimonium Chloride Phosphate, Myristamine Oxide, Myristaminopropionic Acid, Myristic Acid, Myristoyl Ethyltrimonium Methosulfate, Myristoyl Glutamic Acid, Myristoyl Hydrolyzed Collagen, Myristoyl Sarcosine, Myristyl Betaine, Myristyl/Cetyl Amine Oxide, Myristyldimoniumhydroxypropyl Cocoglucosides Chloride, Myristyl Glucoside, Myristyl Phosphate, Nonoxynol-20, Nonoxynol-23, Nonoxynol-25, Nonoxynol-30, Nonoxynol-35, Nonoxynol-40, Nonoxynol-44, Nonoxynol-50, Nonoxynol-100, Nonoxynol-120, Nonoxynol-5 Carboxylic Acid, Nonoxynol-8 Carboxylic Acid, Nonoxynol-10 Carboxylic Acid, Nonoxynol-3 Phosphate, Nonoxynol-4 Phosphate, Nonoxynol-6 Phosphate, Nonoxynol-9 Phosphate, Nonoxynol-10 Phosphate, Nonyl Nonoxynol-30, Nonyl Nonoxynol-49, Nonyl Nonoxynol-100, Nonyl Nonoxynol-150, Nonyl Nonoxynol-7 Phosphate, Nonyl Nonoxynol-8 Phosphate, Nonyl Nonoxynol-9 Phosphate, Nonyl Nonoxynol-10 Phosphate, Nonyl Nonoxynol-11 Phosphate, Nonyl Nonoxynol-15 Phosphate, Nonyl Nonoxynol-24 Phosphate, Oatamidopropyl Betaine, Octoxynol-16, Octoxynol-25, Octoxynol-30, Octoxynol-33, Octoxynol-40, Octoxynol-70, Octoxynol-20 Carboxylic Acid, Octyldodeceth-20, Octyldodeceth-25, Octyldodeceth-30, Oleamidopropylamine Oxide, Oleamidopropyl Betaine, Oleamidopropyl Hydroxysultaine, Oleamine Oxide, Oleic Acid, Oleoyl Hydrolyzed Collagen, Oleoyl Sarcosine, Oleth-20, Oleth-23, Oleth-24, Oleth-25, Oleth-30, Oleth-35, Oleth-40, Oleth-44, Oleth-50, Oleth-3 Carboxylic Acid, Oleth-6 Carboxylic Acid, Oleth-10 Carboxylic Acid, Oleyl Betaine, Olivamidopropylamine Oxide, Olivamidopropyl Betaine, Olive Acid, Olivoyl Hydrolyzed Wheat Protein, Ophiopogon Extract Stearate, Ozonized Oleth-10, Ozonized PEG-10 Oleate, Ozonized PEG-14 Oleate, Ozonized Polysorbate 80, Palm Acid, Palmamidopropyl Betaine, Palmeth-2 Phosphate, Palmitamidopropylamine Oxide, Palmitamidopropyl Betaine, Palmitamine Oxide, Palmitic Acid, Palmitoyl Collagen Amino Acids, Palmitoyl Glycine, Palmitoyl Hydrolyzed Collagen, Palmitoyl Hydrolyzed Milk Protein, Palmitoyl Hydrolyzed Wheat Protein, Palmitoyl Keratin Amino Acids, Palmitoyl Oligopeptide, Palmitoyl Silk Amino Acids, Palm Kernel Acid, Palm Kernelamidopropyl Betaine, Peach Kernel Oil Glycereth-8 Esters, Peanut Acid, PEG-10 Castor Oil, PEG-40 Castor Oil, PEG-44 Castor Oil, PEG-50 Castor Oil, PEG-54 Castor Oil, PEG-55 Castor Oil, PEG-60 Castor Oil, PEG-80 Castor Oil, PEG-100 Castor Oil, PEG-200 Castor Oil, PEG-11 Cocamide, PEG-6 Cocamide Phosphate, PEG-4 Cocamine, PEG-8 Cocamine, PEG-12 Cocamine, PEG-150 Dibehenate, PEG-90 Diisostearate, PEG-75 Dilaurate, PEG-150 Dilaurate, PEG-75 Dioleate, PEG-150 Dioleate, PEG-75 Distearate, PEG-120 Distearate, PEG-150 Distearate, PEG-175 Distearate, PEG-190 Distearate, PEG-250 Distearate, PEG-30 Glyceryl Cocoate, PEG-40 Glyceryl Cocoate, PEG-78 Glyceryl Cocoate, PEG-80 Glyceryl Cocoate, PEG-30 Glyceryl Isostearate, PEG-40 Glyceryl Isostearate, PEG-50 Glyceryl Isostearate, PEG-60 Glyceryl Isostearate, PEG-90 Glyceryl Isostearate, PEG-23 Glyceryl Laurate, PEG-30 Glyceryl Laurate, PEG-25 Glyceryl Oleate, PEG-30 Glyceryl Oleate, PEG-30 Glyceryl Soyate, PEG-25 Glyceryl Stearate, PEG-30 Glyceryl Stearate, PEG-40 Glyceryl Stearate, PEG-120 Glyceryl Stearate, PEG-200 Glyceryl Stearate, PEG-28 Glyceryl Tallowate, PEG-80 Glyceryl Tallowate, PEG-82 Glyceryl Tallowate, PEG-130 Glyceryl Tallowate, PEG-200 Glyceryl Tallowate, PEG-45 Hydrogenated Castor Oil, PEG-50 Hydrogenated Castor Oil, PEG-54 Hydrogenated Castor Oil, PEG-55 Hydrogenated Castor Oil, PEG-60 Hydrogenated Castor Oil, PEG-80 Hydrogenated Castor Oil, PEG-100 Hydrogenated Castor Oil, PEG-200 Hydrogenated Castor Oil, PEG-30 Hydrogenated Lanolin, PEG-70 Hydrogenated Lanolin, PEG-50 Hydrogenated Palmamide, PEG-2 Isostearate, PEG-3 Isostearate, PEG-4 Isostearate, PEG-6 Isostearate, PEG-8 Isostearate, PEG-10 Isostearate, PEG-12 Isostearate, PEG-20 Isostearate, PEG-30 Isostearate, PEG-40 Isostearate, PEG- 26 Jojoba Acid, PEG-40 Jojoba Acid, PEG-15 Jojoba Alcohol, PEG-26 Jojoba Alcohol, PEG-40 Jojoba Alcohol, PEG-35 Lanolin, PEG-40 Lanolin, PEG-50 Lanolin, PEG-55 Lanolin, PEG-60 Lanolin, PEG- 70 Lanolin, PEG-75 Lanolin, PEG-85 Lanolin, PEG-100 Lanolin, PEG-150 Lanolin, PEG-75 Lanolin Oil, PEG-2 Lauramide, PEG-3 Lauramine Oxide, PEG-20 Laurate, PEG-32 Laurate, PEG-75 Laurate, PEG-150 Laurate, PEG-70 Mango Glycerides, PEG-20 Mannitan Laurate, PEG-8 Methyl Ether Dimethicone, PEG-120 Methyl Glucose Dioleate, PEG-80 Methyl Glucose Laurate, PEG-120 Methyl Glucose Trioleate, PEG-4 Montanate, PEG-30 Oleamine, PEG-20 Oleate, PEG-23 Oleate, PEG-32 Oleate, PEG-36 Oleate, PEG-75 Oleate, PEG-150 Oleate, PEG-20 Palmitate, PEG-150 Polyglyceryl-2 Tristearate, PEG/PPG-28/21 Acetate Dimethicone, PEG/PPG-24/18 Butyl Ether Dimethicone, PEG/PPG-3/17 Copolymer, PEG/PPG-5/35 Copolymer, PEG/PPG-8/55 Copolymer, PEG/PPG-10/30 Copolymer, PEG/PPG-10/65 Copolymer, PEG/PPG-12/35 Copolymer, PEG/PPG-16/17 Copolymer, PEG/PPG-20/9 Copolymer, PEG/PPG-20/20 Copolymer, PEG/PPG-20/60 Copolymer, PEG/PPG- 20/65 Copolymer, PEG/PPG-22/25 Copolymer, PEG/PPG-28/30 Copolymer, PEG/PPG-30-35 Copolymer, PEG/PPG-30/55 Copolymer, PEG/PPG-35/40 Copolymer, PEG/PPG-50/40 Copolymer, PEG/PPG-150/35 Copolymer, PEG/PPG-160/30 Copolymer, PEG/PPG-190/60 Copolymer, PEG/PPG-200/40 Copolymer, PEG/PPG-300/55 Copolymer, PEG/PPG-20/22 Methyl Ether Dimethicone, PEG-26-PPG-30 Phosphate, PEG/PPG-4/2 Propylheptyl Ether, PEG/PPG-6/2 Propylheptyl Ether, PEG-7/PPG-2 Propylheptyl Ether, PEG/PPG-8/2 Propylheptyl Ether, PEG/PPG- 10/2 Propylheptyl Ether, PEG/PPG-14/2 Propylheptyl Ether, PEG/PPG-40/2 Propylheptyl Ether, PEG/PPG-10/2 Ricinoleate, PEG/PPG-32/3 Ricinoleate, PEG-55 Propylene Glycol Oleate, PEG-25 Propylene Glycol Stearate, PEG-75 Propylene Glycol Stearate, PEG-120 Propylene Glycol Stearate, PEG-5 Rapeseed Sterol, PEG-10 Rapeseed Sterol, PEG-40 Ricinoleamide, PEG-75 Shea Butter Glycerides, PEG-75 Shorea Butter Glycerides, PEG-20 Sorbitan Cocoate, PEG-20 Sorbitan Isostearate, PEG-40 Sorbitan Lanolate, PEG-75 Sorbitan Lanolate, PEG-10 Sorbitan Laurate, PEG-40 Sorbitan Laurate, PEG-44 Sorbitan Laurate, PEG-75 Sorbitan Laurate, PEG-80 Sorbitan Laurate, PEG-20 Sorbitan Oleate, PEG-80 Sorbitan Palmitate, PEG-40 Sorbitan Stearate, PEG-60 Sorbitan Stearate, PEG-160 Sorbitan Triisostearate, PEG-40 Soy Sterol, PEG-2 Stearamide Carboxylic Acid, PEG-9 Stearamide Carboxylic Acid, PEG-20 Stearate, PEG-23 Stearate, PEG-25 Stearate, PEG-30 Stearate, PEG-32 Stearate, PEG-35 Stearate, PEG-36 Stearate, PEG-40 Stearate, PEG-45 Stearate, PEG-50 Stearate, PEG-55 Stearate, PEG-75 Stearate, PEG-90 Stearate, PEG-100 Stearate, PEG- 120 Stearate, PEG-150 Stearate, PEG-45 Stearate Phosphate, PEG-20 Tallate, PEG-50 Tallow Amide, PEG-2 Tallowamide DEA, PEG-20 Tallowate, PEG-66 Trihydroxystearin, PEG-200 Trihydroxystearin, PEG-60 Tsubakiate Glycerides, Pelargonic Acid, Pentadoxynol-200, Pheneth-6 Phosphate, Poloxamer 105, Poloxamer 108, Poloxamer 182, Poloxamer 183, Poloxamer 184, Poloxamer 188, Poloxamer 217, Poloxamer 234, Poloxamer 235, Poloxamer 237, Poloxamer 238, Poloxamer 288, Poloxamer 334, Poloxamer 335, Poloxamer 338, Poloxamine 908, Poloxamine 1508, Polydimethylsiloxy PEG/PPG-24/19 Butyl Ether Silsesquioxane, Polydimethylsiloxy PPG-13 Butyl Ether Silsesquioxane, Polyglyceryl-6 Caprate, Polyglyceryl-10 Dilaurate, Polyglyceryl-20 Heptacaprylate, Polyglyceryl-20 Hexacaprylate, Polyglyceryl-2 Lauryl Ether, Polyglyceryl-10 Lauryl Ether, Polyglyceryl-20 Octaisononanoate, Polyglyceryl-6 Pentacaprylate, Polyglyceryl-10 Pentacaprylate, Polyglyceryl-3 Polydimethylsiloxyethyl Dimethicone, Polyglyceryl-6 Tetracaprylate, Polyglyceryl-10 Tetralaurate, Polyglyceryl-6 Tricaprylate, Polyglyceryl-10 Trilaurate, Polyquaternium- 77, Polyquaternium-78, Polyquaternium-79, Polyquaternium-80, Polyquaternium-81, Polyquaternium- 82, Pomaderris Kumerahou Flower/Leaf Extract, Poria Cocos Extract, Potassium Abietoyl Hydrolyzed Collagen, Potassium Babassuate, Potassium Behenate, Potassium C9-15 Alkyl Phosphate, Potassium C11-15 Alkyl Phosphate, Potassium C12-13 Alkyl Phosphate, Potassium C12-14 Alkyl Phosphate, Potassium Caprate, Potassium Capryloyl Glutamate, Potassium Capryloyl Hydrolyzed Rice Protein, Potassium Castorate, Potassium Cocoate, Potassium Cocoyl Glutamate, Potassium Cocoyl Glycinate, Potassium Cocoyl Hydrolyzed Casein, Potassium Cocoyl Hydrolyzed Collagen, Potassium Cocoyl Hydrolyzed Corn Protein, Potassium Cocoyl Hydrolyzed Keratin, Potassium Cocoyl Hydrolyzed Oat Protein, Potassium Cocoyl Hydrolyzed Potato Protein, Potassium Cocoyl Hydrolyzed Rice Bran Protein, Potassium Cocoyl Hydrolyzed Rice Protein, Potassium Cocoyl Hydrolyzed Silk, Potassium Cocoyl Hydrolyzed Soy Protein, Potassium Cocoyl Hydrolyzed Wheat Protein, Potassium Cocoyl Hydrolyzed Yeast Protein, Potassium Cocoyl PCA, Potassium Cocoyl Sarcosinate, Potassium Cocoyl Taurate, Potassium Cornate, Potassium Cyclocarboxypropyloleate, Potassium Dihydroxyethyl Cocamine Oxide Phosphate, Potassium Dimethicone PEG-7 Phosphate, Potassium Dodecylbenzenesulfonate, Potassium Hempseedate, Potassium Hydrogenated Cocoate, Potassium Hydrogenated Palmate, Potassium Hydrogenated Tallowate, Potassium Hydroxystearate, Potassium Isostearate, Potassium Lanolate, Potassium Laurate, Potassium Laureth-3 Carboxylate, Potassium Laureth-4 Carboxylate, Potassium Laureth-5 Carboxylate, Potassium Laureth-6 Carboxylate, Potassium Laureth-10 Carboxylate, Potassium Laureth Phosphate, Potassium Lauroyl Collagen Amino Acids, Potassium Lauroyl Glutamate, Potassium Lauroyl Hydrolyzed Collagen, Potassium Lauroyl Hydrolyzed Pea Protein, Potassium Lauroyl Hydrolyzed Soy Protein, Potassium Lauroyl PCA, Potassium Lauroyl Pea Amino Acids, Potassium Lauroyl Sarcosinate, Potassium Lauroyl Silk Amino Acids, Potassium Lauroyl Wheat Amino Acids, Potassium Lauryl Phosphate, Potassium Lauryl Sulfate, Potassium Linoleate, Potassium Metaphosphate, Potassium Methyl Cocoyl Taurate, Potassium Myristate, Potassium Myristoyl Glutamate, Potassium Myristoyl Hydrolyzed Collagen, Potassium Octoxynol-12 Phosphate, Potassium Oleate, Potassium Oleoyl Hydrolyzed Collagen, Potassium Olivate, Potassium Olivoyl Hydrolyzed Oat Protein, Potassium Olivoyl Hydrolyzed Wheat Protein, Potassium Olivoyl/Lauroyl Wheat Amino Acids, Potassium Olivoyl PCA, Potassium Palmate, Potassium Palmitate, Potassium Palmitoyl Hydrolyzed Corn Protein, Potassium Palmitoyl Hydrolyzed Oat Protein, Potassium Palmitoyl Hydrolyzed Rice Protein, Potassium Palmitoyl Hydrolyzed Sweet Almond Protein, Potassium Palmitoyl Hydrolyzed Wheat Protein, Potassium Palm Kernelate, Potassium Peanutate, Potassium Rapeseedate, Potassium Ricinoleate, Potassium Safflowerate, Potassium Soyate, Potassium Stearate, Potassium Stearoyl Hydrolyzed Collagen, Potassium Tallate, Potassium Tallowate, Potassium Taurate, Potassium Taurine Laurate, Potassium Trideceth-3 Carboxylate, Potassium Trideceth-4 Carboxylate, Potassium Trideceth-7 Carboxylate, Potassium Trideceth-15 Carboxylate, Potassium Trideceth-19 Carboxylate, Potassium Trideceth-6 Phosphate, Potassium Trideceth-7 Phosphate, Potassium Tsubakiate, Potassium Undecylenate, Potassium Undecylenoyl Hydrolyzed Collagen, Potassium Undecylenoyl Hydrolyzed Rice Protein, PPG-30- Buteth-30, PPG-36-Buteth-36, PPG-38-Buteth-37, PPG-30-Capryleth-4 Phosphate, PPG-10 Cetyl Ether Phosphate, PPG-2 C9-11 Pareth-8, PPG-1-Deceth-5, PPG-3-Deceth-2 Carboxylic Acid, PPG-30 Ethylhexeth-4 Phosphate, PPG-20-Glycereth-30, PPG-2 Hydroxyethyl Coco/Isostearamide, PPG-2- Isodeceth-8, PPG-2-Isodeceth-10, PPG-2-Isodeceth-18, PPG-2-Isodeceth-25, PPG-4-Isodeceth-10, Propyltrimonium Hydrolyzed Collagen, Quaternium-24, Quaternium-52, Quaternium-87, Rapeseed Acid, Rice Bran Acid, Rice Oil Glycereth-8 Esters, Ricinoleamidopropyl Betaine, Ricinoleic Acid, Ricinoleth-40, Safflower Acid, Sapindus Oahuensis Fruit Extract, Saponaria Officinalis Root Powder, Saponins, Sekken-K, Sekken-Na/K, Sekken Soji, Sekken Soji-K, Sesame Oil Glycereth-8 Esters, Sesamidopropylamine Oxide, Sesamidopropyl Betaine, Shea Butteramidopropyl Betaine, Shea Butter Glycereth-8 Esters, Sodium Arachidate, Sodium Arganampohoacetate, Sodium Astrocaryum Murumuruate, Sodium Avocadoate, Sodium Babassuamphoacetate, Sodium Babassuate, Sodium Babassu Sulfate, Sodium Behenate, Sodium Bisglycol Ricinosulfosuccinate, Sodium Bis- Hydroxyethylglycinate Coco-Glucosides Crosspolymer, Sodium Bis-Hydroxyethylglycinate Lauryl- Glucosides Crosspolymer, Sodium Borageamidopropyl PG-Dimonium Chloride Phosphate, Sodium Butoxynol-12 Sulfate, Sodium Butylglucosides Hydroxypropyl Phosphate, Sodium C13-17 Alkane Sulfonate, Sodium C14-18 Alkane Sulfonate, Sodium C12-15 Alkoxypropyl Iminodipropionate, Sodium C10-16 Alkyl Sulfate, Sodium C11-15 Alkyl Sulfate, Sodium C12-13 Alkyl Sulfate, Sodium C12-15 Alkyl Sulfate, Sodium C12-18 Alkyl Sulfate, Sodium C16-20 Alkyl Sulfate, Sodium C9-22 Alkyl Sec Sulfonate, Sodium C14-17 Alkyl Sec Sulfonate, Sodium Caprate, Sodium Caproamphoacetate, Sodium Caproamphohydroxypropylsulfonate, Sodium Caproamphopropionate, Sodium Caproyl Methyltaurate, Sodium Caprylate, Sodium Capryleth-2 Carboxylate, Sodium Capryleth-9 Carboxylate, Sodium Capryloamphoacetate, Sodium Capryloamphohydroxypropylsulfonate, Sodium Capryloamphopropionate, Sodium Capryloyl Glutamate, Sodium Capryloyl Hydrolyzed Wheat Protein, Sodium Caprylyl PG-Sulfonate, Sodium Caprylyl Sulfonate, Sodium Castorate, Sodium Ceteareth-13 Carboxylate, Sodium Cetearyl Sulfate, Sodium Ceteth-13 Carboxylate, Sodium Cetyl Sulfate, Sodium Cocamidopropyl PG-Dimonium Chloride Phosphate, Sodium Cocaminopropionate, Sodium Coceth Sulfate, Sodium Coceth-30 Sulfate, Sodium Cocoabutteramphoacetate, Sodium Cocoa Butterate, Sodium Cocoamphoacetate, Sodium Cocoamphohydroxypropylsulfonate, Sodium Cocoamphopropionate, Sodium Cocoate, Sodium Coco/Babassu/Andiroba Sulfate, Sodium Coco/Babassu Sulfate, Sodium Cocoglucosides Hydroxypropyl Phosphate, Sodium Cocoglucosides Hydroxypropylsulfonate, Sodium Coco-Glucoside Tartrate, Sodium Cocoglyceryl Ether Sulfonate, Sodium Coco/Hydrogenated Tallow Sulfate, Sodium Cocoiminodiacetate, Sodium Cocomonoglyceride Sulfate, Sodium Cocomonoglyceride Sulfonate, Sodium Coco PG-Dimonium Chloride Phosphate, Sodium Coco-Sulfate, Sodium Coco Sulfoacetate, Sodium Cocoyl Alaninate, Sodium Cocoyl Amino Acids, Sodium Cocoyl Collagen Amino Acids, Sodium Cocoyl Glutamate, Sodium Cocoyl Glutaminate, Sodium Cocoyl Glycinate, Sodium Cocoyl/Hydrogenated Tallow Glutamate, Sodium Cocoyl Hydrolyzed Collagen, Sodium Cocoyl Hydrolyzed Keratin, Sodium Cocoyl Hydrolyzed Rice Protein, Sodium Cocoyl Hydrolyzed Silk, Sodium Cocoyl Hydrolyzed Soy Protein, Sodium Cocoyl Hydrolyzed Sweet Almond Protein, Sodium Cocoyl Hydrolyzed Wheat Protein, Sodium Cocoyl Hydrolyzed Wheat Protein Glutamate, Sodium Cocoyl Isethionate, Sodium Cocoyl Methylaminopropionate, Sodium Cocoyl Oat Amino Acids, Sodium Cocoyl/Palmoyl/Sunfloweroyl Glutamate, Sodium Cocoyl Proline, Sodium Cocoyl Sarcosinate, Sodium Cocoyl Taurate, Sodium Cocoyl Threoninate, Sodium Cocoyl Wheat Amino Acids, Sodium C12-14 Olefin Sulfonate, Sodium C14-16 Olefin Sulfonate, Sodium C14- 18 Olefin Sulfonate, Sodium C16-18 Olefin Sulfonate, Sodium Cornamphopropionate, Sodium Cottonseedamphoacetate, Sodium C13-15 Pareth-8 Butyl Phosphate, Sodium C9-11 Pareth-6 Carboxylate, Sodium C11-15 Pareth-7 Carboxylate, Sodium C12-13 Pareth-5 Carboxylate, Sodium C12-13 Pareth-8 Carboxylate, Sodium C12-13 Pareth-12 Carboxylate, Sodium C12-15 Pareth-6 Carboxylate, Sodium C12-15 Pareth-7 Carboxylate, Sodium C12-15 Pareth-8 Carboxylate, Sodium C14-15 Pareth-8 Carboxylate, Sodium C12-14 Sec-Pareth-8 Carboxylate, Sodium C14-15 Pareth-PG Sulfonate, Sodium C12-13 Pareth-2 Phosphate, Sodium C13-15 Pareth-8 Phosphate, Sodium C9-15 Pareth-3 Sulfate, Sodium C10-15 Pareth Sulfate, Sodium C10-16 Pareth-2 Sulfate, Sodium C12-13 Pareth Sulfate, Sodium C12-15 Pareth Sulfate, Sodium C12-15 Pareth-3 Sulfate, Sodium C13-15 Pareth-3 Sulfate, Sodium C12-14 Sec-Pareth-3 Sulfate, Sodium C12-15 Pareth-3 Sulfonate, Sodium C12-15 Pareth-7 Sulfonate, Sodium C12-15 Pareth-15 Sulfonate, Sodium Deceth-2 Carboxylate, Sodium Deceth Sulfate, Sodium Decylbenzenesulfonate, Sodium Decylglucosides Hydroxypropyl Phosphate, Sodium Decylglucosides Hydroxypropylsulfonate, Sodium Dilaureth-7 Citrate, Sodium Dilaureth-10 Phosphate, Sodium Dilinoleamidopropyl PG-Dimonium Chloride Phosphate, Sodium Dilinoleate, Sodium Dioleth-8 Phosphate, Sodium Dodecylbenzenesulfonate, Sodium Ethyl 2- Sulfolaurate, Sodium Glyceryl Oleate Phosphate, Sodium Grapeseedamidopropyl PG-Dimonium Chloride Phosphate, Sodium Grapeseedamphoacetate, Sodium Grapeseedate, Sodium Hempseedamphoacetate, Sodium Hexeth-4 Carboxylate, Sodium Hydrogenated Cocoate, Sodium Hydrogenated Cocoyl Methyl Isethionate, Sodium Hydrogenated Palmate, Sodium Hydrogenated Tallowate, Sodium Hydrogenated Tallowoyl Glutamate, Sodium Hydroxylauryldimonium Ethyl Phosphate, Sodium Hydroxypropyl Palm Kernelate Sulfonate, Sodium Hydroxypropylphosphate Decylglucoside Crosspolymer, Sodium Hydroxypropylphosphate Laurylglucoside Crosspolymer, Sodium Hydroxypropylsulfonate Cocoglucoside Crosspolymer, Sodium Hydroxypropylsulfonate Decylglucoside Crosspolymer, Sodium Hydroxypropylsulfonate Laurylglucoside Crosspolymer, Sodium Hydroxystearate, Sodium Isostearate, Sodium Isosteareth-6 Carboxylate, Sodium Isosteareth- 11 Carboxylate, Sodium Isostearoamphoacetate, Sodium Isostearoamphopropionate, Sodium N-Isostearoyl Methyltaurate, Sodium Laneth Sulfate, Sodium Lanolate, Sodium Lardate, Sodium Lauramido Diacetate, Sodium Lauraminopropionate, Sodium Laurate, Sodium Laureth-3 Carboxylate, Sodium Laureth-4 Carboxylate, Sodium Laureth-5 Carboxylate, Sodium Laureth-6 Carboxylate, Sodium Laureth-8 Carboxylate, Sodium Laureth-11 Carboxylate, Sodium Laureth-12 Carboxylate, Sodium Laureth-13 Carboxylate, Sodium Laureth-14 Carboxylate, Sodium Laureth-16 Carboxylate, Sodium Laureth-17 Carboxylate, Sodium Laureth Sulfate, Sodium Laureth-5 Sulfate, Sodium Laureth-7 Sulfate, Sodium Laureth-8 Sulfate, Sodium Laureth-12 Sulfate, Sodium Laureth-40 Sulfate, Sodium Laureth-7 Tartrate, Sodium Lauriminodipropionate, Sodium Lauroamphoacetate, Sodium Lauroamphohydroxypropylsulfonate, Sodium Lauroampho PG-Acetate Phosphate, Sodium Lauroamphopropionate, Sodium Lauroyl Aspartate, Sodium Lauroyl Collagen Amino Acids, Sodium Lauroyl Glycine Propionate, Sodium Lauroyl Hydrolyzed Collagen, Sodium Lauroyl Hydrolyzed Silk, Sodium Lauroyl Hydroxypropyl Sulfonate, Sodium Lauroyl Isethionate, Sodium Lauroyl Methylaminopropionate, Sodium Lauroyl Methyl Isethionate, Sodium Lauroyl Millet Amino Acids, Sodium Lauroyl/Myristoyl Aspartate, Sodium Lauroyl Oat Amino Acids, Sodium Lauroyl Sarcosinate, Sodium Lauroyl Silk Amino Acids, Sodium Lauroyl Taurate, Sodium Lauroyl Wheat Amino Acids, Sodium Lauryl Diethylenediaminoglycinate, Sodium Lauryl Glucose Carboxylate, Sodium Laurylglucosides Hydroxypropyl Phosphate, Sodium Laurylglucosides Hydroxypropylsulfonate, Sodium Lauryl Glycol Carboxylate, Sodium Lauryl Hydroxyacetamide Sulfate, Sodium Lauryl Phosphate, Sodium Lauryl Sulfate, Sodium Lauryl Sulfoacetate, Sodium Linoleate, Sodium Macadamiaseedate, Sodium Mangoamphoacetate, Sodium Mangoseedate, Sodium/MEA Laureth-2 Sulfosuccinate, Sodium Methoxy PPG-2 Acetate, Sodium Methyl Cocoyl Taurate, Sodium Methyl Lauroyl Taurate, Sodium Methyl Myristoyl Taurate, Sodium Methyl Oleoyl Taurate, Sodium Methyl Palmitoyl Taurate, Sodium Methyl Stearoyl Taurate, Sodium Methyl 2-Sulfolaurate, Sodium Methyl 2- Sulfopalmitate, Sodium Methyltaurate Isopalmitamide, Sodium Methyltaurine Cocoyl Methyltaurate, Sodium Myreth Sulfate, Sodium Myristate, Sodium Myristoamphoacetate, Sodium Myristoyl Glutamate, Sodium Myristoyl Hydrolyzed Collagen, Sodium Myristoyl Isethionate, Sodium Myristoyl Sarcosinate, Sodium Myristyl Sulfate, Sodium Nonoxynol-6 Phosphate, Sodium Nonoxynol-9 Phosphate, Sodium Nonoxynol-1 Sulfate, Sodium Nonoxynol-3 Sulfate, Sodium Nonoxynol-4 Sulfate, Sodium Nonoxynol-6 Sulfate, Sodium Nonoxynol-8 Sulfate, Sodium Nonoxynol-10 Sulfate, Sodium Nonoxynol-25 Sulfate, Sodium Octoxynol-2 Ethane Sulfonate, Sodium Octoxynol-2 Sulfate, Sodium Octoxynol-6 Sulfate, Sodium Octoxynol-9 Sulfate, Sodium Oleate, Sodium Oleoamphoacetate, Sodium Oleoamphohydroxypropylsulfonate, Sodium Oleoamphopropionate, Sodium Oleoyl Hydrolyzed Collagen, Sodium Oleoyl Isethionate, Sodium Oleth Sulfate, Sodium Oleyl Methyl Isethionate, Sodium Oleyl Sulfate, Sodium Olivamphoacetate, Sodium Olivate, Sodium Olivoyl Glutamate, Sodium Palmamphoacetate, Sodium Palmate, Sodium Palm Glyceride Sulfonate, Sodium Palmitate, Sodium Palmitoyl Hydrolyzed Collagen, Sodium Palmitoyl Hydrolyzed Wheat Protein, Sodium Palmitoyl Sarcosinate, Sodium Palm Kernelate, Sodium Palm Kerneloyl Isethionate, Sodium Palmoyl Glutamate, Sodium Passiflora Edulis Seedate, Sodium Peanutamphoacetate, Sodium Peanutate, Sodium PEG-6 Cocamide Carboxylate, Sodium PEG-8 Cocamide Carboxylate, Sodium PEG-4 Cocamide Sulfate, Sodium PEG-3 Lauramide Carboxylate, Sodium PEG-4 Lauramide Carboxylate, Sodium PEG-8 Palm Glycerides Carboxylate, Sodium Pentaerythrityl Hydroxypropyl Iminodiacetate Dendrimer, Sodium Propoxy PPG-2 Acetate, Sodium Rapeseedate, Sodium Ricebranamphoacetate, Sodium Ricinoleate, Sodium Ricinoleoamphoacetate, Sodium Rose Hipsamphoacetate, Sodium Rosinate, Sodium Safflowerate, Sodium Saffloweroyl Hydrolyzed Soy Protein, Sodium Sesameseedate, Sodium Sesamphoacetate, Sodium Sheabutteramphoacetate, Sodium Soyate, Sodium Soy Hydrolyzed Collagen, Sodium Stearate, Sodium Stearoamphoacetate, Sodium Stearoamphohydroxypropylsulfonate, Sodium Stearoamphopropionate, Sodium Stearoyl Casein, Sodium Stearoyl Glutamate, Sodium Stearoyl Hyaluronate, Sodium Stearoyl Hydrolyzed Collagen, Sodium Stearoyl Hydrolyzed Corn Protein, Sodium Stearoyl Hydrolyzed Silk, Sodium Stearoyl Hydrolyzed Soy Protein, Sodium Stearoyl Hydrolyzed Wheat Protein, Sodium Stearoyl Lactalbumin, Sodium Stearoyl Methyl Isethionate, Sodium Stearoyl Oat Protein, Sodium Stearoyl Pea Protein, Sodium Stearoyl Soy Protein, Sodium Stearyl Dimethyl Glycine, Sodium Stearyl Sulfate, Sodium Sunflowerseedamphoacetate, Sodium Surfactin, Sodium Sweetalmondamphoacetate, Sodium Sweet Almondate, Sodium Tallamphopropionate, Sodium Tallate, Sodium Tallowamphoacetate, Sodium Tallowate, Sodium Tallow Sulfate, Sodium Tamanuseedate, Sodium Taurate, Sodium Taurine Cocoyl Methyltaurate, Sodium Taurine Laurate, Sodium/TEA-Lauroyl Collagen Amino Acids, Sodium/TEA-Lauroyl Hydrolyzed Collagen, Sodium/TEA-Lauroyl Hydrolyzed Keratin, Sodium/TEA- Lauroyl Keratin Amino Acids, Sodium/TEA-Undecylenoyl Collagen Amino Acids, Sodium/TEA- Undecylenoyl Hydrolyzed Collagen, Sodium/TEA-Undecylenoyl Hydrolyzed Corn Protein, Sodium/TEA-Undecylenoyl Hydrolyzed Soy Protein, Sodium/TEA-Undecylenoyl Hydrolyzed Wheat Protein, Sodium Theobroma Grandiflorum Seedate, Sodium Trideceth-3 Carboxylate, Sodium Trideceth-4 Carboxylate, Sodium Trideceth-6 Carboxylate, Sodium Trideceth-7 Carboxylate, Sodium Trideceth-8 Carboxylate, Sodium Trideceth-12 Carboxylate, Sodium Trideceth-15 Carboxylate, Sodium Trideceth-19 Carboxylate, Sodium Trideceth Sulfate, Sodium Tridecylbenzenesulfonate, Sodium Tridecyl Sulfate, Sodium Trimethylolpropane Hydroxypropyl Iminodiacetate Dendrimer, Sodium Undeceth-5 Carboxylate, Sodium Undecylenate, Sodium Undecylenoamphoacetate, Sodium Undecylenoamphopropionate, Sodium Undecylenoyl Glutamate, Sodium Wheat Germamphoacetate, Sorbeth-160 Tristearate, Soy Acid, Soyamidopropylamine Oxide, Soyamidopropyl Betaine, Soybean Oil Glycereth-8 Esters, Stearamidopropylamine Oxide, Stearamidopropyl Betaine, Stearamine Oxide, Steareth-15, Steareth-16, Steareth-20, Steareth-21, Steareth-25, Steareth-27, Steareth-30, Steareth- 40, Steareth-50, Steareth-80, Steareth-100, Steareth-2 Phosphate, Steareth-3 Phosphate, Stearic Acid, Stearoxypropyltrimonium Chloride, Stearoyl Glutamic Acid, Stearoyl Sarcosine, Stearyl Betaine, Stearyldimoniumhydroxypropyl Butylglucosides Chloride, Stearyldimoniumhydroxypropyl Decylglucosides Chloride, Stearyldimoniumhydroxypropyl Laurylglucosides Chloride, Sulfated Castor Oil, Sulfated Coconut Oil, Sulfated Glyceryl Oleate, Sulfated Olive Oil, Sulfated Peanut Oil, Sunfloweramide MEA, Sunflower Seed Acid, Sunflowerseedamidopropyl Hydroxyethyldimonium Chloride, Sunflower Seed Oil Glycereth-8 Esters, Tall Oil Acid, Tallow Acid, Tallowamidopropylamine Oxide, Tallowamidopropyl Betaine, Tallowamidopropyl Hydroxysultaine, Tallowamine Oxide, Tallow Betaine, Tallow Dihydroxyethyl Betaine, Tallowoyl Ethyl Glucoside, TEA-Abietoyl Hydrolyzed Collagen, TEA-C12-14 Alkyl Phosphate, TEA-C10-15 Alkyl Sulfate, TEA-C11-15 Alkyl Sulfate, TEA- C12-13 Alkyl Sulfate, TEA-C12-14 Alkyl Sulfate, TEA-C12-15 Alkyl Sulfate, TEA C14-17 Alkyl Sec Sulfonate, TEA-Canolate, TEA-Cocamide Diacetate, TEA-Cocoate, TEA-Coco-Sulfate, TEA-Cocoyl Alaninate, TEA-Cocoyl Glutamate, TEA-Cocoyl Glutaminate, TEA-Cocoyl Glycinate, TEA-Cocoyl Hydrolyzed Collagen, TEA-Cocoyl Hydrolyzed Soy Protein, TEA-Cocoyl Sarcosinate, TEA- Dimethicone PEG-7 Phosphate, TEA-Dodecylbenzenesulfonate, TEA-Hydrogenated Cocoate, TEA- Hydrogenated Tallowoyl Glutamate, TEA-Isostearate, TEA-Isostearoyl Hydrolyzed Collagen, TEA- Lauraminopropionate, TEA-Laurate, TEA-Laurate/Myristate, TEA-Laureth Sulfate, TEA-Lauroyl Collagen Amino Acids, TEA-Lauroyl Glutamate, TEA-Lauroyl Hydrolyzed Collagen, TEA-Lauroyl Keratin Amino Acids, TEA-Lauroyl Methylaminopropionate, TEA-Lauroyl/Myristoyl Aspartate, TEA- Lauroyl Sarcosinate, TEA-Lauryl Phosphate, TEA-Lauryl Sulfate, TEA-Myristaminopropionate, TEA- Myristate, TEA-Myristoyl Hydrolyzed Collagen, TEA-Oleate, TEA-Oleoyl Hydrolyzed Collagen, TEA- Oleoyl Sarcosinate, TEA-Oleyl Sulfate, TEA-Palmitate, TEA-Palm Kernel Sarcosinate, TEA-PEG-3 Cocamide Sulfate, TEA-Rosinate, TEA-Stearate, TEA-Tallate, TEA-T ridecylbenzenesulfonate, TEA- Undecylenate, TEA-Undecylenoyl Hydrolyzed Collagen, Tetramethyl Decynediol, Tetrasodium Dicarboxyethyl Stearyl Sulfosuccinamate, TIPA-Laureth Sulfate, TIPA-Lauryl Sulfate, TIPA-Myristate, TIPA-Stearate, Tocopheryl Phosphate, Trehalose Undecylenoate, TM-C12-15 Pareth-2 Phosphate, TM-C12-15 Pareth-6 Phosphate, TM-C12-15 Pareth-8 Phosphate, TM-C12-15 Pareth-10 Phosphate, Trideceth-20, Trideceth-50, Trideceth-3 Carboxylic Acid, Trideceth-4 Carboxylic Acid, Trideceth-7 Carboxylic Acid, Trideceth-8 Carboxylic Acid, Trideceth-15 Carboxylic Acid, Trideceth-19 Carboxylic Acid, Trideceth-10 Phosphate, Tridecylbenzenesulfonic Acid, Trilaureth-9 Citrate, Trimethylolpropane Hydroxypropyl Bis-Hydroxyethylamine Dendrimer, Trisodium Lauroampho PG-Acetate Chloride Phosphate, Undecanoic Acid, Undeceth-5 Carboxylic Acid, Undecylenamidopropylamine Oxide, Undecylenamidopropyl Betaine, Undecylenic Acid, Undecylenoyl Collagen Amino Acids, Undecylenoyl Glycine, Undecylenoyl Hydrolyzed Collagen, Undecylenoyl Wheat Amino Acids, Undecyl Glucoside, Wheat Germ Acid, Wheat Germamidopropylamine Oxide, Wheat Germamidopropyl Betaine, Yucca Schidigera Leaf/Root/Stem Extract, Yucca Schidigera Stem Extract, Zinc Coceth Sulfatea and Zinc Coco-Sulfate.

Green and synthetic polymers: The composition as defined herein, is advantageously combined with at least one green or synthetic polymer. Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®}550, Chemviron), polyaminopolyamides and crosslinked watersoluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300. Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones. In a preferred variant, the composition according to the present invention preferably includes an un-crosslinked or polyol-crosslinked polyacrylic acid as additionally polymer component.

Perfume oils and/or fragrances: The composition according to the present invention preferably includes one or more perfume oils and/or fragrances. Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, beta-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

**Anti-cellulite agent:** The composition according to the present invention preferably includes one or more anti-cellulite agents. Anti-cellulite agents and lipolytic agents are preferably selected from the group consisting of beta-adrenergic receptor agonists such as synephrine and its derivatives, and cyclohexyl carbamates. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

**Fat enhancing agents:** The composition according to the present invention preferably includes one or more fat enhancing agents and/or adipogenic agents as well as agents enhancing or boosting the activity of fat enhancing agents. A fat enhancing agent is for example hydroxymethoxyphenyl propylmethylmethoxybenzofuran (trade name: Sym3D^{®}).

**Superfatting agents and/or consistency factors:** The composition according to the present invention preferably includes one or more superfatting agents and/or consistency factors. Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers. The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

**Pearlizing** waxes: The composition according to the present invention preferably includes one or more pearlizing waxes. Suitable pearlizing waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups, and mixtures thereof.

**Silicones:** In order to impart a silky, spreadable, and luxurious texture and to make skin look and feel smoother, and additionally to improve processability (antifoaming) the composition according to the present invention preferably includes one or more silicones or silicone deratives. Suitable silicones can be chosen from the group consisting of Acefylline Methylsilanol Mannuronate, Acetylmethionyl Methylsilanol Elastinate Acrylates/Behenyl, Acrylate/Dimethicone Methacrylate Copolymer, Acrylates/Behenyl Methacrylate/Dimethicone Methacrylate Copolymer, Acrylates/Bis-Hydroxypropyl Dimethicone Crosspolymer, Acrylates/Dimethicone Copolymer, Acrylates/Dimethicone Methacrylate/Ethylhexyl Acrylate Copolymer, Acrylates/Dimethiconol Acrylate Copolymer, Acrylates/Ethylhexyl Acrylate/Dimethicone Methacrylate Copolymer, Acrylates/Octylacrylamide/Diphenyl Amodimethicone Copolymer, Acrylates/Polytrimethylsiloxymethacrylate Copolymer, Acrylates/Propyl Trimethicone Methacrylate Copolymer, Acrylates/Stearyl Acrylate/Dimethicone Methacrylate Copolymer, Acrylates/Tridecyl Acrylate/Triethoxysilylpropyl Methacrylate/Dimethicone Methacrylate Copolymer, Acrylates/Trifluoropropylmethacrylate/Polytrimethyl Siloxymethacrylate Copolymer, Amino Bispropyl Dimethicone, Aminoethylaminopropyl Dimethicone, Aminopropyl Dimethicone, Aminopropyl Phenyl Trimethicone, Aminopropyl Triethoxysilane, Ammonium Dimethicone PEG-7 Sulfate, Amodimethicone, Amodimethicone Hydroxystearate, Amodimethicone/Silsesquioxane Copolymer, Ascorbyl Carboxydecyl Trisiloxane, Ascorbyl Methylsilanol Pectinate, Behenoxy Dimethicone, Behentrimonium Dimethicone PEG-8 Phthalate, Behenyl Dimethicone, Bisamino PEG/PPG-41/3 Aminoethyl PG-Propyl Dimethicone, Bis-Aminopropyl/Ethoxy Aminopropyl Dimethicone, Bis(Butylbenzoate) Diaminotriazine Aminopropyltrisiloxane, Bis-Butyldimethicone Polyglyceryl-3, Bis-Butyloxyamodimethicone/PEG-60 Copolymer, Bis(C13-15 Alkoxy) Hydroxybutamidoamodimethicone, Bis(C13-15 Alkoxy) PG- Amodimethicone, Bis-(C1-8 Alkyl Lauroyl Lysine Decylcarboxamide) Dimethicone, Bis-Cetyl Cetyl Dimethicone, Bis-Cetyl/PEG-8 Cetyl PEG-8 Dimethicone, Bis-Diphenylethyl Disiloxane, Bis-Ethyl Ethyl Methicone, Bis-Gluconamidoethylaminopropyl Dimethicone, Bis-Hydrogen Dimethicone, Bis-Hydroxyethoxypropyl Dimethicone Bis-Hydroxylauryl, Dimethicone/IPDI Copolymer, Bis-Hydroxy/Methoxy Amodimethicone, Bis-Hydroxypropyl Dimethicone Behenate, Bis-Hydroxypropyl Dimethicone/SMDI Copolymer, Bis-Isobutyl PEG-14/Amodimethicone Copolymer, Bis-Isobutyl PEG- 15/Amodimethicone Copolymer, Bis-Isobutyl PEG/PPG-20/35/Amodimethicone Copolymer, Bis- Isobutyl PEG/PPG-10/7/Dimethicone Copolymer, Bis-Isobutyl PEG-24/PPG-7/Dimethicone Copolymer, Bis-PEG-1 Dimethicone, Bis-PEG-4 Dimethicone, Bis-PEG-8 Dimethicone, Bis-PEG-12 Dimethicone,Bis-PEG-20 Dimethicone, Bis-PEG-12 Dimethicone Beeswax, Bis-PEG-12 Dimethicone Candelillate, Bis-PEG-15 Dimethicone/IPDI Copolymer, Bis-PEG-15 Methyl Ether Dimethicone, Bis- PEG-18 Methyl Ether Dimethyl Silane, Bis-PEG/PPG-14/14 Dimethicone, Bis-PEG/PPG-15/5 Dimethicone, Bis-PEG/PPG-18/6 Dimethicone, Bis-PEG/PPG-20/20 Dimethicone, Bis-PEG/PPG- 16/16 PEG/PPG-16/16 Dimethicone, Bis-PEG/PPG-20/5 PEG/PPG-20/5 Dimethicone, Bisphenylhexamethicone, Bis-Phenylpropyl Dimethicone, Bispolyethylene Dimethicone, Bis-(Polyglyceryl-3 Oxyphenylpropyl) Dimethicone, Bis-(Polyglyceryl-7 Oxyphenylpropyl) Dimethicone, Bis-PPG-15 Dimethicone/IPDI Copolymer, Bis(PPG-7 Undeceneth-21) Dimethicone, Bis-Stearyl Dimethicone, Bis-Trimethoxysilylethyl Tetramethyldisiloxyethyl Dimethicone, Bis-Vinyldimethicone, Bis-Vinyl Dimethicone/Dimethicone Copolymer, Borage Seed Oil PEG-7 Dimethicone Esters, Butyl Acrylate/C6-14 Perfluoroalkylethyl Acrylate/Mercaptopropyl Dimethicone Copolymer, Butyl Acrylate/Hydroxypropyl Dimethicone Acrylate Copolymer, Butyl Dimethicone Acrylate/Cyclohexylmethacrylate/Ethylhexyl Acrylate Copolymer, Butyldimethicone Methacrylate/Methyl Methacrylate Crosspolymer, t-Butyl Dimethyl Silyl Grape Seed Extract, Butyl Polydimethylsiloxyl Ethylene/Propylene/Vinylnorbornene Copolymer, C6-8 Alkyl C3-6 Alkyl Glucoside Dimethicone, C20-24 Alkyl Dimethicone,C24-28 Alkyl Dimethicone, C26-28 Alkyl Dimethicone, C30-45 Alkyl Dimethicone, C30-60 Alkyl Dimethicone, C32 Alkyl Dimethicone, C30-45 Alkyl Dimethicone/Polycyclohexene Oxide Crosspolymer, C26-28 Alkyldimethylsilyl Polypropylsilsesquioxane, C30-45 Alkyldimethylsilyl Polypropylsilsesquioxane, C20-24 Alkyl Methicone, C24-28 Alkyl Methicone, C26-28 Alkyl Methicone, C30-45 Alkyl Methicone, C20-28 Alkyl Perfluorodecylethoxy Dimethicone, C26-54 Alkyl Tetradecyl Dimethicone, Capryl Dimethicone, Caprylyl Dimethicone Ethoxy Glucoside, Caprylyl Methicone, Caprylyl Trimethicone, Carboxydecyl Trisiloxane, Castor Oil Bis-Hydroxypropyl Dimethicone Esters Cerotyl Dimethicone, Cetearyl Dimethicone Crosspolymer, Cetearyl Dimethicone/Vinyl Dimethicone Crosspolymer, Cetearyl Methicone, Cetrimonium Carboxydecyl PEG-8 Dimethicone, Cetrimonium Dimethicone PEG-7 Phthalate, Cetyl Behenyl Dimethicone, Cetyl Dimethicone, Cetyl Dimethicone/Bis-Vinyldimethicone Crosspolymer, Cetyl Hexacosyl Dimethicone, Cetyloxy Dimethicone, Cetyl PEG-8 Dimethicone, Cetyl PEG/PPG-15/15 Butyl Ether Dimethicone, Cetyl PEG/PPG-7/3 Dimethicone, Cetyl PEG/PPG-10/1 Dimethicone, Cetyl Triethylmonium Dimethicone PEG-8 Phthalate, Cetyl Triethylmonium Dimethicone PEG-8 Succinate, Copper Acetyl Tyrosinate Methylsilanol, Copper PCA Methylsilanol, C4-14 Perfluoroalkylethoxy Dimethicone, Cycloethoxymethicone, Cycloheptasiloxane, Cyclohexasiloxane, Cyclomethicone, Cyclopentasiloxane, Cyclophenylmethicone, Cyclotetrasiloxane,mCyclovinylmethicone, Cystine Bis-PG-Propyl Silanetriol, DEA PG-Propyl PEG/PPG-18/21 Dimethicone, Diisostearoyl Trimethylolpropane Siloxy Silicate, Dilauroyl Trimethylolpropane Siloxy Silicate, Dilinoleamidopropyl Dimethylamine Dimethicone PEG-7 Phosphate, Dimethicone, Dimethicone Crosspolymer, Dimethicone Crosspolymer-3, Dimethicone/Divinyldimethicone/Silsesquioxane Crosspolymer, Dimethicone Ethoxy Glucoside, Dimethicone Hydroxypropyl Trimonium Chloride, Dimethicone/Mercaptopropyl Methicone Copolymer, Dimethicone PEG-15 Acetate Dimethicone PEG-8 Adipate, Dimethicone PEG-7 Avocadoate, Dimethicone PEG-8 Avocadoate, Dimethicone PEG-8 Beeswax, Dimethicone PEG-8 Benzoate, Dimethicone PEG-8 Borageate, Dimethicone PEG-7 Cocoate, Dimethicone/PEG-10 Crosspolymer, Dimethicone/PEG-10/15 Crosspolymer, Dimethicone/PEG-15 Crosspolymer, Dimethicone PEG-7 Isostearate, Dimethicone PEG-8 Isostearate, Dimethicone PEG-7 Lactate, Dimethicone PEG-8 Lanolate, Dimethicone PEG-8 Laurate, Dimethicone PEG-8 Meadowfoamate, Dimethicone PEG-7 Octyldodecyl Citrate, Dimethicone PEG-7 Olivate, Dimethicone PEG-8 Olivate, Dimethicone PEG-7 Phosphate, Dimethicone PEG-8 Phosphate, Dimethicone PEG-10 Phosphate, Dimethicone PEG-7 Phthalate, Dimethicone PEG-8 Phthalate, Dimethicone PEG-8 Polyacrylate, Dimethicone PEG/PPG- 20/23 Benzoate, Dimethicone PEG/PPG-7/4 Phosphate, Dimethicone PEG/PPG-12/4 Phosphate, Dimethicone PEG-7 Succinate, Dimethicone PEG-8 Succinate, Dimethicone PEG-7 Sulfate, Dimethicone PEG-7 Undecylenate, Dimethicone PG-Diethylmonium Chloride, Dimethicone/Phenyl Vinyl Dimethicone Crosspolymer, Dimethicone/Polyglycerin-3 Crosspolymer, Dimethicone/PPG-20 Crosspolymer, Dimethicone Propylethylenediamine Behenate, Dimethicone Propyl PG-Betaine, Dimethicone/Silsesquioxane Copolymer, Dimethicone Silylate, Dimethicone Dimethicone Crosspolymer, Dimethicone/Vinyltrimethylsiloxysilicate Crosspolymer, Dimethiconol, Dimethiconol Arginine, Dimethiconol Beeswax, Dimethiconol Behenate, Dimethiconol Borageate, Dimethiconol Candelillate, Dimethiconol Carnaubate, Dimethiconol Cysteine, Dimethiconol Dhupa Butterate, Dimethiconol Fluoroalcohol Dilinoleic Acid, Dimethiconol Hydroxystearate, Dimethiconol Illipe Butterate, Dimethiconol/IPDI Copolymer, Dimethiconol Isostearate, Dimethiconol Kokum Butterate, Dimethiconol Lactate, Dimethiconol Meadowfoamate, Dimethiconol Methionine, Dimethiconol/Methylsilanol/Silicate Crosspolymer, Dimethiconol Mohwa Butterate, Dimethiconol Panthenol, Dimethiconol Sal Butterate, Dimethiconol/Silica Crosspolymer, Dimethiconol/Silsesquioxane Copolymer, Dimethiconol Stearate, Dimethiconol/Stearyl, Methicone/Phenyl Trimethicone Copolymer, Dimethoxysilyl Ethylenediaminopropyl Dimethicone, Dimethylaminopropylamido PCA Dimethicone, Dimethyl Oxobenzo Dioxasilane, Dimethylsilanol Hyaluronate, Dioleyl Tocopheryl Methylsilanol, Diphenyl Amodimethicone, Diphenyl Dimethicone, Diphenyl Dimethicone Crosspolymer Diphenyl Dimethicone?/inyl Diphenyl Dimethicone/Silsesquioxane Crosspolymer, Diphenylethyl Benzyloxy Dilsiloxane, Diphenylisopropyl Dimethicone, Diphenylsiloxy Phenyl/Propyl Trimethicone, Diphenylsiloxy Phenyl Trimethicone Disiloxane, Disodium Amodimethicone Disuccinamide, Disodium PEG-12 Dimethicone Sulfosuccinate, Disodium PEG-8 Lauryl Dimethicone Sulfosuccinate, Divinyldimethicone/Dimethicone Copolymer, Divinyldimethicone/Dimethicone Crosspolymer, Drometrizole Trisiloxane, Ethylhexyl Acrylate/VP/Dimethicone Methacrylate Copolymer, Ethyl Methicone, Ethyl Trisiloxane, Fluoro C2-8 Alkyldimethicone, Gluconamidopropyl Aminopropyl Dimethicone, 4-(2-Beta-Glucopyranosiloxy) Propoxy-2-Hydroxybenzophenone, Glyceryl Undecyl Dimethicone, Glycidoxy Dimethicone, Hexadecyl Methicone, Hexyl Dimethicone, Hexyl Methicone, Hexyltrimethoxysilane, Hydrogen Dimethicone, Hydrogen Dimethicone/Octyl Silsesquioxane Copolymer, Hydrolyzed Collagen PG-Propyl Dimethiconol, Hydrolyzed Collagen PG-Propyl Methylsilanediol, Hydrolyzed Collagen PG-Propyl Silanetriol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Silk PG-Propyl Methylsilanediol Crosspolymer, Hydrolyzed Soy Protein/Dimethicone PEG-7 Acetate, Hydrolyzed Soy Protein PG-Propyl Methylsilanediol, Hydrolyzed Vegetable Protein PG-Propyl Silanetriol, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein PG-Propyl Methylsilanediol, Hydrolyzed Wheat Protein PG-Propyl Silanetriol, Hydroxyethyl Acetomonium PG-Dimethicone, Hydroxypropyldimethicone, Hydroxypropyl Dimethicone Behenate, Hydroxypropyl Dimethicone Isostearate, Hydroxypropyl Dimethicone Stearate, Isobutylmethacrylate/Bis-Hydroxypropyl Dimethicone Acrylate Copolymer, Isobutylmethacrylate/Trifluoroethylmethacrylate/Bis-Hydroxypropyl Dimethicone Acrylate Copolymer, Isopentyl Trimethoxycinnamate Trisiloxane, Isopolyglyceryl-3 Dimethicone, Isopolyglyceryl-3 Dimethiconol, Isopropyl Titanium Triisostearate/Triethoxysilylethyl, Polydimethylsiloxyethyl Dimethicone Crosspolymer, Isostearyl Carboxydecyl PEG-8 Dimethicone, Lactoyl Methylsilanol Elastinate, Lauryl Dimethicone, Lauryl Dimethicone PEG-15 Crosspolymer, Lauryl Dimethicone PEG- 10 Phosphate, Lauryl Dimethicone/Polyglycerin-3 Crosspolymer, Lauryl Methicone, Lauryl PEG-8 Dimethicone, Lauryl PEG-10 Methyl Ether Dimethicone, Lauryl PEG-9 Polydimethylsiloxyethyl Dimethicone, Lauryl PEG/PPG-18/18 Methicone, Lauryl Phenylisopropyl Methicone, Lauryl Phenylpropyl Methicone, Lauryl Polydimethylsiloxyethyl Dimethicone/Bis-Vinyldimethicone Crosspolymer, Lauryl Polyglyceryl-3 Polydimethylsiloxyethyl Dimethicone, Lauryl Trimethicone, Linoleamidopropyl PG-Dimonium Chloride Phosphate Dimethicone, Methacryloyl Propyltrimethoxysilane, Methicone, Methoxy Amodimethicone/Silsesquioxane Copolymer, Methoxycinnamidopropyl Polysilsesquioxane, Methoxycinnamoylpropyl Silsesquioxane Silicate, Methoxy PEG-13 Ethyl Polysilsesquioxane, Methoxy PEG/PPG-7/3 Aminopropyl Dimethicone, Methoxy PEG/PPG-25/4 Dimethicone, Methoxy PEG-10 Propyltrimethoxysilane, Methyleugenyl PEG- 8 Dimethicone, Methylpolysiloxane Emulsion, Methylsilanol Acetylmethionate, Methylsilanol Acetyltyrosine, Methylsilanol Ascorbate, Methylsilanol Carboxymethyl Theophylline, Methylsilanol Carboxymethyl Theophylline Alginate, Methylsilanol Elastinate, Methylsilanol Glycyrrhizinate, Methylsilanol Hydroxyproline, Methylsilanol Hydroxyproline Aspartate, Methylsilanol Mannuronate, Methylsilanol PCA, Methylsilanol PEG-7 Glyceryl Cocoate, Methylsilanol/Silicate Crosspolymer, Methylsilanol Spirulinate, Methylsilanol Tri-PEG-8 Glyceryl Cocoate, Methyl Trimethicone, Methyltrimethoxysilane, Myristylamidopropyl Dimethylamine Dimethicone PEG-7 Phosphate, Myristyl Methicone, Myristyl Trisiloxane, Nylon-611/Dimethicone Copolymer, PCA Dimethicone, PEG-7 Amodimethicone, PEG-8 Amodimethicone, PEG-8 Cetyl Dimethicone, PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone, PEG-17 Dimethicone, PEG-10 Dimethicone Crosspolymer, PEG-12 Dimethicone Crosspolymer, PEG-8 Dimethicone Dimer Dilinoleate, PEG-8 Dimethicone/Dimer Dilinoleic Acid Copolymer, PEG-10 Dimethicone/Vinyl Dimethicone Crosspolymer, PEG-8 Distearmonium Chloride PG-Dimethicone, PEG-10/Lauryl Dimethicone Crosspolymer, PEG- 15/Lauryl Dimethicone Crosspolymer, PEG-15/Lauryl Polydimethylsiloxyethyl Dimethicone Crosspolymer, PEG-8 Methicone, PEG-6 Methicone Acetate, PEG-6 Methyl Ether Dimethicone, PEG- 7 Methyl Ether Dimethicone, PEG-8 Methyl Ether Dimethicone, PEG-9 Methyl Ether Dimethicone, PEG-10 Methyl Ether Dimethicone, PEG-11 Methyl Ether Dimethicone, PEG-32 Methyl Ether Dimethicone, PEG-8 Methyl Ether Triethoxysilane, PEG-10 Nonafluorohexyl Dimethicone Copolymer, PEG-4 PEG-12 Dimethicone, PEG-8 PG-Coco-Glucoside Dimethicone, PEG-9 Polydimethylsiloxyethyl Dimethicone, PEG/PPG-20/22 Butyl Ether Dimethicone, PEG/PPG-22/22 Butyl Ether Dimethicone, PEG/PPG-23/23 Butyl Ether Dimethicone, PEG/PPG-24/18 Butyl Ether Dimethicone, PEG/PPG-27/9 Butyl Ether Dimethicone, PEG/PPG-3/10 Dimethicone, PEG/PPG-4/12 Dimethicone, PEG/PPG-6/4 Dimethicone, PEG/PPG-6/11 Dimethicone, PEG/PPG-8/14 Dimethicone, PEG/PPG-8/26 Dimethicone, PEG/PPG-10/2 Dimethicone, PEG/PPG-12/16 Dimethicone, PEG/PPG- 12/18 Dimethicone, PEG/PPG-14/4 Dimethicone, PEG/PPG-15/5 Dimethicone, PEG/PPG-15/15 Dimethicone, PEG/PPG-16/2 Dimethicone, PEG/PPG-16/8 Dimethicone, PEG/PPG-17/18 Dimethicone, PEG/PPG-18/6 Dimethicone, PEG/PPG-18/12 Dimethicone, PEG/PPG-18/18 Dimethicone, PEG/PPG-19/19 Dimethicone, PEG/PPG-20/6 Dimethicone, PEG/PPG-20/15 Dimethicone, PEG/PPG-20/20 Dimethicone, PEG/PPG-20/23 Dimethicone, PEG/PPG-20/29 Dimethicone, PEG/PPG-22/23 Dimethicone, PEG/PPG-22/24 Dimethicone, PEG/PPG-23/6 Dimethicone, PEG/PPG-25/25 Dimethicone, PEG/PPG-27/27 Dimethicone, PEG/PPG-30/10 Dimethicone, PEG/PPG-25/25 Dimethicone/Acrylates Copolymer, PEG/PPG-20/22 Methyl Ether Dimethicone, PEG/PPG-24/24 Methyl Ether Glycidoxy Dimethicone, PEG/PPG-10/3 Oleyl Ether Dimethicone, PEG/PPG-5/3 Trisiloxane, PEG-4 Trifluoropropyl Dimethicone Copolymer, PEG-8 Trifluoropropyl Dimethicone Copolymer, PEG-10 Trifluoropropyl Dimethicone Copolymer, PEG-8 Trisiloxane, Perfluorocaprylyl riethoxysilylethyl Methicone, Perfluorononyl Dimethicone, Perfluorononyl Dimethicone/Methicone/Amodimethicone Crosspolymer, Perfluorononylethyl Carboxydecyl Behenyl Dimethicone, Perfluorononylethyl Carboxydecyl Hexacosyl Dimethicone, Perfluorononylethyl Carboxydecyl Lauryl/Behenyl Dimethicone, Perfluorononylethyl Carboxydecyl Lauryl Dimethicone, Perfluorononylethyl Carboxydecyl PEG-8 Dimethicone, Perfluorononylethyl Carboxydecyl PEG-10 Dimethicone, Perfluorononylethyl Dimethicone/Methicone Copolymer, Perfluorononylethyl PEG-8 Dimethicone, Perfluorononylethyl Stearyl Dimethicone, Perfluorooctylethyl/Diphenyl Dimethicone Copolymer, Perfluorooctylethyl Triethoxysilane, Perfluorooctylethyl Trimethoxysilane, Perfluorooctylethyl Trisiloxane, Perfluorooctyl Triethoxysilane, PG-Amodimethicone, Phenethyl Dimethicone, Phenethyl Disiloxane, Phenyl Dimethicone, Phenylisopropyl Dimethicone, Phenyl Methicone, Phenyl Methiconol, Phenylpropyldimethylsiloxysilicate, Phenylpropyl Ethyl Methicone, Phenyl Propyl Trimethicone, Phenyl Propyl Trimethicone/Diphenylmethicone, Phenyl Trimethicone, Platinum Divinyldisiloxane, Polyacrylate-6, Polydiethylsiloxane, Polydimethylsiloxyethyl Dimethicone/Bis-Vinyldimethicone Crosspolymer, Polydimethylsiloxyethyl Dimethicone/Methicone Copolymer, Polydimethylsiloxy PEG/PPG-24/19 Butyl Ether Silsesquioxane, Polydimethylsiloxy PPG- 13 Butyl Ether Silsesquioxane, Polyglyceryl-3 Disiloxane Dimethicone, Polyglyceryl-3/Lauryl Polydimethylsiloxyethyl Dimethicone Crosspolymer, Polyglyceryl-3 Polydimethylsiloxyethyl Dimethicone, Poly(Glycol Adipate)/Bis-Hydroxyethoxypropyl Dimethicone Copolymer, Polymethylsilsesquioxane, Polymethylsilsesquioxane/Trimethylsiloxysilicate, Polyphenylsilsesquioxane, Polypropylsilsesquioxane, Polysilicone-1, Polysilicone-2, Polysilicone-3, Polysilicone-4, Polysilicone-5, Polysilicone-6, Polysilicone-7, Polysilicone-8, Polysilicone-9, Polysilicone-10, Polysilicone-11, Polysilicone-12, Polysilicone-13, Polysilicone-14, Polysilicone-15, Polysilicone-16, Polysilicone-17, Polysilicone-18, Polysilicone-19, Polysilicone-20, Polysilicone-21, Polysilicone-18 Cetyl Phosphate, Polysilicone-1 Crosspolymer, Polysilicone-18 Stearate, Polyurethane-10, Potassium Dimethicone PEG-7 Panthenyl Phosphate, Potassium Dimethicone PEG- 7 Phosphate, PPG-12 Butyl Ether Dimethicone, PPG-2 Dimethicone, PPG-12 Dimethicone, PPG-27 Dimethicone, PPG-4 Oleth-10 Dimethicone, Propoxytetramethyl Piperidinyl Dimethicone, Propyl Trimethicone, Quaternium-80, Retinoxytrimethylsilane, Silanediol Salicylate, Silanetriol, Silanetriol Arginate, Silanetriol Glutamate, Silanetriol Lysinate, Silanetriol Melaninate, Silanetriol Trehalose Ether, Silica, Silica Dimethicone Silylate, Silica Dimethyl Silylate, Silica Silylate, Silicon Carbide, Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, SiliconeQuaternium-16, Silicone Quaternium-16/Glycidoxy Dimethicone Crosspolymer, Silicone Quaternium-17, Silicone Quaternium- 18, Silicone Quaternium-19, Silicone Quaternium-20, Silicone Quaternium-21, Silicone Quaternium- 22, Silicone Quaternium-24, Silicone Quaternium-25, Siloxanetriol Alginate, Siloxanetriol Phytate, Simethicone, Sodium Carboxydecyl PEG-8 Dimethicone, Sodium Dimethicone PEG-7 Acetyl Methyltaurate, Sodium Hyaluronate Dimethylsilanol, Sodium Lactate Methylsilanol, Sodium Mannuronate Methylsilanol, Sodium PCA Methylsilanol, Sodium PG-Propyldimethicone Thiosulfate Copolymer, Sodium PG-Propyl Thiosulfate Dimethicone, Sodium Propoxyhydroxypropyl Thiosulfate Silica, Sorbityl Silanediol, Soy Triethoxysilylpropyldimonium Chloride, Stearalkonium Dimethicone PEG-8 Phthalate, Stearamidopropyl Dimethicone, Steardimonium Hydroxypropyl Panthenyl PEG-7 Dimethicone Phosphate Chloride, Steardimonium Hydroxypropyl PEG-7 Dimethicone Phosphate Chloride, Stearoxy Dimethicone, Stearoxymethicone/Dimethicone Copolymer, Stearoxytrimethylsilane, Stearyl Aminopropyl Methicone, Stearyl Dimethicone, Stearyl/Lauryl Methacrylate Crosspolymer, Stearyl Methicone, Stearyl Triethoxysilanek, Stearyl Trimethicone, Styrene/Acrylates/Dimethicone Acrylate Crosspolymer, Styrene/Acrylates/Dimethicone Copolymer, TEA-Dimethicone PEG-7 Phosphate, Tetrabutoxypropyl Trisiloxane, Tetramethyl Hexaphenyl Tetrasiloxane, Tetramethyl Tetraphenyl Trisiloxane, Tocopheryloxypropyl Trisiloxane, Trideceth-9 PG-Amodimethicone, Triethoxycaprylylsilane, Triethoxysilylethyl Dimethicone/Methicone Copolymer, Triethoxysilylethyl Polydimethylsiloxyethyl Dimethicone, Triethoxysilylethyl Polydimethylsiloxyethyl Hexyl Dimethicone, Triethoxysilylpropylcarbamoyl Ethoxypropyl Butyl Dimethicone, Trifluoromethyl C1-4 Alkyl Dimethicone, Trifluoropropyl Cyclopentasiloxane, Trifluoropropyl Cyclotetrasiloxane, Trifluoropropyl Dimethicone, Trifluoropropyl Dimethicone/PEG-10 Crosspolymer, Trifluoropropyl Dimethicone/Trifluoropropyl Divinyldimethicone Crosspolymer, Trifluoropropyl Dimethicone/Vinyl Trifluoropropyl, Dimethicone/Silsesquioxane Crosspolymer, Trifluoropropyl Dimethiconol, Trifluoropropyldimethyl/trimethylsiloxysilicate, Trifluoropropyl Methicone, Trimethoxycaprylylsilane, Trimethoxysilyl Dimethicone, Trimethyl Pentaphenyl Trisiloxane, Trimethylsiloxyamodimethicone, Trimethylsiloxyphenyl Dimethicone, Trimethylsiloxysilicate, Trimethylsiloxysilicate/Dimethicone Crosspolymer, Trimethylsiloxysilicate/Dimethiconol Crosspolymer, Trimethylsiloxysilylcarbamoyl Pullulan, Trimethylsilyl Hydrolyzed Conchiolin Protein PG-Propyl Methylsilanediol Crosspolymer, Trimethylsilyl Hydrolyzed Silk PG-Propyl Methylsilanediol Crosspolymer, Trimethylsilyl Hydrolyzed Wheat Protein PG-Propyl Methylsilanediol Crosspolymer, Trimethylsilyl Pullulan, Trimethylsilyl Trimethylsiloxy Glycolate, Trimethylsilyl Trimethylsiloxy Lactate, Trimethylsilyl Trimethylsiloxy Salicylate, Triphenyl Trimethicone, Trisiloxane, Tris-Tributoxysiloxymethylsilane, Undecylcrylene Dimethicone, Vinyl Dimethicone, Vinyl Dimethicone/Lauryl Dimethicone Crosspolymer, Vinyl Dimethicone/Methicone Silsesquioxane Crosspolymer, Vinyldimethyl/Trimethylsiloxysilicate Stearyl Dimethicone Crosspolymer, VP/Dimethiconylacrylate/Polycarbamyl/Polyglycol Ester, Zinc Carboxydecyl Trisiloxane, Zinc Dimethicone PEG-8 Succinate, and mixtures thereof.

More preferably the silicones to be contained in the composition according to the invention are Dimethicone, Cyclomethicone, Cyclopentasiloxane, Cyclotetrasiloxane, Phenyl Trimethicone, and Cyclohexasiloxane.

Waxes **and/or stabilizers:** Besides natural oils used, one or more waxes may also be present in the composition according to the present invention, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes. Metal salts of fatty acids such as, for example, magnesium, aluminium and/or zinc stearate or ricinoleate may be used as stabilizers.

The composition as defined herein, is advantageously combined with at least one primary sun protection factor and/or with at least one secondary sun protection factor, in order to increase the SPF, i.e. to obtain a high SPF and to cover a broad UVA and UVB range.

An UV filter is a compound or a mixture of compounds that block or absorb ultraviolet (UV) light. Since excessive UV radiation can cause sunburn, photoaging, and skin cancer, care products such as sunscreen usually include a classification for the specific wavelengths they filter. UV classifications include UVA (320 - 400 nm), UVB (290 - 320 nm) and UVC (200 - 280 nm). UV-absorbing compounds are used not only in sunscreen, but also in other personal care products, such as lipstick, shampoo, hair spray, body wash, toilet soap, and insect repellent. Chemical filters protect against UV radiation by absorbing, reflecting, or scattering ist. Reflection and scattering are accomplished by inorganic physical UV filters, such as titanium dioxide (TiO₂) and zinc oxide (ZnO). Absorption, mainly of UVB, is done by organic UV filters, which are known as chemical UV filters.

**Primary sun protection factors:** The composition according to the present invention preferably includes one or more further primary sun protection factors. Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The composition according to the invention advantageously contains at least one UV-A filter and/or at least one UV-B filter and/or a broadband filter and/or at least one inorganic pigment. Compositions according to the invention preferably contain at least one UV-B filter or a broadband filter, more particularly preferably at least one UV-A filter and at least one UV-B filter. Preferred cosmetic compositions, preferably topical compositions according to the present invention comprise one, two, three or more sun protection factors selected from the group consisting of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

In addition, a combination with active ingredients which penetrate into the skin and protect the skin cells from inside against sunlight-induced damage and reduce the level of cutaneous matrix metalloproteases is advantageously. Preferred respective ingredients are so called aryl hydrocarbon receptor antagonists. Preferred is 2-benzylidene-5,6-dimethoxy-3,3-dimethylindan-1-one.

The UV filters cited below which can be used within the context of the present invention are preferred but naturally are not limiting.

In a preferred variant the composition according to the present invention may include a liquid UV filter which is selected from the group consisting of Octocrylene, Ethylhexyl Salicylate, Homosalate, Ethylhexyl Methoxycinnamate, Isoamyl p-Methoxycinnamate, Camphor Benzalkonium Methosulfate, Polyarylamidomethyl Benzylidene Camphor, Isooctyl Methoxycionnamate, Ethylhexyl Triazone, Drometrizole Trisiloxane, Diethylhexyl Butamido Triazone, 3-Benzylidene Camphor, Octyl Salicylate, Ethylhexyl Dimethyl PABA, Bis-Ethylhexyloxyphenol Methoxyphenyl Triazine, Polysilicone-15, Diethylamino Hydroxybenzoyl Hexyl benzoate, N,N,N-Trimethyl-4-(2-oxoborn-3-ylidenemethyl) anilinium methyl sulfate, Benzoic acid-2-hydroxy-3,3,5-trimethylcyclohexyl ester/Homosalate, 2-Cyano-3,3-diphenyl acrylic acid, 2- Ethylhexyl ester/Octocrilene, 2-Ethylhexyl-4-methoxycinnamate/Octinoxate, Ethoxylated Ethyl-4-aminobenzoate, 2-Ethylhexyl salicylate/Octisalate, 2-Ethylhexyl-4-(dimethylamino)benzoate/Padimate O (USAN:BAN), 2,2'-Methylene-bis(6-(2H-benzotriazol-2-yl)-4-(1,1,3,3-tetramethyl-butyl)phenol)/Bisoctrizole, and Dimethico-diethylbenzalmalonate. The aforementioned UV filter(s) can be used either as a single component or in mixture with one or more further different UV filter(s) as further described below.

UV filters which are preferably used in the composition according to the present invention are selected from the group consisting of
- 1-(4-tert-Butylphenyl)-3-(4-methoxyphenyl)-propane-1,3-dione (INCI: Butyl Methoxydibenzoylmethane; Avobenzone),
- Sodium salt of 2,2'-bis(1,4-phenylene)-1H-benzimidazole-4,6-disulfonic acid (INCI: Disodium Phenyl Dibenzimidazole Tetrasulfonate),
- 2-Hydroxy-4-methoxybenzophenone (INCI: Benzophenone-3),
- 2,2'-(6-(4-Methoxyphenyl)-1,3,5-triazine-2,4-diyl)bis(5-((2-ethylhexyl)oxy)phenol) (INCI: Bis-Ethylhexyloxyphenol Methoxyphenyl Triazine; Bemotrizinol),
- Isopentyl-4-methoxycinnamate (INCI: Isoamyl p-Methoxycinnamate; Amiloxate),
- 2-Phenylbenzimidazole-5-sulfonic acid and its potassium, sodium and triethanolamine salts (INCI: Phenylbenzimidazole Sulfonic Acid),
- 3-(4-Methylbenzylidene)-d1 camphor (INCI: 4-Methylbenzylidene Camphor),
- 1,3,5-Triazine-2,4,6-tris[1,1'-biphenyl]-4-yl (INCI: Tris-biphenyl Triazine, incl. Nanomaterial),
- 2-Hydroxy-4-methoxybenzophenone- 5-sulfonic acid and its sodium salt (INCI: Benzophenone-4/Benzophenone-5),
- Zinc Oxide (INCI: Zinc Oxide),
- Benzoic acid, 2-[-4-(diethylamino)-2-hydroxybenzoyl]-hexylester (INCI: Diethylamino Hydroxybenzoyl Hexyl Benzoate),
- 2,4,6-Trianilino-(p-carbo-2'-ethylhexyl-1'-oxy)-1,3,5-triazine (INCI: Ethylhexyl Triazone),
- alpha-(2-Oxoborn-3-ylidene)-toluene-4-sulphonic acid and its salts (INCI: Benzylidene Camphor Sulfonic Acid),
- Polymer of N-{(2 and 4)-[(2-oxoborn-3-ylidene)-methyl]-benzyl}-acrylamide (INCI: Polyacrylamido Methyl Benzylidene Camphor),
- 3,3'-(1,4-Phenylenedimethylene)-bis-(7, 7-dimethyl-2-oxobicyclo-[2.2.1]-hept-1-yl-methanesulfonic acid) and its salts (INCI: Terephthalylidene Dicamphor Sulfonic Acid),
- Ethoxylated Ethyl-4-aminobenzoate (INCI: PEG-25 PABA),
- Benzoic acid, 4,4-((6-((4-(((1,1-dimethylethyl)-amino)-carbonyl)-phenyl)-amino)-1,3,5-triazine-2,4- diyl)-diimino)-bis, bis-(2-ethylhexyl)-ester (INCI: Diethylhexyl Butamido Triazone),
- Phenol-2-(2H-benzotriazol-2-yl)-4- methyl-6-(2-methyl-3-(1,3,3,3-tetramethyl-1-(trimethylsilyl)-oxy)-disiloxanyl)-propyl) (INCI: Drometrizole Trisiloxane),
- 3-Benzylidene camphor (INCI: 3-Benzylidene Camphor),
- Titanium dioxide (INCI: Titanium Dioxide),
and mixtures of two or more of the aforesaid solid UV filters.

UV filters which are also preferably used are selected from the group consisting of:
- p-aminobenzoic acid,
- p-aminobenzoic acid ethyl ester (25 mol) ethoxylated (INCI name: PEG-25 PABA),
- p-dimethylaminobenzoic acid-2-ethylhexyl ester,
- p-aminobenzoic acid ethyl ester (2 mol) N-propoxylated,
- p-aminobenzoic acid glycerol ester,
- salicylic acid homomenthyl ester (homosalates) (Neo Heliopan^{®}HMS),
- salicylic acid-2-ethylhexyl ester (Neo Heliopan^{®}OS),
- triethanolamine salicylate,
- 4-isopropyl benzyl salicylate,
- anthranilic acid menthyl ester (Neo Heliopan^{®}MA),
- diisopropyl cinnamic acid ethyl ester,
- p-methoxycinnamic acid-2-ethylhexyl ester (Neo Heliopan^{®}AV),
- diisopropyl cinnamic acid methyl ester,
- p-methoxycinnamic acid isoamyl ester (Neo Heliopan^{®}E 1000),
- p-methoxycinnamic acid diethanolamine salt,
- p-methoxycinnamic acid isopropyl ester,
- 2-phenylbenzimidazole sulfonic acid and salts (Neo Heliopan^{®}Hydro),
- 3-(4'-trimethylammonium) benzylidene bornan-2-one methyl sulfate,
- beta-imidazole-4(5)-acrylic acid (urocanic acid),
- 3-(4'-sulfo)benzylidene bornan-2-one and salts,
- 3-(4'-methyl benzylidene)-D,L-camphor (Neo Heliopan^{®}MBC),
- 3-benzylidene-D,L-camphor,
- N-[(2 and 4)-[2-(oxoborn-3-ylidene) methyl]benzyl] acrylamide polymer,
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB),
- benzylidene malonate polysiloxane (Parsol^{®}SLX),
- glyceryl ethylhexanoate dimethoxycinnamate,
- dipropylene glycol salicylate,
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150),
and mixutres thereof

In a preferred variant the composition according to the present invention comprises a combination with one or more broadband filters which are selected from the group consisting of:
- 2-ethylhexyl-2-cyano-3,3-diphenyl acrylate,
- ethyl-2-cyano-3,3'-diphenyl acrylate,
- dihydroxy-4-methoxybenzophenone,
- 2,4-dihydroxybenzophenone,
- tetrahydroxybenzophenone,
- 2,2'-dihydroxy-4,4'-dimethoxybenzophenone,
- 2-hydroxy-4-n-octoxybenzophenone,
- 2-hydroxy-4-methoxy-4'-methylbenzophenone,
- sodium hydroxymethoxybenzophenone sulfonate,
- disodium-2,2'-dihydroxy-4,4'-dimethoxy-5,5'-disulfobenzophenone,
- phenol, 2-(2H-benzotriazol-2-yl)-4-methyl-6-(2-methyl-3(1,3,3,3-tetramethyl-1-(trimethylsilyl)oxy)disiloxyanyl) propyl),
- 2,2'-methylene-bis-(6-(2H-benzotriazol-2-yl)-4-1,1 ,3,3-tetramethylbutyl)phenol) (Methylene-Bis-Benzotriazolyl Tetramethylbutylphenol),
- 2,4-bis-[4-(2-ethylhexyloxy)-2-hydroxyphenyl]-1,3,5-triazine,
- 2,4-bis-[{(4-(2-ethylhexyloxy)-2-hydroxy}phenyl]-6-(4-methoxyphenyl)-1,3,5-triazine (INCI: Aniso Triazin),
- 2,4-bis-[{(4-(3-sulfonato)-2-hydroxypropyloxy)-2-hydroxy}phenyl]-6-(4-methoxyphenyl)-1,3,5-triazine sodium salt,
- 2,4-bis-[{(3-(2-propyloxy)-2-hydroxypropyloxy)-2-hydroxy}phenyl]-6-(4-methoxyphenyl)-1,3,5-triazine,
- 2,4-bis-[{4-(2-ethylhexyloxy)-2-hydroxy}phenyl]-6-[4-(2-methoxyethylcarbonyl) phenylamino]-1,3,5-triazine,
- 2,4-bis-[{4-(3-(2-propyloxy)-2-hydroxypropyloxy)-2-hydroxy}phenyl]-6-[4-(2-ethylcarboxyl) phenylamino]-1,3,5-triazine,
- 2,-bis-[{4-(2-ethylhexyloxy)-2-hydroxy}phenyl]-6-(1-methylpyrrol-2-yl)-1,3,5-triazine,
- 2,4-bis-[{4-tris-(trimethylsiloxysilylpropyloxy)-2-hydroxy}phenyl]-6-(4-methoxyphenyl)-1,3,5-triazine,
- 2,4-bis-[{4-(2"-methylpropenyloxy)-2-hydroxy}phenyl]-6-(4-methoxyphenyl)-1,3,5-triazine,
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine,
- dioctylbutylamidotriazone (INCI: Dioctylbutamidotriazone),
- 2,4-bis-[5-1(dimethylpropyl)benzoxazol-2-yl-(4-phenyl)-imino]-6-(2-ethylhexyl)-imino-1,3,5-triazine,
- 4,4',4"-(1,3,5-triazine-2,4,6-triyltriimino)tris-benzoic acid-tris(2-ethyl-hexylester) (also: 2,4,6-tris[anilino(p-carbo-2'-ethyl-1'-hexyloxy)]-1,3,5-triazine (INCI: Octyl Triazone),
- 2,4,6-tribiphenyl-4-yl-1,3,5-triazine,
- 2-Ethylhexyl 4-methoxycinnamate (Ethylhexyl Methoxycinnamate),
- Benzoic acid, 2-hydroxy-, 3,3,5-trimethylcyclohexyl ester (Homosalate),
- 2-Ethylhexyl salicylate (Ethylhexyl Salicylate),
- 2-Ethylhexyl 4-(dimethylamino)benzoate (Ethylhexyl Dimethyl PABA),
- N,N,N-Trimethyl-4-(2-oxoborn-3-ylidenemethyl) anilinium methyl sulfate (Camphor Benzalkonium Methosulfate),
- Dimethicodiethylbenzalmalonate (Polysilicone-15),
and mixtures thereof.

In a preferred variant the composition according to the present invention comprises a combination with one or more UV-A filters which are selected from the group consisting of:
- 4-isopropyl dibenzoyl methane,
- terephthalylidene dibornane sulfonic acid and salts,
- 4-t-butyl-4'-methoxydibenzoyl methane (avobenzone),
- phenylene bis-benzimidazyl tetrasulfonic acid disodium salt,
- 2,2'-(1,4-phenylene)-bis-(1H-benzimidazole-4,6-disulfonic acid), monosodium salt,
- 2-(4-diethylamino-2-hydroxybenzoyl) benzoic acid hexyl ester,
- indanylidene compounds,
and mixtures thereof.

In a more preferred variant, the composition according to the present invention comprises a combination with one or more UV filters which are are selected from the group consisting of:
- p-aminobenzoic acid,
- 3-(4'-trimethylammonium) benzylidene bornan-2-one methyl sulfate.
- salicylic acid homomenthyl ester (Neo Heliopan^{®}HMS),
- 2-phenylbenzimidazole sulfonic acid (Neo Heliopan^{®}Hydro),
- terephthalylidene dibornane sulfonic acid and salts (Mexoryl^{®}SX),
- 4-tert-butyl-4'-methoxydibenzoyl methane (Neo Heliopan^{®}357),
- 3-(4'-sulfo)benzylidene bornan-2-one and salts,
- 2-ethylhexyl-2-cyano-3,3-diphenyl acrylate (Neo Heliopan^{®}303),
- N-[(2 and 4)-[2-(oxoborn-3-ylidene) methyl]benzyl] acrylamide polymer,
- p-methoxycinnamic acid-2-ethylhexyl ester (Neo Heliopan^{®}AV),
- p-aminobenzoic acid ethyl ester (25 mol) ethoxylated (INCI name: PEG-25 PABA),
- p-methoxycinnamic acid isoamyl ester (Neo Heliopan^{®}E1000),
- 2,4,6-trianilino-(p-carbo-2'-ethylhexyl-1'-oxy)-1,3,5-triazine (Uvinul^{®}T150),
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL),
- 4,4'-[(6-[4-(1,1-dimethyl)aminocarbonyl) phenylamino]-1,3,5-triazine-2,4-diyl)di-imino]-bis-(benzoic acid-2-ethylhexyl ester) (Uvasorb HEB),
- 3-(4'-methyl benzylidene)-D,L-camphor (Neo Heliopan^{®}MBC),
- 3-benzylidene camphor,
- salicylic acid-2-ethylhexyl ester (Neo Heliopan^{®}OS),
- 4-dimethylaminobenzoic acid-2-ethylhexyl ester (Padimate O),
- hydroxy-4-methoxybenzophenone-5-sulfonic acid and Na salt,
- 2,2'-methylene bis-(6-(2H-benzotriazol-2-yl)-4-1,1,3,3-tetramethylbutyl) phenol) (Tinosorb^{®}M),
- phenylene bis-benzimidazyl tetrasulfonic acid disodium salt (Neo Heliopan^{®}AP),
- 2,4-bis-[{(4-(2-ethylhexyloxy)-2-hydroxy}phenyl]-6-(4-methoxyphenyl)-1,3,5-triazine (Tinosorb^{®}S),
- benzylidene malonate polysiloxane (Parsol^{®}SLX),
- menthyl anthranilate (Neo Heliopan^{®}MA),
- 2-(4-diethylamino-2-hydroxybenzoyl) benzoic acid hexyl ester (Uvinul^{®} A Plus),
- indanylidene compounds,
and mixtures thereof.

In a further preferred variant the composition according to the invention contains a total amount of sunscreen agents, i.e. in particular UV filters and/or inorganic pigments (UV filtering pigments) so that the composition according to the invention has a sun protection factor SPF of 5 to 50, preferably 5 to 60. Such compositions according to the invention are particularly suitable for protecting the skin and hair.

Secondary sun protection factors: Besides the groups of primary sun protection factors mentioned above, secondary sun protection factors of the antioxidant type may also be advantageously used in the composition according to the present invention. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, MGDA (Trisodiumdicarboxymethyl alaninante), N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxy fatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts. The total quantity of inorganic pigments, in particular hydrophobic inorganic micro-pigments in the finished cosmetic preparation according to the present invention is advantageously from 0.1 to 30 % by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

**Actives modulating skin and/or hair pigmentation:** The composition according to the present invention may include active ingredients for skin and/or hair lightening. Preferred active ingredients for skin and/or hair lightening are selected from the group consisting of kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates, sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract.

Preferred skin lighteners are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophylline and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-Ile- Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(II) bicarbonate complexes ("pseudocat-alases"), tetrasubstituted cyclohexene derivatives, isoprenoids, melanin derivatives such as Melasyn-100 and MelanZe, diacyl glycerols, aliphatic or cyclic diols, psoralens, prostaglandins and ana-logues thereof, activators of adenylate cyclase and compounds which activate the transfer of melanosomes to keratinocytes such as serine proteases or agonists of the PAR-2 receptor, extracts of plants and plant parts of the chrysanthemum species, san-guisorba species, walnut extracts, urucum extracts, rhubarb extracts, microalgae extracts, in particular Isochrysis galbana, trehalose, erythrulose and dihydroxyacetone. Flavonoids which bring about skin and hair tinting or browning (e.g. quercetin, rhamnetin, kaempferol, fisetin, genistein, daidzein, chrysin and apigenin, epicatechin, diosmin and diosmetin, morin, quercitrin, naringenin, hesperidin, phloridzin and phloretin) can also be used.

Hair growth activators or inhibitors: The composition according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormones, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, the composition according to the present invention may include one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

**Enzyme inhibitors:** The composition according to the present invention may comprise one or more enzyme inhibitors. Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

Odour absorbers and/or antiperspirant active agents: The composition according to the present invention may include one or more odour absorbers and/or antiperspirant active agents (antiperspirants). Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour- neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

**Film formers:** The composition according to the present invention may include one or more film formers. Standard film formers are preferably chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

**Carriers and hydrotropes:** The composition according to the present invention may comprise a carrier or a mixture of different carriers. Preferred cosmetics carrier materials are solid or liquid at 25 °C and 1013 mbar (including highly viscous substances) as for example glycerol, 1,2-propylene glycol, 1,2-butylene glycol, 1,3-propylene glycol, 1,3-butylene glycol, ethanol, water, and mixtures of two or more of said liquid carrier materials with water.

The composition as defined herein may optionally include powders. The optional powders provide formulas that are smoother and softer on the skin. Representative powders include talc, mica, magnesium carbonate, calcium carbonate, magnesium silicate, aluminium magnesium silicate, silica, titanium dioxide, zinc oxide, red iron oxide, yellow iron oxide, black iron oxide, polyethylene powder, methacrylate powder, polystyrene powder, silk powder, Preferred solid powder materials, which may be a component of the composition according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin, inulin, and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine; and
- dialcoholamines, such as diethanolamine or 2-aminopropane-1,3-diol.

Other preferred liquid carrier substances, which may be a component of the composition according to the present invention are selected from the group consisting of oils such as vegetable oil, neutral oil and mineral oil.

The composition as defined herein, is preferably based on a carrier, which comprises at least one oil phase.

The oil phase or oil component in the composition according to the present invention which may be suitable are for example plant oils, hydrocarbons, fatty alcohols, fatty acid esters, or mixtures of two or more of the aforesaid oil components.

The oil phase or oil component in the composition is preferably a plant oil and even more preferably a liquid plant oil. It can also advantageously be a mixture of two or more plant oils components, especially liquid plant oil mixtures.

Plant oils or vegetable oils are oils extracted from seeds, or less often, from other parts of fruits. Like animal fats, plant oils are mixtures of triglycerides. Soybean oil, rapeseed oil and cocoa butter are examples of plant oils from seeds. Olive oil, palm oil and rice bran oil are examples of oils from other parts of fruits. In common usage, plant oil or vegetable oil may refer exclusively to vegetable fats which are liquid at room temperature or at 35 to 37 °C skin temperature. Vegetable oils are usually edible.

The term "plant oils" also includes unsaturated plant oils. Unsaturated oils or vegetable oils can be transformed through partial or complete "hydrogenation" into oils of higher melting point. The hydrogenation process involves "sparging" the oil at high temperature and pressure with hydrogen in the presence of a catalyst, typically a powdered nickel compound. As each carbon-carbon double-bond is chemically reduced to a single bond, two hydrogen atoms each form single bonds with the two carbon atoms. The elimination of double bonds by adding hydrogen atoms is called saturation; as the degree of saturation increases, the oil progresses toward being fully hydrogenated. An oil may be hydrogenated to increase resistance to rancidity (oxidation) or to change its physical characteristics. As the degree of saturation increases, the oil's viscosity and melting point increase.

In a preferred variant, the plant oil is selected from the group consisting of Persea Gratissima (Avocado Oil), Abies Alba Seed Oil, Acacia Victoriae Seed Oil, Actinidia Chinensis (Kiwi) Seed Oil, Amaranthus Hypochondriacus Seed Oil, Arachis Hypogaea (Peanut) Oil, Astrocaryum Murumuru Seed Butter, Astrocaryum Tucuma Seed Butter, Astrocaryum Tucuma Seed Oil, Astrocaryum Vulgare Fruit Oil, Astrocaryum Vulgare Kernel Oil, Avena Sativa (Oat) Kernel Oil, Brassica Alba Seed Oil, Brassica Campestris (Rapeseed) Seed Oil, Butyrospermum Parkii (Shea) Butter, Butyrospermum Parkii (Shea) Oil, Calendula Officinalis Seed Oil, Calophyllum Inophyllum Seed Oil, Calophyllum Tacamahaca Seed Oil, Camellia Oleifera Seed Oil, Camellia Reticulata Seed Oil, Camellia Sinensis Seed Oil, Cannabis Sativa Seed Oil, Cannabis Sativa Seed/Stem Oil, Canola Oil, Carthamus Tinctorius (Safflower) Seed Oil, Chlorella Vulgaris Oil, Citrullus Lanatus (Watermelon) Seed Oil, Citrus Aurantifolia (Lime) Seed Oil, Citrus Aurantium Dulcis (Orange) Seed Oil, Citrus Grandis (Grapefruit) Seed Oil, Cocos Nucifera (Coconut) Oil, Cocos Nucifera (Coconut) Seed Butter, Coffea Arabica (Coffee) Seed Oil, Chlorella Oil (biotech), Corylus Americana (Hazelnut) Seed Oil, Corylus Avellana (Hazelnut) Seed Oil, Cucumis Melo (Melon) Seed Oil, Cucumis Sativus (Cucumber) Seed Oil, Cucurbita Pepo (Pumpkin) Seed Oil, Elaeis Guineensis (Palm) Oil, Elaeis (Palm) Fruit Oil, Glycine Soja (Soybean) Oil, Gossypium Herbaceum (Cotton) Seed Oil, Gossypium Hirsutum (Cotton) Seed Oil, Helianthus Annuus (Sunflower) Seed Oil, Macadamia Integrifolia Seed Oil, Macadamia Ternifolia Seed Oil, Mangifera Indica (Mango) Seed Butter, Mangifera Indica (Mango) Seed Oil, Melissa Officinalis Seed Oil, Microalgae Oil, Moringa Oleifera Seed Oil, Moringa Peregrina Seed Oil, Oenothera Biennis (Evening Primrose) Oil, Olea Europaea (Olive) Fruit Oil, Olus Oil, Orbignya Oleifera Seed Oil, Orbignya Speciosa Kernel Oil, Oryza Sativa (Rice) Bran/Germ Oil, Oryza Sativa (Rice) Bran Oil, Oryza Sativa (Rice) Germ Oil, Oryza Sativa (Rice) Lipids, Oryza Sativa (Rice) Seed Oil, Papaver Somniferum Seed Oil, Passiflora Edulis Seed Oil, Persea Gratissima (Avocado) Butter, Persea Gratissima (Avocado) Oil, Prunus Amygdalus Dulcis (Sweet Almond) Oil, Prunus Armeniaca (Apricot) Kernel Oil, Prunus Persica (Peach) Kernel Oil, Punica Granatum Seed Oil, Pyrus Malus (Apple) Seed Oil, Ricinoleic/Caproic/Caprylic/Capric Triglyceride(s), Ricinus Communis (Castor) Seed Oil, Rosa Canina Fruit Oil, Rosa Moschata Seed Oil, Rubus Idaeus (Raspberry) Seed Oil, Sesamum Indicum (Sesame) Seed Butter, Soybean Glycerides, Theobroma Cacao (Cocoa) Seed Butter, Theobroma Grandiflorum Seed Butter, Triticum Vulgare (Wheat) Bran Lipids, Triticum Vulgare (Wheat) Germ Oil, Vitis Vinifera (Grape) Seed Oil, Zea Mays (Corn) Germ Oil, and Zea Mays (Corn) Oil.

In a particular preferred variant, the composition according to the present invention comprises as oil component a plant oil selected from the group consisting of Caprylic Capric Triglycerides, Helianthus Annuus (Sunflower) Seed Oil, Simmondsia Chinensis ((Jojoba) Seed Oil, Olea Europaea (Olive) Fruit Oil, Argania spinosa kernel oil (Argan oil), Prunus Amygdalus Dulcis (Sweet Almond) Oil, Persea Gratissima (Avocado Oil), Butyrospermum Parkii (Shea) Butter, Cocos Nucifera (Coconut) Oil, Theobroma Cacao (Cocoa) Seed Butter, and mixtures of two or more of the aforesaid plant oils.

The plant oil can be used either as a single component or in mixture with one or more further different plant oil(s) as specified above.

Hydrocarbons (mineral oils) are in general organic compounds consisting entirely of hydrogen and carbon. As defined by IUPAC nomenclature or organic chemistry, the classifications for hydrocarbons are:
2. Saturated hydrocarbons are the simplest of the hydrocarbon species. They are composed entirely of single bonds and are saturated with hydrogen. The formula for acyclic saturated hydrocarbons (i.e., alkanes) is C*ₙ*H_{*2n*+2}.The most general form of saturated hydrocarbons is CₙH₂ₙ₊₂₍₁₋ᵣ₎, where *r* is the number of rings. Those with exactly one ring are the cycloalkanes. Saturated hydrocarbons are the basis of petroleum fuels and are found as either linear or branched species.
3. Unsaturated hydrocarbons have one or more double or triple bonds between carbon atoms. Those with double bond are called alkenes. Those with one double bond have the formula CₙH₂ₙ (assuming non-cyclic structures). Those containing triple bonds are called alkynes. Those with one triple bond have the formula CₙH₂ₙ₋₂.
4. Aromatic hydrocarbons, also known as arenes, are hydrocarbons that have at least one aromatic ring.

Hydrocarbons can be inter alia liquids (e.g. hexane and benzene), waxes or low melting solids (e.g. paraffin wax and naphthalene). The term 'aliphatic' refers to non-aromatic hydrocarbons. Saturated aliphatic hydrocarbons are sometimes referred to as "paraffins". Mineral oils and waxes are mixtures of predominantly saturated hydrocarbons consisting of straight-chain, branched and ring structures with carbon chain lengths greater than C14. Mineral oils and waxes are chemical substances prepared from naturally occurring crude petroleum oil. They mainly consist of mineral oil saturated hydrocarbons (MOSH) and mineral oil aromatic hydrocarbons (MOAH). Hydrocarbons have been used for many decades in skin and lip care cosmetic products due to their excellent skin tolerance as well as their high protecting and cleansing performance and broad viscosity options. In contrast to vegetable oils, mineral oils are non-allergenic since they are highly stable and not susceptible to oxidation or rancidity.

In a preferred variant, the hydrocarbon is selected from the group consisting of undecane, tridecane, mineral oil, petrolatum squalane, isohexadecane, C7 - C8 isoparaffin, C8 - C9 isoparaffin, C9 - C11 isoparaffin, C9 - C12 isoparaffin, C9 - C13 isoparaffin, C9 - C14 isoparaffin, C9 - C16 isoparaffin, C10 - C11 isoparaffin, C10 - C12 isoparaffin, C10 - C13 isoparaffin, C11 - C12 isoparaffin, C11 - C13 isoparaffin, C11 - C14 isoparaffin, C12 - C14 isoparaffin, C12 - C15 isoparaffin, C12 - C20 isoparaffin, C13 - C14 isoparaffin, C13 - C16 isoparaffin, C14 - C16 isoparaffin, C15 - C19 isoparaffin, and C18 - C70 isoparaffin.

The hydrocarbon can be used either as a single component or in mixture with one or more further different hydrocarbon(s) as specified above.

A fatty alcohol (or long-chain alcohol) is usually a high-molecular-weight, straight-chain primary alcohol, but can also range from as few as 4 to 6 carbons to as many as 22 to 26, derived from natural fats and oils. The precise chain length varies with the source. Some commercially important fatty alcohols are lauryl, stearyl and oleyl alcohols. They are colourless oily liquids (for smaller carbon numbers) or waxy solids, although impure samples may appear yellow. Fatty alcohols usually have an even number of carbon atoms and a single alcohol group (-OH) attached to the terminal carbon. Some are unsaturated and some are branched. Most fatty alcohols in nature are found as waxes which are esters with fatty acids and fatty alcohols. The traditional sources of fatty alcohols have largely been various vegetable oils and these remain a large-scale feedstock. The alcohols are obtained from the triglycerides (fatty acid triesters), which form the bulk of the oil. The process involves the transesterification of the triglycerides to give methyl esters which are then hydrogenated to give the fatty alcohols. Fatty alcohols are also prepared from petrochemical sources. In the Ziegler process, ethylene is oligomerized using triethylaluminium followed by air oxidation. Alternatively, ethylene can be oligomerized to give mixtures of alkenes, which are subjected to hydroformylation, this process affording odd-numbered aldehyde, which is subsequently hydrogenated. Fatty alcohols are mainly used in the production of detergents and surfactants. They are components also of cosmetic solvents. They find use as co-emulsifiers, emollients and thickeners in cosmetics.

In a preferred variant, the fatty alcohol is selected from the group consisting of phenyl propanol, dimethyl phenylbutanol, hexyldecanol, octyldodecanol, octyldecanol, tridecylalcohol, isostearyl alcohol, phenylisohexanol, phenylpropanol, trimethylbenzenepropanol, isoamylalcohol, isostearyl alcohol, and isotridecyl alcohol. The fatty alcohol can be used either as a single component or in mixture with one or more further different fatty alcohol(s) as specified above.

A fatty acid ester is a type of ester that results from the combination of a fatty acid with an alcohol. When the alcohol component is glycerol, the fatty acid esters produced can be monoglycerides, diglycerides or triglycerides. Fatty acid esters have a conditioning effect of softening the skin to create a smoothing sensation. They are also added to cosmetics to dissolve high-polarity active ingredients and UV absorbers. Esters of straight-chain fatty acids and lower alcohols are effective for dissolving slightly soluble ingredients for oils with a light touch during application. Isostearic acids and other liquid oils with branched fatty acids and unsaturated fatty acids are commonly used as emollients. Higher fatty acid esters and esters of higher alcohols with relatively high melting points are added to skin creams to adjust the application touch.

In a preferred variant, the fatty acid ester is selected from the group consisting of C12 - C15 Alkyl Benzoate, Caprylic/Capric Triglyceride, Caprylic/Capric/Lauric Triglyceride, Caprylic/Capric/Linoleic Triglyceride, Caprylic/Capric/Myristic/Stearic Triglyceride, Caprylic/Capric/Palmitic/Stearic Triglyceride, Caprylic/Capric/Stearic Triglyceride, Caprylic/Capric/Succinic Triglyceride, Caprylyl Caprylate, Cetearyl Ethylhexanoate, Cetearyl Isononanoate, Cetearyl Nonanoate, Coco-Caprylate, Decyl Cocoate, Decyl Oleate, Dicaprylyl Carbonate, Diethyl Succinate, Diethylhexyl 2,6-Naphthalate, Diethylhexyl Carbonate, Dibutyl Adipate, Diisopropyl Adipate, Dipropylheptyl Carbonate, Ethyl Laurate, Ethylhexyl Isononanoate, Ethylhexyl Palmitate, Ethylhexyl Stearate, Glyceryl Caprylate Caprate, Glyceryl Caprylate, Glyceryl Laurate, Glyceryl Triacetyl Hydroxystearate, Glyceryl Triacetyl Ricinoleate, Hexyl Laurate, Isoamyl acetate, Isoamyl Cocoate, Isopropyl Palmitate, Isosorbide Dicaprylate, Isopropyl Myristate, Myristyl Myristate, Oleic/Linoleic Triglyceride, Oleic/Palmitic Triglyceride, Oleic/Palmitic/Lauric/Myristic/Linoleic Triglyceride, Propanediol Caprylate, Ricinoleic/Caproic/Caprylic/ Capric Triglyceride, Oleostearine, Oleyl Erucate, Palmitic/Stearic Triglyceride, Propanediol Dicaprylate Caprate, Propylheptyl Caprylate, Stearyl Heptanoate/Stearyl Caprylate, Triheptanoin, Trihydroxystearin, Triisononanoin, Triisopalmitin, Triisostearin, Trilaurin, Trilinolein, Trilinolenin, Trimyristin, Triolein, Tripalmitin, Tripalmitolein, Tripelargonin, Triricinolein, and Tristearin.

The fatty acid ester can be used either as a single component or in mixture with one or more further different fatty ester(s) as specified above.

In addition to the oil phase or oil component as defined above, the composition according to the present invention preferably includes one or more oil bodies. Suitable oil bodies, which form constituents of the O/W emulsions, are, for example, Guerbet alcohols based on fatty alcohols having 6 to 18, preferably 8 to 10, carbon atoms, esters of linear C5 - C22 fatty acids with linear or branched C5 - C22 fatty alcohols or esters of branched C1 - C13 carboxylic acids with linear or branched C6 - C22 fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6 - C22 fatty acids with branched alcohols, in particular 2-ethylhexanol, esters of C18 - C38 alkylhydroxy carboxylic acids with linear or branched C6 - C22 fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C6 - C10 fatty acids, liquid mono-/di-/triglyceride mixtures based on C6 - C18 fatty acids, esters of C6 - C22 fatty alcohols and/or Guerbet alcohols with aromatic carboxylic acids, in particular benzoic acid, esters of C2 - C12 dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched CC - C22 fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

Dyes: The composition according to the present invention may comprise one or more dyes. Suitable dyes are any of the substances suitable and approved for cosmetic purposes. Examples include cochineal red A (CI. 16255), patent blue V (CI. 42051), indigotin (CI. 73015), chlorophyllin (CI. 75810), quinoline yellow (CI. 47005), titanium dioxide (CI. 77891), indanthrene blue RS (CI. 69800) and madder lake (CI. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

In addition to the above-described substances, further ingredients commonly used in the food industry, cosmetic or pharmaceutical industry or for homecare products, which are suitable or customary in the compositions of the present invention, can be used.

The 2,3-octanediol or the mixture comprising 1,2-octanediol and 2,3-octanediol are present in the composition according to the present invention or in the ready to use product, i.e., in the final food, cosmetic or pharmaceutical preparation or homecare product, in an amount of 0.001 to 15.0 % by weight, based on the total weight of the composition or final food, cosmetic or pharmaceutical preparation or homecare product. In a preferred variant, the composition or the final preparation comprises the 2,3-octanediol or the mixture comprising 1,2-octanediol and 2,3-octanediol in an amount of 0.01 to 10.0 % by weight, based on the total weight of the composition or final food, cosmetic or pharmaceutical preparation or homecare product. In a more preferred variant, the 2,3-octanediol or the mixture comprising 1,2-octanediol and 2,3-octanediol is advantageously used in the composition or final preparation in an amount of 0.1 to 5.0 % by weight, based on the total weight of the composition or final food, cosmetic or pharmaceutical preparation or homecare product. In a still more preferred variant, the 2,3-octanediol or the mixture comprising 1,2-octanediol and 2,3-octanediol is advantageously used in the composition or final preparation in an amount of 0.3 to 3.0 % by weight, based on the total weight of the composition or final food, cosmetic or pharmaceutical preparation or homecare product. In a most preferred variant, the 2,3-octanediol or the mixture comprising 1,2-octanediol and 2,3-octanediol is advantageously used in the composition or final preparation in an amount of of 0.5 to 1.0 % by weight, based on the total weight of the composition or final food, cosmetic or pharmaceutical preparation or homecare product.

For the octanediol mixture, the above amounts relate to the total content of the 1,2-octanediol and the 2,3-octanediol in the mixture, i.e. the amount is the sum of the content of the 1,2-octanediol and 2,3-octanediol in the mixture.

In a particular preferred variant according to the present invention, the composition comprises the 2,3-octanediol as linear alkanediol or of the mixture comprising 1,2-octanediol and 2,3-octanediol in an amount of 0.001 to 15.0 % by weight, preferably in an amount of 0.01 to 10.0 % by weight, more preferred in an amount of 0.1 to 5.0 % by weight, still more preferred in an amount of 0.3 to 3.0 % by weight, and most preferred in an amount of 0.5 to 1.0 % by weight, based on the total weight of the composition or final food, cosmetic or pharmaceutical preparation or homecare product.

Even more preferred, the composition comprises the 2,3-octanediol as linear alkanediol or of the mixture comprising 1,2-octanediol and 2,3-octanediol in an amount of 0.001 to 0.5 % weight, preferably in an amount of 0.005 to 0.1 % by weight and most preferably in an amount of 0.01 to 0.075 % by weight, based on the total weight of the composition or final food, cosmetic or pharmaceutical preparation or homecare product.

The antimicrobial component 4-hydroxyacetophenone is present in the composition according to the present invention or the final cosmetic or pharmaceutical preparation in an amount of 0.01 to 25.0 % by weight, based on the total weight of the composition or final food, cosmetic or pharmaceutical preparation or homecare product. In a preferred variant, the composition or the final food, cosmetic or pharmaceutical preparation or homecare product comprises the antimicrobial component 4-hydroxyacetophenone in an amount of 0.02 to 15.0 % by weight, based on the total weight of the composition or final cosmetic or pharmaceutical preparation. In a particular preferred variant, the antimicrobial component 4-hydroxyacetophenone is advantageously used in the composition or in the final food, cosmetic or pharmaceutical preparation or homecare product in an amount of at 0.05 to 6.0 % by weight, based on the total weight of the composition or final food, cosmetic or pharmaceutical preparation or homecare product.

A synergistic antimicrobial effect is also achieved for 2,3-alkanedioles, namely 2,3-octanediol. The use of the 2,3-alkanediols also considerably boosts the antimicrobial effect of an antimicrobial component against bacteria, yeast and fungi/mould in food, a cosmetic or pharmaceutical preparation or a homecare product. The use of 2,3-octanediol results in a synergistic antimicrobial efficacy in combination with an antimicrobial component.

Additionally, the 2,3-alkanediols have the advantage that they are liquid, and, thus, can be easier incorporated into semi-finished products or final products and furnished in higher conentrations in the final product. Hence, a good compromise can be achieved when the 2,3-alkanediol is combined in such a way as to maintain the antimicrobial effect, while improving processability in formulations.

The blend including 1,2-octanediol and 2,3-octanediol, when used with the antimicrobial component 4-hydroxyacetophenone considerably enhances the antimicrobial effect of an antimicrobial component as it is demonstrated in the following examples.

The synergistic antimicrobial effect obtained by use of an alkanediol or a mixture of alkanediols according to the present invention has the advantage that the antimicrobial efficiency of the antimicrobial component 4-hydroxyacetophenone is broadened, either by achieving a higher performance with the same amounts, or, vice versa, by achieving the same performance with lower amounts of the antimicrobial component 4-hydroxyacetophenone, so that the overall content of the antimicrobial component in the final product can be reduced, compared to a final product including an antimicrobial component without the addition of a composition according to the present invention.

Due to the described advantageously properties, the composition according to the present invention can be used for the preparation of food, cosmetic or pharmaceutical **preparations** or homecare products which can be microbially stabilized thereby. The microbial stabilization allows the food, cosmetic or pharmaceutical preparations or homecare product to be stored without microbial degradation and deterioration, thus improving the shelf life of a cosmetic or pharmaceutical preparation.

Hence, the present invention relates in a further aspect of the present invention to food, cosmetic or pharmaceutical preparations or homecare products comprising the antimicrobial composition according to the present invention.

The cosmetic or pharmaceutical, in particular dermatological, composition according to the present invention is intended for topical applications. The term "topical" is understood to mean external applications on a mammal's skin, which are in particular for the treatment, protection, care and cleansing of the skin, scalp, eyelashes, eyebrows, nails, mucous membranes and hair. The mammal is preferably a human.

For topical application, the cosmetic or pharmaceutical preparation is either a rinse off or leave on preparation.

The cosmetic or pharmaceutical, in particular dermatological, preparation according to the present invention can be present in different forms of, e.g. in the form of a dispersion, in the form of a liquid surfactant formulation, in the form of a solid surfactant formulation, or in the form of an aqueous, aqueous/alcoholic, in particular aqueous/ethanolic, or an aqueous/glycolic solution.

In a particular preferred variant, the cosmetic or pharmaceutical preparation according to the present invention is a cold formulation preparation, which can be prepared without a heating step. In comparison to solid alkanediols, the use of liquid alkanediols, having the advantageous effects as described before, has the benefit that they can be easily used in liquid polymer dispersions without heating, for example in the preparation of cold processed cosmetic or pharmaceutical preparations.

In a preferred variant, the cosmetic or pharmaceutical preparation according to the present invention is a dispersion. The term "dispersion" in the context of the present invention means, that the cosmetic or pharmaceutical preparation is a disperse two-phase system consisting of colloidal particles (disperse phase) and a medium in which they are suspended (disperse medium). Both phases are not miscible with each other, only with an emulsifier. Such dispersions, for example emulsions, comprise the oil component preferably in an amount of ≥ 1 % by weight, more preferably in an amount of ≥ 3 % by weight.

If the cosmetic or pharmaceutical preparation according to the present invention is a dispersion, preferably an emulsion, the oil component is present in the cosmetic or pharmaceutical preparation in an amount of 0.01 % to 50.0 by weight, based on the total weight of the composition. In a preferred variant, the cosmetic or pharmaceutical preparation comprises the oil component in an amount of 1.0 to 40.0 % by weight, based on the total weight of the composition. In a particular preferred variant, the oil component is advantageously used **in the cosmetic** or pharmaceutical preparation in an amount of at 3.0 to 30 % by weight, based on the total weight of the composition.

Preferably, the cosmetic or pharmaceutical preparation according to the present invention takes various forms such as an emulsion, in particular a O/W emulsion, a W/O emulsion, a multiple emulsion, a hydro dispersion gel, a balm, a multiple emulsion of the water-in-oil type (W/O/WO) or of the oil-in-water type (O/W/O), PIT emulsion, Pickering emulsion, a micro-emulsion, a liquid, a lotion, a suspension, a milk, an ointment, a paste, a gel, a cream based, an oil based or a liposome-based formulation.

In a further alternative, the cosmetic or pharmaceutical preparation according to the present invention is a liquid surfactant formulation.

Such liquid surfactant formulations include for example shampoo, shower gel, micellar water, liquid soap, cleansing preparations.

If the cosmetic or pharmaceutical preparation according to the present invention is a liquid surfactant composition, the surfactant component is present in the cosmetic or pharmaceutical preparation in an amount of 1 to 40 % by weight, preferably in an amount of 3 to 30 % by weight, more preferably in an amount of 5 to 25 % by weight, based on the total weight of the composition.

In a further alternative, the cosmetic or pharmaceutical preparation according to the present invention is a solid surfactant formulation.

Such solid surfactant formulations include for example solid shampoos, solid body wash, bar soaps.

Alternatively and particularly preferred, the cosmetic or pharmaceutical preparation as disclosed herein is an aqueous or aqueous/alcoholic or aqueous/glycolic based solution. The aqueous/alcoholic or aqueous/glycolic based solution comprises an aliphatic alcohol or a glycol in an amount of 0.1 to ≤ 50 % by weight, based on the total weight of the solution. The aliphatic alcohol is preferably selected from the group consisting of ethanol, isopropanol, n-propanol. The glycol is preferably selected from the group consisting of glycerin, propylene glycol, butylene glycol or dipropylene glycol. Preferably, the overall water content in the final composition of such compositions can be ≥ 60 %, more preferably ≥ 70 %, even more preferably ≥ 80 %, even more preferably ≥ 90 %, and in special cases preferably ≥ 90 %. New applications such as those including water wipes have high water content. In a particular variant, the inventive compositions can be used for such wet wipe applications. They may then most preferably contain ≥ 95 % water, or even ≥ 98 % water.

Such aqueous or aqueous/alcoholic or aqueous/glycolic based solutions include for example deo/antiperspirant roll-ons, after shaves, cleansing preparations, anti-acne preparations, or wet wipe solutions.

In one preferred variant, the cosmetic or pharmaceutical preparation according to the present invention is in the form of an emulsion as defined herein, advantageously in the form of an oil-in-water (O/W) emulsion comprising an oily phase dispersed in an aqueous phase in the presence of an O/W emulsifier.

In a most preferred variant, the cosmetic or pharmaceutical preparation according to the present invention is in the form of an aqueous or aqueous/alcoholic, preferably aqueous/ethanolic, or aqueous/glycolic based solution. Typically, this could be glycerin in water compositions.

The above cosmetic or pharmaceutical preparations of formulations are prepared according to usual and known methods.

In a preferred variant, the cosmetic preparation according to the present invention is a preparation for cosmetic and/or non-therapeutic use for personal care, skin protection, skin care, scalp protection, scalp care, hair care, nail care, in particular for the prevention and/or treatment of skin conditions, intolerant or sensitive skin, skin irritation, skin reddening, rosacea, wheals, pruritus (itching), skin aging, wrinkle formation, loss of skin volume, loss of skin elasticity, pigment spots, pigment abnormalities, skin dryness, flaking, greasiness, hypopigmentation and/or hyperpigmentation of the skin; or for animal care.

Examples of personal care are preferably anti-ageing preparations, skin care emulsions, body oils, body lotions, cleansing lotions, face or body balms, after shave balms, after sun balms, deo emulsions, cationic emulsions, body gels, treatment creams, skin protection ointments, moisturizing gels, face and/or body moisturizers, light protective preparations (sunscreens), micellar water, hair spray, colour protection hair care products, skin lightening product, anti-dark spot preparations, etc.

The pharmaceutical, in particular dermatological, preparation according to the present invention is a preparation for the prevention and treatment of a condition of the skin or mucosa.

In order to be used, the cosmetic or pharmaceutical, in particular dermatological preparation according to the present invention is applied to the skin, hair, scalp and/or nails in an adequate amount in such manner as is customary with cosmetics and dermatological products.

The homecare products according to the present invention are predominantly detergent formulations, usually liquids, powders, sprays, granules or tablets, used to remove dirt, including dust, stains, bad smells and clutter on surfaces or other types of housecleaning or laundry detergent that is added for cleaning laundry or liquid soap. Typical homecare products include all purpose cleaners, dishwashing detergens, hard floor and surface cleaners, glass cleaners, carpet cleaners, oven cleaners, laundry detergents, fabric softeners, laundry scent, scent lotions, car shampoo, rim block gel, all aforementioned goods also in encapsulated form, air fresheners or furniture polish.

Due to their outstanding antimicrobial efficacy as described above, the composition according to the present invention is suitable for the preservation of food, cosmetic or pharmaceutical preparations or homecare products.

Thus, in a further aspect the present invention relates to the use of the composition according to the present invention for suppressing or inhibiting microorganism growth in food, a cosmetic or pharmaceutical preparations or homecare products. Here the present compositions are valuable in ensuring good preservation of said products. The suppression or inhibition of the growth of microorganisms is a reduction of the microorganisms' growth rate and/or stagnation or reduction of the number of living microorganisms. By suppressing or inhibiting the microorganisms' growth, the degradation and deterioration of the cosmetic or pharmaceutical preparation, in particular during storage, which sometimes goes along with impairment of the sensory quality of the products, can be prevented or at least minimized.

A use is further preferred, wherein the microorganisms are selected from the group consisting of the genus Pseudomonas, Staphylococcus, Echerichia, Candida, Aspergillus, and combinations thereof, in particular selected from the group consisting of *Pseudomonas aeruginosa, Staphylococcus aureus, Escherichia coli, Candida albicans* und *Aspergillus brasiliensis,* and combinations thereof, more particular *Candida albicans* und *Aspergillus brasiliensis.* The antimicrobial composition according to the present invention showed excellent efficacy against these microorganism.

Surprisingly, 2,3-octanediol or an alkaned mixture including 1,2-octanediol and 2,3-octanediol combination with the antimicrobial component 4-hydroxyacetophenone has been found to synergistically enhance the antimicrobial effect thereof.

Thus, the present invention relates in a further aspect to the use of 2,3-octanediol or a mixture comprising 1,2-octanediol and 2,3-octanediol for boosting the efficacy of 4-hydroxyacetophenone, preferably in food, a cosmetic or pharmaceutical preparation or a homecare product.

In addition, due to the above-described advantageous properties resulting from the use of 2,3-heptanediol or a mixture comprising1,2-octanediol and 2,3-octanediol, the amount of the antimicrobial agent 4-hydroxacetophenone in a cosmetic or pharmaceutical preparation can be reduced.

Finally, the present invention also relates to a method for suppressing or inhibiting of microorganisms' growth in food, a cosmetic or a pharmaceutical preparation or homecare product as defined herein. In the method according to the invention a food, a cosmetic or a pharmaceutical preparation or homecare product is provided. Next, an effective amount of an antimicrobial composition according to the present invention is incorporated into the food, cosmetic or pharmaceutical preparation or homecare product thereby achieving preservation of said products.

The present invention shall now be described in detail with reference to the following examples, which are merely illustrative of the present invention, such that the content of the present invention is not limited by or to the following examples.

### Examples

The following preservation efficacy tests (PET) were performed to show the microbial efficacy of compositions according to the present invention and in particular to demonstrate the boosting, i.e. synergistic antimicrobial efficacy of said compositions.

For carrying out the preservation efficacy test in different media, the following specified antimicrobial components were incorporated into different cosmetic preparations as described below:

### Preparation (1): O/W emulsion, anionic

**Table 1:**

| **Ingredients** | **INCI** | **w/w %** |
|---|---|---|
| **Phase A** | | |
| Carbopol ETD 2050 ex Lubrizol | Carbomer | 0.2 |
| PCL Solid | Stearyl Heptanoate, Stearyl Caprylate | 4.0 |
| Dragoxat^{®} 89 | Ethylhexyl Isononanoate | 5.0 |
| Lanette 18 ex BASF | Stearyl Alcohol | 2.0 |

| **Phase B** | | |
|---|---|---|
| Water (ultrapure) | Water (Aqua) | ad 100 |
| Dracorin^{®} CE | Glyceryl Stearate Citrate | 3.0 |

| **Phase C** | | |
|---|---|---|
| NaOH 10% aqueous sol. | Sodium Hydroxyide | 0.3 |

| **Phase D** | | |
|---|---|---|
| Antimicrobial | | s.a |
| pH-value | | 5.8 |

Production method: Heat phases A and B (without Carbopol ETD 2050) separately to approx. 80 °C. Add Carbopol ETD 2050 to phase A and homogenize. Add phase B to phase A and emulsify (Ultra Turrax Stirrer, 2 min, 4000 rpm/min). Allow to cool by using a vane stirrer, reduce stirring speed while base is cooling. Adjust the pH value with Phase C. Add phase D and stir until a homogeneous emulsion is achieved.

### Preparation (2): O/W emulsion, anionic

**Table 2:**

| **Ingredients** | **INCI** | **w/w %** |
|---|---|---|
| **Phase A** | | |
| Carbopol ETD 2050 | Carbomer | 0.2 |
| Keltrol CG | Xanthan Gum | 0.1 |
| Emulsiphos | Potassium Cetyl Phosphate, Hydrogenated Palm Glycerides | 1.0 |
| SymMollient S | Cetearyl Nonanoate | 1.0 |
| Neutral Oil | Caprylic/Capric Triglycerides | 5.0 |
| Lanette 18 | Stearyl Alcohol | 1.5 |
| PCL Liquid 100 | Cetearyl Ethylhexanoate | 4.0 |
| Xiameter 246 | Cyclohexasiloxane, Cyclopentasiloxane | 2.0 |

| **Phase B** | | |
|---|---|---|
| Water (ultrapure) | Water (Aqua) | ad 100 |

| **Phase C** | | |
|---|---|---|
| NaOH 10% aqueous sol. | Sodium Hydroxide | 0.6 |

| **Phase D** | | |
|---|---|---|
| Antimicrobial | | 0.6 |
| **pH-value** | | 5.8 |

Production Method: Heat phases A (without Carbomer and Xanthan Gum) and B up to 80 °C. Disperse Carbomer and Xanthan Gum in phase A. Add phase B A and homogenize by using an Ultra Turrax Stirrer. Allow to cool down to ambient temperature by stirring with a vane stirrer. Add phase D and stir until a homogeneous emulsion is achieved.

### Preparation (3): O/W emulsion, anionic, cold process

**Table 3:**

| **Ingredients** | **INCI** | **w/w %** |
|---|---|---|
| **Phase A** | | |
| Dracorin^{®} GOC | Glyceryl Oleate Citrate Caprylic/Capric Triglyceride | 2.0 |
| PCL Liquid^{®} 100 | Cetearyl Ethylhexanoate | 8.0 |
| Neutral Oil | Caprylic/Capric Triglyceride | 7.0 |
| Xiameter PMX 0246 | Cyclohexasiloxane | 2.0 |

| **Phase B** | | |
|---|---|---|
| Water dem. | Water (Aqua) | ad 100 |
| Carbopol ETD 2020 | Acrylates/C10-30 Alkyl Acrylate Crosspolymer | 0.2 |
| Glycerin 85% | Glycerin | 2.0 |
| Antimicrobial | | - |

| **Phase C** | | |
|---|---|---|
| NaOH 10% aqueous sol. | Sodium hydroxide | 0.2 |
| pH-value | | 6.0 |

Production method: Disperse Carbopol ETD 2020 in water, add the other ingredients of phase B. Premix phase A. Add phase B to phase A and emulsify by using an Ultra Turrax Stirrer. Add phase C to adjust the pH value.

### Preparation (4): Wet wipe solution

**Table 4:**

| **Ingredients** | **INCI** | **w/w %** |
|---|---|---|
| Propylenglycol | Propylene Glycol | 8.0 |
| Solubilizer | PEG-40 Hydrogenated Castor Oil, Trideceth-9, Propylene Glycol, Water (Aqua) | 2.0 |
| Antimicrobial | - | - |
| Water (ultrapure) | Water (Aqua) | ad 100 |
| Citric Acid, 10% sol. | Citric Acid | 0.1 |
| pH-value | | 6.0 |

Production method: Presolve the antimicrobial in solubilizer, add propylene glycol and water while stirring. Adjust the pH value with citric acid solution. Ratio non woven/wet wipe solution 1 : 6. Non-woven quality, ex Sandler: Spunlace (Polyester/Viscose).

### Preparation (5): Shampoo

**Table 5:**

| | **Ingredient** | **INCI** | **w/w %** |
|---|---|---|---|
| **A.** | Plantacare PS 10, ex BASF | Sodium Laureth Sulfate, Lauryl Glucoside | 17.00 |
| | Citric Acid | Citric Acid | 0.15 |
| | Sodium Chloride | Sodium Chloride | 0.40 |
| **B.** | Water (ultrapure) | Water (aqua) | Ad 100 |
| **C.** | Tego Betain F50, ex Evonik | Cocoamidopropyl Betaine | 5.00 |
| D. | Antimicrobial | - | - |

Production method: Blend Plantacare PS10, sodium chloride and citric acid slowly (avoid foaming). Add water step by step while stirring by using a vane stirrer. Add phase C and D one after the other while stirring. Adjust the pH value to pH 5.8.

In case of solubility issues, it may be impossible to incorporate the antimicrobial afterwards into the bulk. Then the antimicrobial should be pre-solved in parts of the water or surfactant phase.

### Preparation (6): Sun: O/W emulsion, SPF 30

**Table 6:**

| | **Ingredient** | **INCI** | **w/w %** |
|---|---|---|---|
| **A.** | Emulsiphos^{®} | Potassium Cetyl Phosphate, Hydrogenated Palm Glycerides | 2.00 |
| | PCL Solid | Stearyl Heptanoate, Stearyl Caprylate | 2.00 |
| | Lanette 18 ex. BASF | Stearyl Alcohol | 1.50 |
| | Dragoxat^{®} 89 | Ethylhexyl Isononanoate | 2.00 |
| | Neutral Oil | Caprylic/Capric Triglycerides | 3.00 |
| | Tegosoft TN ex. Evonik | C12-15 Alkyl Benzoate | 3.00 |
| | Neo Heliopan^{®} BB | Benzophenone-3 | 6.00 |
| | Neo Heliopan^{®} HMS | Homosalate | 10.00 |
| | Neo Heliopan^{®} OS | Ethylhexyl Salicylate | 5.00 |
| | Neo Heliopan^{®} 357 | Butyl Methoxydibenzoylmethane | 3.00 |
| | Neo Heliopan^{®} AV | Ethylhexyl Methoxycinnamate | 7.50 |
| **B.** | Carbopol ETD 2050 ex Lubrizol | Carbomer | 0.20 |
| | Keltrol T ex CP Kelco | Xanthan Gum | 0.20 |
| **C.** | Water (ultrapure) | Water (Aqua) | Ad 100 |
| | Glycerin 99°/ig | Glycerin | 3.00 |
| | Butylenglycol | Butylene Glycol | 2.00 |
| | Antimicrobial | | s.a. |
| | EDTA BD ex. BASF | Disodium EDTA | 0.10 |
| **D.** | AMP 95 ex. Angus | Aminomethyl Propanol | 0.15 |
| **E.** | Antimicrobial | - | - |
| | pH-value | | 5.5 |

**Production method:** Heat phase A to 80 °C. Add phase B to A and disperse. Heat phase C and add to phases AB and emulsify by using an Ultra Turrax Stirrer (3 min, 6000 u/min). Allow to cool down by using a vane stirrer. Add phase D to neutralize. Adjust the pH value to 5.5. Add phase E and stir until a homogeneous emulsion is achieved.

In some cases, due to solubility issues it is impossible to incorporate the respective stabilizer/preservative into the bulk, then it can be added to the water or oil phase.

The preservation efficacy test (PET) was performed in accordance with European Pharmacopoeia § 5.1.3: The test is a reference European Pharmacopoeia method, that is to be used to evaluate the preservation system of a cosmetic or pharmaceutical preparation. The test consists of challenging the preparation under test, with prescribed inoculums of suitable microorganisms. The preservation properties of the preparation are adequate if, in the conditions of the test, there is a significant fall or no increase, as appropriate, in the number of microorganisms in the inoculated preparations after the time prescribed.

The test microorganisms which were used, are the following:
Pseudomonas aeruginosa (PS) (bacterium);
Staphylococcus aureus (SA) (bacterium);
Escherichia coli (EC) (bacterium);
Candida albicans (CA) (yeast); and
Aspergillus brasiliensis (AB) (fungus).

### Example 1: Synergistic antimicrobial effect against bacteria, yeast and fungi/mould

**(not in accordance with the present invention)**

### Example 1.2: Antimicrobial efficacy of 1,2-heptanediol, 2,3-heptanediol, 2,3-octanediol and 2,3-nonanediol in shampoo (not in accordance with the present invention)

**Table 8:**

| **Substance** | | **Inoculum** | **2 days** | **7 days** | **14 days** | **28 days** |
|---|---|---|---|---|---|---|
| 0,1 % 1,2 Heptanediol | PA | 4.700.000 | 2.500 | 10 | 1 | 1 |
| | SA | 3.200.000 | 10 | 10 | 1 | 1 |
| | EC | 4.600.000 | 2.100.000 | 1.400.000 | 200.000 | 1 |
| | CA | 3.000.000 | 220.000 | 62.000 | 8.100 | 1 |
| | AB | 2.500.000 | 240.000 | 220.000 | 150.000 | 19.000 |
| 0,1% 2,3 Heptandiol | PS | 4.900.000 | 230.000 | 730.000 | 3.500.000 | 7.000.000 |
| | SA | 5.900.000 | 27.000 | 490 | 1 | 1 |
| | EC | 3.300.000 | 1.100.000 | 1.500.000 | 1.600.000 | 1.400.000 |
| | CA | 1.800.000 | 110.000 | 20.000 | 100 | 1 |
| | AB | 3.700.000 | 1.600.000 | 970.000 | 110.000 | 120.000 |
| 0,4% 2,3 Heptandiol | PS | 4.900.000 | 2.900 | 1 | 1 | 1 |
| | SA | 5.900.000 | 3.500 | 1 | 1 | 1 |
| | EC | 3.300.000 | 1.200.000 | 100.000 | 75.000 | 380.000 |
| | CA | 1.800.000 | 81 | 1 | 1 | 1 |
| | AB | 3.700.000 | 2.200.000 | 350.000 | 130.000 | 140.000 |
| 0,1% 2,3 Octanediol | PS | 4.900.000 | 280.000 | 3.700.000 | 13.000.000 | 8.100.000 |
| | SA | 5.900.000 | 2.000 | 1 | 1 | 1 |
| | EC | 3.300.000 | 2.600.000 | 3.800.000 | 1.500.000 | 1.300.000 |
| | CA | 1.800.000 | 200.000 | 19.000 | 390 | 1 |
| | AB | 3.700.000 | 2.300.000 | 1.300.000 | 250.000 | 250.000 |
| 0,4% 2,3 Octanediol | PS | 4.900.000 | 21.000 | 1 | 1 | 1 |
| | SA | 5.900.000 | 4.000 | 1 | 1 | 1 |
| | EC | 3.300.000 | 1.100.000 | 1.900.000 | 2.100.000 | 600.000 |
| | CA | 1.800.000 | 44.000 | 17.000 | 510 | 1 |
| | AB | 3.700.000 | 1.100.000 | 1.100.000 | 2.100.000 | 410.000 |
| 0,1% 2,3 Nonanediol | PS | 4.900.000 | 160.000 | 2.000.000 | 10.000.000 | 8.800.000 |
| | SA | 5.900.000 | 31.000 | 200 | 1 | 1 |
| | EC | 3.300.000 | 2.300.000 | 4.200.000 | 1.800.000 | 1.100.000 |
| | CA | 1.800.000 | 110.000 | 29.000 | 870 | 1 |
| | AB | 3.700.000 | 1.800.000 | 2.000.000 | 680.000 | 530.000 |
| 0,4% 2,3 Nonanediol | PS | 4.900.000 | 17.000 | 1 | 1 | 1 |
| | SA | 5.900.000 | 1.800 | 1 | 1 | 1 |
| | EC | 3.300.000 | 3.200.000 | 4.200.000 | 1.300.000 | 1.400.000 |
| | CA | 1.800.000 | 68.000 | 14.000 | 690 | 1 |
| | AB | 3.700.000 | 2.100.000 | 1.800.000 | 1.200.000 | 1.100.000 |

From the above Table 8 it is obvious that 2,3-heptanediol, 2,3-octanediol and 2,3-nonanediol also have an antimicrobial efficacy against different bacteria (represented by *Pseudomonas aeruginosa* (PA), *Staphylococcus aureus* (SA), *Escherichia coli* (EC), yeast (represented by *Candida albicans* (CA) and fungi (represented by *Aspergillus brasiliensis* (AB)) when used in a shampoo formulation.

### Example 2: Synergistic antimicrobial effect against yeast and fungi/mould

### Example 2.2: Synergistic antimicrobial efficacy of a 1,2-octanediol and 2,3-ocatenediol blend and 4-Hydroxyacetophenone

**Table 14:**

| **Substance** | | **Inoculum** | **2 days** | **7 days** | **14 days** | **28 days** |
|---|---|---|---|---|---|---|
| 0.1% Blend (1,2-octanediol and 2,3-ocatenediol, 95:5) | PA | 3.100.000 | 1 | 1 | 1 | 1 |
| | SA | 4.000.000 | 9.500 | 1 | 1 | 1 |
| | EC | 4.600.000 | 320.000 | 3.000 | 54 | 1 |
| | CA | 2.100.000 | 1.300.000 | 1.200.000 | 690.000 | 1.100.000 |
| | AB | 4.300.000 | 3.000.000 | 2.800.000 | 2.300.000 | 3.000.000 |
| 0.1% 4-Hydroxyacetophenone | PA | 3.100.000 | 1 | 1 | 1 | 1 |
| | SA | 4.000.000 | 2.300.000 | 24.000 | 1 | 1 |
| | EC | 4.600.000 | 110.000 | 290 | 27 | 1 |
| | CA | 2.100.000 | 1.400.000 | 170.000 | 130.000 | 55.000 |
| | AB | 4.300.000 | 3.100.000 | 2.300.000 | 2.100.000 | 1.500.000 |
| 0.05% 4-Hydroxyacetophenone and 0.05% Blend (1,2-octanediol and 2,3-ocatenediol, 95:5) | PA | 3.100.000 | 1 | 1 | 1 | 1 |
| | SA | 4.000.000 | 1 | 1 | 1 | 1 |
| | EC | 4.600.000 | 1 | 1 | 1 | 1 |
| | CA | 2.100.000 | 850.000 | 91.000 | 1 | 1 |
| | AB | 4.300.000 | 2.500.000 | 2.500.000 | 980.000 | 120.000 |

### Example 6: Synergistic antimicrobial effect against yeast

### Example 7.4: Synergistic antimicrobial efficacy of 2,3-octanediol and 4-Hydroxyacetophenone

**Table 46:**

| **Substance** | | **Inoculum** | **2 days** | **7 days** | **14 days** | **28 days** |
|---|---|---|---|---|---|---|
| 0.1% 2,3-Octanediol | PA | 3.100.000 | 1 | 1 | 1 | 1 |
| | SA | 4.000.000 | 1.200.000 | 9.500 | 1 | 1 |
| | EC | 4.600.000 | 690.000 | 11.000 | 1.200 | 1 |
| | CA | 2.100.000 | 1.800.000 | 3.200.000 | 2.600.000 | 1.100.000 |
| | AB | 4.300.000 | 2.500.000 | 3.300.000 | 2.900.000 | 2.500.000 |
| 0.1% 4-Hydroxyacetophenone | PA | 3.100.000 | 1 | 1 | 1 | 1 |
| | SA | 4.000.000 | 1.700.000 | 78.000 | 1 | 1 |
| | EC | 4.600.000 | 110.000 | 36 | 1 | 1 |
| | CA | 2.100.000 | 1.200.000 | 310.000 | 190.000 | 100.000 |
| | AB | 4.300.000 | 3.100.000 | 3.000.000 | 1.400.000 | 910.000 |
| 0.05% 4-Hydroxyacetophenone + 0.05% 2,3-Octanediol | PA | 3.100.000 | 1 | 1 | 1 | 1 |
| | SA | 4.000.000 | 960.000 | 14.000 | 1 | 1 |
| | EC | 4.600.000 | 35.000 | 340 | 1 | 1 |
| | CA | 2.100.000 | 2.000.000 | 1.400.000 | 1.300.000 | 190.000 |
| | AB | 4.300.000 | 1.800.000 | 3.100.000 | 2.100.000 | 1.100.000 |

### Example 7.7: Synergistic antimicrobial efficacy of a 1,2-octanediol and 2,3-heptanediol blend and 4-Hydroxyacetophenone (not in accordance with the present invention)

**Table 50:**

| **Substance** | | **Inoculum** | **2 days** | **7 days** | **14 days** | **28 days** |
|---|---|---|---|---|---|---|
| 0.4% Blend (1,2-octanediol and 2,3-heptanediol 95:5) | PA | 3.100.000 | 1 | 1 | 1 | 1 |
| | SA | 4.000.000 | 1 | 1 | 1 | 1 |
| | EC | 4.600.000 | 1 | 1 | 1 | 1 |
| | CA | 2.100.000 | 31.000 | 1 | 1 | 1 |
| | AB | 4.300.000 | 2.100.000 | 1.200.000 | 930.000 | 810.000 |
| 0.1% 4-Hydroxyacetophenone | PA | 3.100.000 | 1 | 1 | 1 | 1 |
| | SA | 4.000.000 | 2.300.000 | 24.000 | 1 | 1 |
| | EC | 4.600.000 | 110.000 | 290 | 27 | 1 |
| | CA | 2.100.000 | 1.400.000 | 170.000 | 130.000 | 55.000 |
| | AB | 4.300.000 | 3.100.000 | 2.300.000 | 2.100.000 | 1.500.000 |
| 0.05% 4-Hydroxyacetophenone + 0.2% Blend (1,2-octanediol and 2,3-heptanediol , 95:5) | PA | 3.100.000 | 1 | 1 | 1 | 1 |
| | SA | 4.000.000 | 55 | 1 | 1 | 1 |
| | EC | 4.600.000 | 1 | 1 | 1 | 1 |
| | CA | 2.100.000 | 790.000 | 89.000 | 1 | 1 |
| | AB | 4.300.000 | 3.400.000 | 1.700.000 | 470.000 | 33.000 |

An overview of the results of the above preservation efficacy test (PET) is shown in the following Table 53:

**Table 53:**

| **Example** | | **Synergistic effect for** | | | **Formulation** | | |
|---|---|---|---|---|---|---|---|
| | | bacteria 78 | yeast | fungi/ | o/w emulsion | shampoo | wet wipe solution |
| | | | | mold | | | |

| **Synergistic antimicrobial efficacy against bacteria, yeast and fungi/mold** | | | | | | | |
|---|---|---|---|---|---|---|---|
| | | | | | | | |
| | | | | | | | |
| | | | | | | | |
| | | | | | | | |
| | | | | | | | |

| **Synergistic antimicrobial efficacy against yeast and fungi/mold** | | | | | | | |
|---|---|---|---|---|---|---|---|
| | | | | | | | |
| 2.2 | Blend (1,2-octanediol + 2,3-octanediol, 95:5) and 4-Hydroxyacetophenone | - | x | x | x | | |
| | | | | | | | |
| | | | | | | | |
| | | | | | | | |
| | | | | | | | |
| | | | | | | | |

| **Synergistic antimicrobial efficacy against bacteria and yeast** | | | | | | | |
|---|---|---|---|---|---|---|---|
| | | | | | | | |
| | | | | | | | |
| | | | | | | | |
| | | | | | | | |
| | | | | | | | |
| | | | | | | | |
| | | | | | | | |

| **Synergistic antimicrobial efficacy against bacteria and fungi/mold** | | | | | | | |
|---|---|---|---|---|---|---|---|
| | | | | | | | |
| | | | | | | | |
| | | | | | | | |
| | | | | | | | |
| | | | | | | | |

| **Synergistic antimicrobial efficacy against bacteria** | | | | | | | |
|---|---|---|---|---|---|---|---|
| | | | | | | | |
| | | | | | | | |
| | | | | | | | |
| | | | | | | | |
| | | | | | | | |
| | | | | | | | |
| | | | | | | | |
| | | | | | | | |
| | | | | | | | |
| | | | | | | | |
| | | | | | | | |
| | | | | | | | |
| | | | | | | | |

| **Synergistic antimicrobial efficacy against yeast** | | | | | | | |
|---|---|---|---|---|---|---|---|
| | | | | | | | |
| | | | | | | | |
| | | | | | | | |

| **Synergistic antimicrobial efficacy against fungi** | | | | | | | |
|---|---|---|---|---|---|---|---|
| | | | | | | | |
| | | | | | | | |
| | | | | | | | |
| 7.4 | 2,3-octanediol and Hydroxyacetophenone | | | x | x | | |
| | | | | | | | |
| | | | | | | | |
| 7.7 | Blend (1,2-Octanediol + 2,3-heptanediol, 95:5) and 4-Hydroxyacetophenone | | | x | x | | |
| | | | | | | | |
| | | | | | | | |

As can be seen from Table 53, a synergistic antimicrobial effect is achieved for 2,3-alkanedioles, in particular 2,3-octanediol. The use of 2,3-octanediol considerably boosts the antimicrobial effect of antimicrobial component 4-hydroxyacetophenone against bacteria, yeast and fungi/mould in food, a cosmetic or pharmaceutical preparation or a homecare product. In particular, the use of 2,3-octanediol results in a synergistic antimicrobial efficacy in combination with the antimicrobial component 4-hydroxyacetophenone.

Also an alkanediol blend including 1,2-octanediol and 2,3-octanediol, when used with the antimicrobial component 4-hydroxyacetophenone considerably enhances the antimicrobial effect of the antimicrobial component.

### Example 8: Solubility of alkanediols in water

For the determination of the maximum water solubility of an alkanediol, water and different concentrations of the respective alkanediol were blended by stirring. The assessment of solubility was carried out at ambient temperature after 24 h storage at 5 °C. The determination of whether the alkanediol has dissolved in water is based entirely on visual observation. An alkanediol compound has dissolved if the mixture is clear and shows no signs of cloudiness or precipitation.

**Table 54:**

| **Tradename/ Chemical name** | **INCI** | **Maximum solubility in water (% by weight)** |
|---|---|---|
| Hydrolite^{®} 5 green | Pentylene Glycol | 50 |
| Hydrolite^{®} 6 | Hexanediol | 50 |
| 1,2-heptanediol | Heptanediol | 2.5 |
| Hydrolite^{®} 8 | Caprylyl Glycol | 0.5 |
| SymDiol^{®} 68 | Hexanediol, Caprylyl Glycol | 1.0 |
| 1,2-nonanediol | Nonanediol | 0.1 |
| SymClariol^{®} | Decylene Glycol | - |
| 1,2-undecanediol | Undecanediol | - |
| 2,3-heptanediol | - | 3.6 |
| 2,3-octanediol | - | 1.5 |
| 2,3-nonanediol | - | 0.3 |
| 2,3-undecanediol | - | - |

### Example 9: Formulation examples

The following formulations according to the present invention were prepared:
▪ Cream O/W
▪ Hand and Body Cream
▪ Daily face cream, SPF 20
▪ Body lotion
▪ Antibacterial body lotion, sprayable
▪ Barrier repair cream
▪ Skin soothing lotion
▪ Baby Nappy Rash Cream w/o
▪ Sunscreen lotion (o/w; broadband protection)
▪ Sun protection soft cream (w/o), SPF 40
▪ Clear Anti Acne Wipe
▪ PEG-free Wet Wipe
▪ Baby Soft Wet Wipes
▪ Intimate Wipes
▪ After-Shave Wipes
▪ Biotic Acne Control Gel
▪ Anti-wrinkle gel
▪ Cleansing Micellar Gel
▪ Clear Beard Gel
▪ Sprayable Anti Cellulite Gel
▪ Aloe Vera Gel
▪ Antidandruff Shampoo
▪ Hair Conditioner, rinse-off
▪ Solid Shampoo

In the following formulation examples the following five perfume oils PFO1, PFO2, PFO3, PFO4 or PFO5 were each used as fragrance.

**Table 55: Composition of perfume oil 1; PO1 (amounts in ‰ by weight)**

| **Ingredients** | **Amount** |
|---|---|
| ALDEHYDE C14 SO-CALLED | 2 |
| ALLYL AMYL GLYCOLATE 10% DPG | 5 |
| ANISIC ALDEHYDE PURE | 5 |
| APPLE OLIFFAC TYPE | 10 |
| Benzylacetate | 50 |
| BERGAMOT IDENTOIL^{®} COLOURLESS | 15 |
| CANTHOXAL | 5 |
| CETALOX 10% IPM | 3 |
| CITRONELLOL 950 | 40 |
| DAMASCENONE TOTAL 1% DPG | 5 |
| DAMASCONE ALPHA 10% DPG | 5 |
| DAMASCONE DELTA 10% DPG | 2 |
| DIMETHYL BENZYL CARBINYL BUTYRATE | 2 |
| DIPROPYLENE GLYCOL | 178 |
| EBANOL | 2 |
| ETHYL DECADIENOATE TRANS CIS-2.4 10% IPM | 2 |
| FLOROSA | 5 |
| FRAMBINON^{®} 10% DPG | 7 |
| GALAXOLIDE 50% IN IPM | 100 |
| GALBEX TYPE BASE | 1 |
| GERANYL ACETATE PURE | 2 |
| HEDIONE | 30 |
| HELIOTROPIN | 10 |
| HEXENYL ACETATE CIS-3 10% DPG | 1 |
| HEXENYL SALICYLATE CIS-3 | 5 |
| HEXYL CINNAMIC ALDEHYDE ALPHA | 70 |
| HEXYL SALICYLATE | 50 |
| HYDROXY CITRONELLAL | 10 |
| ISO E SUPER | 15 |
| ISORALDEINE 70 | 20 |
| LEAFOVERT^{®} | 1 |
| LILIAL | 60 |
| LINALOOL | 60 |
| LINALYL ACETATE | 20 |
| LYRAL | 7 |
| MANZANATE | 2 |
| PHENOXANOL | 7 |
| PHENYLETHYL ALCOHOL | 120 |
| SANDAL MYSORE CORE | 2 |
| SANDRANOL^{®} | 7 |
| STYRALYL ACETATE | 3 |
| TAGETES RCO 10% TEC | 2 |
| TERPINEOL PURE | 20 |
| TETRAHYDROGERANIOL 10% DPG | 5 |
| TONALIDE | 7 |
| VERTOCITRAL 10% DPG | 5 |
| VERTOFIX | 15 |
| **Total:** | **1000** |

**Table 56: Composition of perfume oil 2; PO2 (amounts in ‰ by weight)**

| **Ingredients** | **Amount** |
|---|---|
| Acetophenone. 10% in DPG | 10 |
| n-Undecanal | 5 |
| Aldehyde C14 (peach aldehyde) | 15 |
| Allylamyl glycolate, 10% in DPG | 20 |
| Amyl salicylate | 25 |
| Benzyl acetate | 60 |
| Citronellol | 80 |
| d-Limonene | 50 |
| Decenol trans-9 | 15 |
| Dihydromyrcenol | 50 |
| Dimethylbenzylcarbinyl acetate | 30 |
| Diphenyloxide | 5 |
| Eucalyptol | 10 |
| Geraniol | 40 |
| Nerol | 20 |
| Geranium oil | 15 |
| Hexenol cis-3. 10% in DPG | 5 |
| Hexenyl salicylate cis-3 | 20 |
| Indole. 10% in DPG | 10 |
| Alpha-ionone | 15 |
| Beta-ionone | 5 |
| Lilial^{®} (2-methyl-3-(4-tert-butyl-phenyl)propanal) | 60 |
| Linalool | 40 |
| Methylphenyl acetate | 10 |
| Phenylethyl alcohol | 275 |
| Styrolyl acetate | 20 |
| Terpineol | 30 |
| Tetrahydrolinalool | 50 |
| Cinnamyl alcohol | 10 |
| **Total:** | **1000** |

**Table 57: Composition of perfume oil 3; PO3 (amounts in ‰ by weight)**

| **Ingredients** | **Amount** |
|---|---|
| Benzyl acetate | 60 |
| Citronellyl acetate | 60 |
| Cyclamenaldehyde (2-methyl-3-(4-isopropylphenyl)propanal | 20 |
| Dipropylene glycol (DPG) | 60 |
| Ethyllinalool | 40 |
| Florol (2-isobutyl-4-methyltetrahydro-2H-pyran-4-ol) | 30 |
| Globanone^{®} [(E/Z)-8-cyclohexadecen-1-one] | 180 |
| Hedione^{®} (methyldihydrojasmonate) | 140 |
| Hexenyl salicylate. cis-3 | 10 |
| Vertocitral (2.4-dimethyl-3-cyclohexenecarboxaldehyde) | 5 |
| Hydratropaldehyde. 10% in DPG | 5 |
| Isodamascone (1-(2.4.4-trimethyl-2-cyclohexen-1-yl)-2-buten-1-one. 10% in DPG | 5 |
| Isomuscone (cyclohexadecanone) | 40 |
| Jacinthaflor (2-methyl-4-phenyl-1.3-dioxolane) | 10 |
| Cis-jasmone. 10% in DPG | 20 |
| Linalool | 50 |
| Linalyl acetate | 30 |
| Methyl benzoate. 10% in DPG | 25 |
| para-Methyl cresol. 10% in DPG | 10 |
| Nerol | 20 |
| Phenylpropylaldehyde | 5 |
| 2-Phenylethyl alcohol | 82 |
| Tetrahydrogeraniol | 13 |
| 2.2-Dimethyl-3-cyclohexyl-1-propanol | 80 |
| **Total:** | **1000** |

**Table 58: Composition of perfume oil 4; PO4 (amounts in ‰ by weight)**

| **Ingredients** | **Amount** |
|---|---|
| AMBRETTOLIDE (MACRO) | 10 |
| AMBROXIDE 10% in IPM | 10 |
| BENZYL ACETATE | 20 |
| BENZYL SALICYLATE | 15 |
| BERGAMOT OIL. bergapten-free | 60 |
| CALONE^{®} 1951 10% in DPG | 15 |
| COUMARIN | 5 |
| CYCLOGALBANATE^{®} 10% in DPG | 10 |
| ALPHA -DAMASCONE 1% in DPG | 20 |
| DIHYDROMYRCENOL | 10 |
| ETHYL LINALOOL | 75 |
| ETHYL LINALYLACETATE | 50 |
| ETHYL MALTOL 1% in DEP | 10 |
| ETHYLENE BRASSYLATE (MACRO) | 80 |
| FLOROSA | 40 |
| GERANYLACETATE | 10 |
| HEDIONE^{®} HC/30 | 35 |
| HEDIONE^{®} | 210 |
| HELIONAL^{®} | 15 |
| HELVETOLIDE^{®} (ALICYC) | 30 |
| HEXENYLSALICYLATE CIS-3 | 20 |
| ISO E SUPER^{®} | 40 |
| LEAFOVERT^{®} 10% in DEP | 10 |
| LILIAL^{®} | 80 |
| LYRAL^{®} | 20 |
| MANDARIN OIL | 10 |
| STYRALYL ACETATE | 5 |
| SYMROSE^{®} | 15 |
| VANILLIN 10% in DEP | 20 |
| DIPROPYLENE GLYCOL (DPG) | 50 |
| **TOTAL:** | **1000** |

**Table 59: Composition of perfume oil 5; PO5 (amounts in %o by weight)**

| **Ingredients** | **Amount** |
|---|---|
| AMAROCITE^{®} | 10 |
| AMBROCENIDE^{®} 10% in DPG | 5 |
| AMBROXIDE | 15 |
| AURELIONE^{®} (7/8-Cyclohexadecenone) (MACRO) | 70 |
| BERGAMOT OIL. bergapten-free | 90 |
| CALONE^{®} 1951 10% in DPG | 20 |
| CARAWAY OIL | 10 |
| CITRAL | 20 |
| COUMARIN | 10 |
| ALPHA-DAMASCONE 1% in DPG | 15 |
| DIHYDROMYRCENOL | 70 |
| ESTRAGON OIL | 10 |
| ETHYL LINALOOL | 100 |
| ETHYL LINALYLACETATE | 90 |
| EUGENOL | 10 |
| EVERNYL^{®} | 5 |
| FRUCTATE^{®} | 5 |
| GERANIUM OIL | 5 |
| HEDIONE^{®} HC/30 | 100 |
| HELIONAL^{®} | 10 |
| INDOLE 10% in DPG | 5 |
| ISO E SUPER^{®} | 100 |
| KEPHALIS^{®} | 5 |
| LAVENDER OIL | 40 |
| CITRUS OIL | 80 |
| LILIAL^{®} | 30 |
| MANDARIN OIL | 20 |
| MUSCENONE (MACRO) | 5 |
| SANDRANOL^{®} | 10 |
| VANILLIN 10% in DPG | 5 |
| DIPROPYLENE GLYCOL | 30 |
| **TOTAL:** | **1000** |

Cosmetic formulations (compositions) - amounts are indicated as % by weight for all formulations.

**Table 60: Cream o/w**

| **Ingredients** | **INCI** | **Amount** |
|---|---|---|
| Dracorin^{®} CE | Glyceryl Stearate Citrate | 1.0 |
| Lanette^{®} O | Cetearyl Alcohol | 2.0 |
| Cutina^{®} GMS-V | Glyceryl Stearate | 1.0 |
| Tegosoft^{®} MM | Myristyl Myristate | 1.0 |
| Hydrolite-6 | Hexanediol | 1.0 |
| Xiameter^{®} PMX-0246, Cyclosiloxane | Cyclohexasiloxane (and) Cyclopentasiloxane | 0.5 |
| Dragoxat^{®} 89 | Ethylhexyl Isononanoate | 2.0 |
| PCL-Liquid 100 | Cetearyl Ethylhexanoate | 4.0 |
| Neutral Oil | Caprylic/Capric Triglyceride | 4.0 |
| Carbopol^{®} Ultrez 21 | Acrylates/C10-30 Alkyl Acrylate Crosspolymer | 0.2 |
| Keltrol^{®} CG-T | Xanthan Gum | 0.1 |
| Water | Water (Aqua) | ad 100 |
| Glycerol 99,5 P. | Glycerol | 3.0 |
| 1,2-Propylene Glycol 99 P GC | Propylene Glycol | 2.0 |
| Sodium Benzoate | Sodium Benzoate | 0.2 |
| Sodium Hydroxide 10% aqueous solution | Sodium Hydroxide | 0.5 |
| Perfume oil PO1, PO2, PO3, PO4 or PO5 | Perfume | 0.3 |
| SymSave^{®} H | 4-Hydroxyacetophenone | 0.5 |
| Propanediol Caprylate | Propanediol Caprylate | 0.1 |
| Undecylenoyl glycine | Undecylenoyl glycine | 0.1 |
| 1,2-heptanediol | 1,2-heptanediol | 0.5 |
| 2,3-heptanediol | 2,3-heptanediol | 0.05 |
| 1,2-octanediol | 1,2-octanediol | 0.2 |
| 2,3-octanediol | 2,3-octanediol | 0.01 |

**Table 61: Hand and body cream (not in accordance with the present invention)**

| **Ingredients** | **INCI** | **Amount** |
|---|---|---|
| Dracorin^{®} GOC | Glyceryl Oleate Citrate, Caprylic/Capric Triglyceride | 2.0 |
| PCL-Solid | Stearyl Heptanoate, Stearyl Caprylate | 2.5 |
| Lanette^{®} O | Cetearyl Alcohol | 1.5 |
| Cutina^{®} GMS-V | Glyceryl Stearate | 1.0 |
| Dragoxat^{®} 89 | Ethylhexyl Isononanoate | 3.0 |
| PCL-Liquid^{®} 100 | Cetearyl Ethylhexanoate | 7.0 |
| Isodragol^{®} | Triisononanoin | 4.0 |
| Xiameter^{®} PMX-0345 Cyclosiloxane | Cyclopentasiloxane (and) Cyclohexasiloxane | 0.5 |
| Water | Water (Aqua) | ad 100 |
| Carbopol^{®} Ultrez 21 | Acrylates/C10-30 Alkyl Acrylate Crosspolymer | 0.2 |
| Keltrol^{®} CG-RD | Xanthan Gum | 0.1 |
| Glycerol 85 P. | Glycerol | 3.0 |
| DragoBetaGlucan | Water (Aqua), Butylene Glycol, Glycerol, Avena Sativa (Oat) Kernel Extract | 1.5 |
| Potassium Sorbate | Potassium Sorbate | 0.1 |
| Sodium Hydroxide 10% solution | Sodium Hydroxide | 0.5 |
| Perfume oil PO1, PO2, PO3, PO4 or PO5 | Perfume | 0.2 |
| Euxyl^{®} K300 | Methyl-, Butyl-, Ethyl-, Propyl, Isobutylparaben, Phenoxyethanol | 0.3 |
| Glyceryl Caprate | Glyceryl Caprate | 0.2 |
| Sorbitan Caprylate | Sorbitan Caprylate | 0.1 |
| 1,2-heptanediol | 1,2-heptanediol | 0.3 |
| 2,3-heptanediol | 2,3-heptanediol | 0.05 |
| 1,2-octanediol | 1,2-octanediol | 0.3 |
| 2,3-octanediol | 2,3-octanediol | 0.01 |

**Table 62: Daily face cream SPF 20 (not in accordance with the present invention)**

| **Ingredients** | **INCI** | **Amount** |
|---|---|---|
| SymOcide^{®} PH | Phenoxyethanol, Hydroxyacetophenone, Caprylyl Glycol, Water (Aqua) | 1.0 |
| Ascorbyl Palmitate | Ascorbyl Palmitate | 0.1 |
| Biotive L-Arginine | Arginine | 0.2 |
| Buriti oil | Mauritia Flexuosa Fruit Oil | 1.0 |
| Cocoa butter | Theobroma Cacao (Cocoa) Seed Butter | 2.0 |
| Dimethicone | Dimethicone | 0.5 |
| Disodium EDTA | Disodium EDTA | 0.1 |
| Dragosantol 100 | Bisabolol | 0.1 |
| Dragoxat^{®} 89 | Ethylhexyl Isononanoate | 5.0 |
| Emulsiphos | Potassium Cetyl Phosphate, Hydrogenated Palm Glycerides | 2.0 |
| Glycerin | Glycerin | 3.0 |
| Isoadipate | Diisopropyl Adipate | 5.0 |
| Jojoba Wax Flakes | Hydrogenated Jojoba Oil | 1.0 |
| Keltrol CG-T | Xanthan Gum | 0.1 |
| Lanette O | Cetearyl Alcohol | 5.0 |
| Lanette 16 | Cetyl Alcohol | 1.0 |
| Lanette 22 | Behenyl Alcohol | 1.0 |
| Neo Heliopan^{®} 357 | Butyl Methoxydibenzoylmethane | 3.0 |
| Neo Heliopan^{®} HMS | Homosalate | 10.0 |
| Neo Heliopan^{®} Hydro used as a 25% aq, Solution neutralized by arginine | Phenylbenzimidazole Sulfonic Acid | 8.0 |
| Neo Heliopan^{®} OS | Ethylhexyl Salicylate | 5.0 |
| Orgasol Caresse | Polyamide-5 | 1.0 |
| Perfume oil PO1, PO2, PO3, PO4 or PO5 | Perfume | 0.1 |
| Shea butter | Butyrospermum Parkii (Shea) Butter | 3.0 |
| Simugel EG | Sodium Acrylate / Sodium Acryloyldimethyl Taurate Copolymer, Isohexadecane, Polysorbate 80 | 1.0 |
| SymFinity^{®} 1298 | Echinacea Purpurea Extract | 0.1 |
| SymMatrix^{®} | Maltodextrin, Rubus Fructicosus (Blackberry) Leaf Extract | 0.1 |
| SymSitive^{®} 1609 | Pentylene Glycol, 4-t-Butylcyclohexanol | 1.0 |
| Tegosoft TN | C12-15 Alkyl Benzoate | 4.0 |
| Hydrolite-8 | Caprylyl Glycol | 0.5 |
| Levulinic Acid | Levulinic Acid | 0.2 |
| TEA-salicylate | TEA-salicylate | 0.1 |
| Water | Water (aqua) | ad 100 |
| 1,2-heptanediol | 1,2-heptanediol | 0.3 |
| 2,3-heptanediol | 2,3-heptanediol | 0.05 |
| 1,2-octanediol | 1,2-octanediol | 0.3 |
| 2,3-octanediol | 2,3-octanediol | 0.05 |

**Table 63: Body lotion (not in accordance with the present invention)**

| **Ingredients** | **Amount** |
|---|---|
| Cetearyl Alcohol | 2.0 |
| Ethylhexyl Isononanoate | 5.0 |
| Cetearyl Ethylhexanoate, Isopropyl Myristate | 3.0 |
| Glyceryl Oleate Citrate, Caprylic/Capric Triglyceride | 4.0 |
| Water (Aqua) | ad 100 |
| Carbomer | 0.3 |
| DMDM Hydantoin | 0.1 |
| Anisic Acid | 0.3 |
| Propylene Glycol | 5.0 |
| Sodium Hydroxide 30% aqueous solution | 0.3 |
| Perfume oil PO1, PO2, PO3, PO4 or PO5 | 0.3 |
| Triethylene Glycol, Imidazolidinyl Urea, Methylparaben, Propylparaben, Dehydroacetic Acid | 0.2 |
| Tetrasodium Glutamate Diacetate | 0.1 |
| 1,2-heptanediol | 1.0 |
| 2,3-heptanediol | 0.05 |
| 1,2-octanediol | 0.1 |
| 2,3-octanediol | 0.001 |

**Table 64: Antibacterial body lotion, sprayable**

| **Ingredients** | **INCI** | **Amount** |
|---|---|---|
| Dow Corning 345 Fluid | Cyclomethicone | 0.5 |
| Dracorin^{®} GOC | Glyceryl Oleate Citrate, Caprylic/Capric Triglyceride | 2.0 |
| Drago-Calm | Water, Glycerin, Avena Sativa (Oat) Kernel Extract | 1.0 |
| Dragosantol^{®} 100* | Bisabolol | 0.1 |
| Perfume oil PO1, PO2, PO3, PO4 or PO5 | Perfume | 0.3 |
| Hydrolite^{®}-5 | Pentylene Glycol | 5.0 |
| Neutral Oil | Caprylic/Capric Triglyceride | 4.0 |
| Paraffin Oil | Mineral Oil | 4.0 |
| PCL Liquid 100 | Cetearyl Ethylhexoate | 7.0 |
| Pemulen TR-2 | Acrylates/C10-30 Alkyl Acrylate Crosspolymer | 0.2 |
| Sodium Hydroxide (10% aqueous sol.) | Sodium Hydroxide | 0.4 |
| SymDeo^{®} MPP | Dimethyl Phenylbutanol | 0.5 |
| SymRelief1 100 | Bisabolol, Zingiber Officinale (Ginger) Root Extract | 0.1 |
| Caprylhydroxamic Acid | Caprylhydroxamic Acid | 0.2 |
| Dehydracetic Acid | Dehydracetic Acid | 0.1 |
| SymSave^{@} H | 4-Hydroxyacetophenone | 0.2 |
| Water demineralized | Water (aqua) | ad 100 |
| 1,2-heptanediol | 1,2-heptanediol | 0.8 |
| 2,3-heptanediol | 2,3-heptanediol | 0.03 |
| 1,2-octanediol | 1,2-octanediol | 0.5 |
| 2,3-octanediol | 2,3-octanediol | 0.02 |

**Table 65: Barrier repair cream (not in accordance with the present invention)**

| **Ingredients** | **INCI** | **Amount** |
|---|---|---|
| Abil 350 | Dimethicone | 0.5 |
| Allantoin | Allantoin | 0.25 |
| Ceramide BIO* | Cetylhydroxyproline Palmitamide | 0.5 |
| Dracorin^{®} CE | Glyceryl Stearate Citrate | 1.5 |
| Dragoxat^{®} 89 | Ethylhexylisononanoate | 2.0 |
| Emulsiphos^{®} | Potassium Cetyl Phosphate, Hydrogenated Palm Glycerides | 2.0 |
| Extrapone^{®} Rosemary GW | Glycerin, Water (Aqua), Rosmarinus officinalis (Rosemary) Leaf Extract | 0.5 |
| Perfume oil PO1, PO2, PO3, PO4 or PO5 | Perfume | 0.1 |
| Glycerol 85 % | Glycerin | 3.0 |
| Glyceryl Stearate | Glyceryl Stearate | 2.0 |
| Hydroviton^{®} 24 | Water, Glycerin, Sodium Lactate, TEA Lactate, Serine, Lactic Acid, Urea, Sorbitol, Sodium Chloride, Lauryl Diethylenedi-aminoglycine, Lauryl Aminopropyl-glycine, Allantoin | 1.0 |
| Isodragol^{®} | Triisononanoin | 3.0 |
| Lanette O | Cetearyl Alcohol | 2.0 |
| NaOH 10% sol, | Sodium Hydroxide | 0.3 |
| Neutral Oil | Caprylic/Capric Triglyceride | 10.0 |
| SymCalmin^{®} | Pentylene Glycol, Butylene Glycol, Hydroxyphenyl Propamidobenzoic Acid | 1.0 |
| SymRepair^{®} 100 | Hexyldecanol, Bisabolol, Cetylhydroxyproline Palmitamide, Stearic Acid, Brassica Campestris (Rapeseed) Sterols | 2.0 |
| SymDiol^{®} 68 T | Caprylyl glycol, 1,2-Hexanediol, Tropolone | 1.0 |
| Tegosoft PC 31 | Polyglyceryl 3- Caprate | 0.3 |
| Tocopherol Acetate | Tocopheryl Acetate | 0.3 |
| Water (demineralized) | Water (Aqua) | ad 100 |
| Trisodium Ethylenediamine Disuccinate | Trisodium Ethylenediamine Disuccinate | 0.1 |
| Phenoxyethanol | Phenoxyethanol | 0.5 |
| 1,2-heptanediol | 1,2-heptanediol | 0.7 |
| 2,3-heptanediol | 2,3-heptanediol | 0.05 |
| 1,2-octanediol | 1,2-octanediol | 0.1 |
| 2,3-octanediol | 2,3-octanediol | 0.01 |

**Table 66: Skin soothing lotion (not in accordance with the present invention)**

| **Ingredients** | **INCI** | **Amount** |
|---|---|---|
| Allantoin | Allantoin | 0.2 |
| Carbopol Ultrez-10 | Carbomer | 0.1 |
| Ceramide BIO* | Cetylhydroxyproline Palmitamide | 0.1 |
| Citric Acid 10% sol, | Citric Acid | 0.4 |
| Emulsiphos^{®} | Potassium Cetyl Phosphate, Hydrogenated Palm Glycerides | 2.0 |
| Extrapone^{®} Green Tea GW | Glycerin, Water (Aqua), Camellia Sinensis Leaf Extract | 0.2 |
| Extrapone^{®} Rosemary GW | Glycerin, Water (Aqua), Rosmarinus officinalis (Rosemary) Leaf Extract | 0.3 |
| Perfume oil PO1, PO2, PO3, PO4 or PO5 | Perfume | 0.3 |
| Glycerol 85 % | Glycerin | 2.0 |
| Glyceryl Stearate | Glyceryl Stearate | 2.0 |
| Isodragol^{®} | Triisononanoin | 2.0 |
| Keltrol RD | Xanthan Gum | 0.1 |
| Lanette O | Cetearyl Alcohol | 3.0 |
| Neo PCL wssl, N | Trideceth-9, PEG-5 Ethylhexanoate, Water | 1.0 |
| PCL Liquid 100 | Cetearyl Ethylhexanoate | 5.0 |
| PCL Solid | Stearyl Heptanoate, Stearyl Caprylate | 2.0 |
| Phenoxyethanol | Phenoxyethanol | 0.8 |
| Propylene Glycol | Propylene Glycol | 5.0 |
| Sodium Hydroxide 10% aqueous solution | Sodium Hydroxide | 0.3 |
| Sodium Methylparaben | Sodium Methylparaben | 0.2 |
| SymCalmin^{®} | Pentylene Glycol, Butylene Glycol, Hydroxyphenyl Propamidobenzoic Acid | 2.0 |
| SymMatrix^{®} | Maltodextrin, Rubus Fruticosus (Blackberry) Leaf Extract | 0.1 |
| SymOcide^{®} C | o-cymen-5-ol | 0.1 |
| Anisic Acid | Anisic Acid | 0.4 |
| SymSitive^{®}1609 | Pentylene Glycol, 4-t-Butylcyclohexanol | 1.5 |
| Water (demineralized) | Water (Aqua) | ad 100 |
| 1,2-heptanediol | 1,2-heptanediol | 0.3 |
| 2,3-heptanediol | 2,3-heptanediol | 0.05 |
| 1,2-octanediol | 1,2-octanediol | 0.3 |
| 2,3-octanediol | 2,3-octanediol | 0.01 |

**Table 67: Baby Nappy Rash Cream w/o (not in accordance with the present invention)**

| **Ingredients** | **Amount** |
|---|---|
| **SymOcide PH** | 1.0 |
| Phenoxyethanol, Hydroxyacetophenone, Caprylyl Glycol, Water (Aqua) | |
| **Cupuagu butter** | 1.0 |
| Theobroma Grandiflorum Seed Butter | |
| **Cutina HR Powder** | 1.5 |
| Hydrogenated Castor Oil | |
| **Dehymuls PGPH** | 5.0 |
| Polyglyceryl-2 Dipolyhydroxystearate | |
| **Glycerin** | 5.0 |
| Glycerin | |
| **Jojoba oil** | 5.0 |
| Simmondsia Chinensis (Jojoba) Seed Oil | |
| **Magnesium Sulfate Hepta Hydrate** | 0.5 |
| Magnesium Sulfate | |
| **Monomuls 90-O18** | 1.0 |
| Glyceryl Oleate | |
| **Neutral oil** | 8.0 |
| Caprylic/capric triglyceride | |
| **PCL Liquid 100** | 5.0 |
| Cetearyl Ethylhexanoate | |
| **SymCalmin** | 1.0 |
| Butylene Glycol, Pentylene Glycol, Hydroxyphenyl Propamidobenzoic Acid | |
| **Benzyl Alcohol** | 0.3 |
| **Tetrasodium EDTA** | 0.1 |
| Tetrasodium EDTA | |
| **Titan dioxide** | 4.0 |
| Titan dioxide | |
| **Water** | ad 100 |
| Aqua | |
| **Wheat germ oil** | 2.0 |
| Triticum Vulgare (Wheat) Germ Oil | |
| **Zinc oxide** | 10.0 |
| Zinc oxide | |
| **Perfume oil PO1, PO2, PO3, PO4 or PO5** | 0.1 |
| Perfume | |
| **Itaconic Acid** | 0.3 |
| **1,2-heptanediol / 2,3-heptanediol (95:5)** | 0.5 |

**Table 68: Sunscreen lotion (o/w; broadband protection) (not in accordance with the present invention)**

| **Ingredients** | **INCI** | **Amount** |
|---|---|---|
| 1,2-heptanediol | 1,2-heptanediol | 0.6 |
| 2,3-heptanediol | 2,3-heptanediol | 0.3 |
| 1,2-octanediol | 1,2-octanediol | 0.2 |
| 2,3-octanediol | 2,3-octanediol | 0.01 |
| Benzyl Salicylate | Benzyl Salicylate | 0.5 |
| Carbopol Ultrez-10 | Carbomer | 0.2 |
| Dow Corning 246 Fluid | Cyclohexasiloxane and Cyclopentasiloxane | 2.0 |
| Dragosantol^{®} 100* | Bisabolol | 0.3 |
| EDETA BD | Disodium EDTA | 0.1 |
| Emulsiphos^{®} | Potassium Cetyl Phosphate, Hydrogenated Palm Glycerides | 1.5 |
| Perfume oil PO1, PO2, PO3, PO4 or PO5 | Perfume | 0.4 |
| Frescolat^{®} MGA | Menthone Glycerol Acetal | 0.3 |
| Glycerol 85 % | Glycerin | 4.7 |
| Keltrol RD | Xanthan Gum | 0.2 |
| Lanette O | Cetearyl Alcohol | 1.0 |
| Neo Heliopan^{®} 357 | Butyl Methoxy-dibenzoyl-methane | 1.0 |
| Neo Heliopan^{®} AP (10 % as sodium salt) | Disodium Phenyl Dibenzimidazole Tetrasulfonate | 10.0 |
| Neo Heliopan^{®} AV | Ethylhexyl Methoxy-cinnamate | 3.0 |
| Neo Heliopan^{®} Hydro (15 % as sodium salt) | Phenylbenz-imidazole Sulfonic Acid | 6.7 |
| Neo Heliopan^{®} MBC | 4-Methylbenzyl-idene Camphor | 1.5 |
| Neo Heliopan^{®} OS | Ethylhexyl Salicylate | 5.0 |
| Neutral Oil | Caprylic/Capric Triglyceride | 2.0 |
| SymMatrix^{®} | Maltodextrin, Rubus Fruticosus (Blackberry) Leaf Extract | 0.3 |
| Methylisothiazolinone | Methylisothiazolinone | 0.1 |
| SymOcide^{®} BHO | Hydroxyacetophenone, Benzyl alcohol, Caprylyl glycol, Aqua | 0.5 |
| Tegosoft TN | C12-15 Alkyl Benzoate | 5.0 |
| Tocopherol Acetate | Tocopheryl Acetate | 0.5 |
| Triethanolamine, 99% | Triethanolamine | 0.5 |
| Water (demineralized) | Water (Aqua) | ad 100 |

**Table 69: Sun protection soft cream (w/o), SPF 40 (not in accordance with the present invention)**

| **Ingredients** | **INCI** | **Amount** |
|---|---|---|
| Dehymuls PGPH | Polyglyceryl-2 dipolyhydroxystearate | 5.0 |
| Copherol 1250 | Tocopheryl acetate | 0.5 |
| Permulqin 3220 | Ozocerite | 0.5 |
| Zinc stearate | Zinc stearate | 0.5 |
| Teqosoft TN | C12-15 Alkyl benzoate | 10.0 |
| Neo Heliopan^{®} E1000 | Isoamyl-p-methoxycinnamate | 2.0 |
| Neo Heliopan^{®} 303 | Octocrylene | 5.0 |
| Neo Heliopan^{®} MBC | 4-Methylbenzylidene camphor | 3.0 |
| Zinc oxide, neutral | Zinc oxide | 5.0 |
| Water, distilled | Water (aqua) | ad 100 |
| EDETA BD | Disodium EDTA | 0.1 |
| Glycerol | Glycerol | 4.0 |
| Magnesium sulfate | Magnesium sulfate | 0.5 |
| Perfume oil PO1, PO2, PO3, PO4 or PO5 | Perfume | 0.3 |
| Symdiol^{®} 68 | Hexanediol, Caprylylglycol | 0.5 |
| 1,2-heptanediol | 1,2-heptanediol | 0.2 |
| 2,3-heptanediol | 2,3-heptanediol | 0.01 |
| 1,2-octanediol | 1,2-octanediol | 0.2 |
| 2,3-octanediol | 2,3-octanediol | 0.01 |

**Table 70: Clear Anti Acne Wipe (not in accordance with the present invention)**

| **Ingredients** | **INCI** | **Amount** |
|---|---|---|
| Farnesol | Farnesol | 0.2 |
| Sensiva SC 50 | Ethylhexylglycerin | 0.3 |
| Phenethylalcohol | Phenethylalcohol | 0.3 |
| Isopropanol | Isopropanol | 45.0 |
| Water, distilled | Water (Aqua) | ad 100 |
| Camomile CL | Chamomilla Recutita (Matricaria) Flower Water, Butylene Glycol, Pentylene Glycol | 1.0 |
| Witch Hazel-Distillate | Hamamelis Virginiana (Witch Hazel) Water, Water (Aqua), Alcohol, Hamamelis Virginiana (Witch Hazel) Extract | 1.0 |
| Citric Acid 10 % aqueous solution | Citric Acid | 0.03 |
| Perfume oil PO1, PO2, PO3, PO4 or PO5 | Perfume | 0.1 |
| Hydroxyethoxyphenyl Butanone | Hydroxyethoxyphenyl Butanone | 0.4 |
| 1,2-heptanediol | 1,2-heptanediol | 1.0 |
| 2,3-heptanediol | 2,3-heptanediol | 0.05 |
| 1,2-octanediol | 1,2-octanediol | 0.3 |
| 2,3-octanediol | 2,3-octanediol | 0.01 |

**Table 71: PEG-free Wet Wipe (not in accordance with the present invention)**

| **Ingredients** | **INCI** | **Amount** |
|---|---|---|
| Tegosoft^{®} PC 41 | Polyglyceryl-4 Caprate | 2.0 |
| Phenethylalcohol | Phenethylalcohol | 0.5 |
| Tegosoft^{®} M | Isopropyl Myristate | 0.2 |
| Perfume oil PO1, PO2, PO3, PO4 or PO5 | Perfume | 0.1 |
| Propylengycol | Propylene Glycol | 3.0 |
| Water, distilled | Water (Aqua) | ad 100 |
| Cetrimonium Bromide, 40% | Cetrimonium Bromide | 0.25 |
| Chlorphenesine | Chlorphenesine | 0.1 |
| Hydroxyethoxyphenyl Butanol | Hydroxyethoxyphenyl Butanol | 0.6 |
| 1,2-heptanediol | 1,2-heptanediol | 1.5 |
| 2,3-heptanediol | 2,3-heptanediol | 0.05 |
| 1,2-octanediol | 1,2-octanediol | 0.4 |
| 2,3-octanediol | 2,3-octanediol | 0.01 |

**Table 72: Baby Soft Wet Wipes (not in accordance with the present invention)**

| **Ingredients** | **INCI** | **Amount** |
|---|---|---|
| Mineral Oil | Paraffinum Liquidum | 4.0 |
| Isopropylpalmitat | Isopropyl Palmitate | 3.0 |
| Eumulqin B2 | Ceteareth-20 | 2.5 |
| Lanette O | Cetearyl Alcohol | 2.5 |
| Draqosantol | Bisabolol | 0.1 |
| Olive Oil | Olea Europaea (Olive) Fruit Oil | 0.5 |
| Isopropylmyristat | Isopropyl Myristate | 0.5 |
| Dow Corning 345 Fluid | Cyclomethicone | 0.2 |
| Super Hartolan | Lanolin Alcohol | 0.2 |
| Taqat L2 | PEG-20 Glyceryl Laurate | 2.5 |
| Water, distilled | Water (Aqua) | ad 100 |
| Glycerin 99,5% | Glycerin | 3.0 |
| Citric Acid Sol, aqueous solution 10% | Citric Acid | 0.1 |
| Perfume oil PO1, PO2, PO3, PO4 or PO5 | Perfume | 0.1 |
| Sensiva SC 50 | Ethylhexylglycerin | 0.3 |
| 1,2-heptanediol | 1,2-heptanediol | 1.0 |
| 2,3-heptanediol | 2,3-heptanediol | 0.02 |
| 1,2-octanediol | 1,2-octanediol | 0.3 |
| 2,3-octanediol | 2,3-octanediol | 0.01 |

**Table 73: Intimate Wipes (not in accordance with the present invention)**

| **Ingredients** | **INCI** | **Amount** |
|---|---|---|
| Dracorin^{®} GOC | Glyceryl Oleate Citrate, Caprylic/Capric Triglyceride | 2.2 |
| PCL-Liquid^{®} 100 | Cetearyl Ethylhexanoate | 3.0 |
| SymCalmin^{®} | Pentylene Glycol, Butylene Glycol, Hydroxyphenyl Propamidobenzoic Acid | 1.0 |
| Frescolat^{®} X-Cool | Menthyl Ethylamido Oxalate | 0.5 |
| Perfume oil PO1, PO2, PO3, PO4 or PO5 | Perfume | 0.2 |
| Water, distilled | Water (Aqua) | ad 100 |
| PemulenTM TR-2 Polymeric Emulsifier | Acrylates/C10-30 Alkyl Acrylate Crosspolymer | 0.2 |
| Sensiva SC 50 | Ethylhexylglycerin | 0.2 |
| Symdiol^{®} 68T | Hexanediol, Caprylyl Glycol, Tropolone | 0.5 |
| Potassium Sorbate | Potassium Sorbate | 0.1 |
| Extrapone^{®} Camomille GW | Glycerin,Aqua, Chamomilla, Recutita Flower Extract | 0.5 |
| Sodium Hydroxide 10% solution | Aqua, Sodium Hydroxide | 0.45 |
| PCA ethyl cocoyl arginate | PCA ethyl cocoyl arginate | 0.1 |
| 1,2-heptanediol | 1,2-heptanediol | 0.5 |
| 2,3-heptanediol | 2,3-heptanediol | 0.5 |
| 1,2-octanediol | 1,2-octanediol | 0.2 |
| 2,3-octanediol | 2,3-octanediol | 0.01 |

**Table 74: After-Shave Wipes (not in accordance with the present invention)**

| **Ingredients** | **INCI** | **Amount** |
|---|---|---|
| Acrylates/C10-30 Alkyl | Acrylates/C10-30 Alkyl | 0.1 |
| Acrylate Crosspolymer | Acrylate Crosspolymer | |
| Water, distilled | Water (Aqua) | Ad 100 |
| Sodium Hydroxide 10% solution | Aqua, Sodium Hydroxide | 0.13 |
| Sodium Anisate | Sodium Anisate | 0.3 |
| Glyceryl Undecylenate | Glyceryl Undecylenate | 0.1 |
| Glycerin | Glycerin | 4.0 |
| DOWSIL^{™} 2501 | Bis-PEG-18 Methyl Ether | 2.0 |
| Cosmetic Wax | Dimethyl Silane | |
| Aloe Vera | Aloe Vera | 0.6 |
| Propylene Glycol | Propylene Glycol | 0.3 |
| Witch Hazel Extract | Hamamelis Virginiana (Witch Hazel) Extract | 0.7 |
| C12-15 Alkyl Benzoate | C12-15 Alkyl Benzoate | 2.7 |
| Polysorbate-20 | Polysorbate-20 | 0.5 |
| DOWSIL^{™} 9040 Silicone Elastomer Blend | Cyclopentasiloxane (and) Dimethicone Crosspolymer | 3.5 |
| XIAMETER^{™} PMX-1184 Silicone Fluid | Dimethicone (and) Trisiloxane | 5.8 |
| Perfume oil PO1, PO2, PO3, PO4 or PO5 | Perfume | 0.3 |
| DOWSIL^{™} 9509 Silicone Elastomer Suspension | Dimethicone / Vinyl Dimethicone Crosspolymer (and) C12-14 Pareth-12 | 2.0 |
| Phytic Acid | Phytic Acid | 0.2 |
| Salicylic Acid | Salicylic Acid | 0.5 |
| 1,2-heptanediol/ 2,3-heptanediol blend (96:4 w/w%) | - | 0.5 |
| 1,2-octanediol/2,3-octanediol blend (98:2 w/w%) | - | 0.5 |

**Table 75: Biotic Acne Control Gel (not in accordance with the present invention)**

| **Ingredients** | **INCI** | **Amount** |
|---|---|---|
| Water, distilled | Water (Aqua) | ad 100 |
| SymSave^{®} H | Hydroxyacetophenone | 0.5 |
| Carbopol^{®} Ultrez 21 Polymer | Acrylates/C10-30 Alkyl Acrylate Crossploymer | 0.5 |
| Solubilizer | PEG-40 Hydrogenated Castor Oil, Trideceth-9, Propylene Glycol, Aqua | 2.5 |
| SymClariol^{®} | Decylene Glycol | 0.5 |
| SymMollient^{®} W/S | Trideceth-9, PEG-5 Isononanoate, Aqua | 1.0 |
| Hydrolite^{®}- 5 green | Pentylene Glycol | 5.0 |
| Dragosantol^{®} | Bisabolol | 0.05 |
| Farnesol | Farnesol | 0.3 |
| Ethanol | Alcohol | 5.0 |
| Flowerpone^{®} Lavender | PEG-40 Hydrogenated Castor Oil, Trideceth-9, Pentylene Glycol, Lavandula Angustifolia Flower Extract, Propylene Glycol, Bisabolol | 0.5 |
| SymReboot^{™} L19 | Maltodextrin, Lactobacillus Ferment | 0.5 |
| Sodium Hydroxide 10% solution | Aqua, Sodium Hydroxide | 1.1 |
| Colour | Colour | 0.2 |
| Perfume oil PO1, PO2, PO3, PO4 or PO5 | Perfume | 0.3 |
| Diazolidinyl Urea | Diazolidinyl Urea | 0.1 |
| Capryloyl glycine | Capryloyl glycine | 0.1 |
| 1,2-heptanediol | 1,2-heptanediol | 0.4 |
| 2,3-heptanediol | 2,3-heptanediol | 0.02 |
| 1,2-octanediol | 1,2-octanediol | 0.6 |
| 2,3-octanediol | 2,3-octanediol | 0.01 |

**Table 76: Anti-wrinkle gel (not in accordance with the present invention)**

| **Ingredients** | **INCI** | **Amount** |
|---|---|---|
| EMT-10 | Hydroxyethyl acrylate/sodium acryloyldimethyl taurate copolymer | 1.2 |
| CC | Dicaprylyl Carbonate | 2.0 |
| SpecKare^{™} 3GF | Glyceryl linoleate,Glyceryl oleate,Glyceryl linolenate | 1.0 |
| EH | Ethylhexyl Ethylhexanoate | 2.0 |
| CDM3526 | C26-28 alkyl dimethicone | 0.2 |
| SpecKareTM VEA | Tocopheryl Acetate | 0.2 |
| Glycerin | Glycerin | 8.0 |
| SpecThem^{®} XTG200 | Xanthan Gum | 0.18 |
| SpecThem^{®} SCB21 | Acrylates/c10-30 alkyl acrylate crosspolymer | 0.15 |
| SpecKareTM ALLA | Allantoin | 0.1 |
| H-200 | Glyceryl Polyacrylate | 6.0 |
| Water, distilled | Water (Aqua) | ad 100 |
| SpecPure^{®} PC1(1%Liquid) | Poria cocos sclerotium extract | 2.0 |
| SpecKareTM HAL(1 %Liquid) | Sodium Hyaluronate | 5.0 |
| EDTA-2Na | Disodium EDTA | 0.1 |
| SpecKareTM NMF50 | Betaine | 2.0 |
| Triethanolamine | Triethanolamine | 0.13 |
| NK2 | Dipotassium Glycyrrhizinate | 0.1 |
| Phenylpropanol | Phenylpropanol | 0.8 |
| Perfume oil PO1, PO2, PO3, PO4 or PO5 | Perfume | 0.1 |
| SpecPed^{®} Co-De BPS | Polyglyceryl-10 tristearate, Caprylic/capric triglyceride, Butylene glycol, Glycerin, Hexylene glycol, Carnosine, Lecithin, Hydroxylated lecithin, Cholesterol, Palmitoyl tripeptide-5, Acetyl tetrapeptide-5, Palmitoyl pentapeptide-4, Aqua | 5.0 |
| 1,2-heptanediol | 1,2-heptanediol | 0.3 |
| 2,3-heptanediol | 2,3-heptanediol | 0.01 |
| 1,2-octanediol | 1,2-octanediol | 0.4 |
| 2,3-octanediol | 2,3-octanediol | 0.01 |

**Table 77: Cleansing Micellar Ge (not in accordance with the present invention)I**

| **Ingredients** | **INCI** | **Amount** |
|---|---|---|
| Water, distilled | Water (Aqua) | ad 100 |
| Carbopol^{®} ETD 2050 Polymer | Carbomer | 0.7 |
| Hydroviton^{®} PLUS 2290 | Aqua, Pentylene glycol, Glycerin Fructose, Urea, Citric acid, Sodium hydroxide, Maltose Sodium pca, Sodium chloride Sodium lactate, Trehalose Allantoin, Sodium hyaluronate Glucose | 2.0 |
| SymOcide^{®} PS | Phenoxyethanol, | 1.0 |
| | Decylene glycol, | |
| | 1,2-hexanediol | |
| Sodium Hydroxide 10% solution | Aqua, Sodium Hydroxide | 3.28 |
| SymMollient^{®} W/S | Trideceth-9, PEG-5 Isononanoate, Aqua | 1.0 |
| SymSol^{®} PF-3 | Aqua, Pentylene glycol, Sodium lauryl sulfoacetate, Sodium oleoyl sarcosinate, Sodium chloride, Sodium oleate | 3.0 |
| Perfume oil PO1, PO2, PO3, PO4 or PO5 | Perfume | 1.0 |
| SymVital^{®} AR | Zingiber officinale (ginger) root extract | 0.2 |
| Extrapone^{®} Seaweed | Aqua | 1.0 |
| | Butylene glycol | |
| | Fucus vesiculosus extract | |
| Actipone^{®} Dandelion Juice (Organic) GW | Glycerin | 1.0 |
| | Taraxacum officinale juice | |
| | Aqua | |
| Colour I | Colour | 0.35 |
| Colour II | Colour | 0.20 |
| Caprylhydroxamic Acid | Caprylhydroxamic Acid | 0.2 |
| Phenylpropanol | Phenylpropanol | 0.2 |
| Salicylic Acid | Salicylic Acid | 0.2 |
| 1,2-heptanediol | 1,2-heptanediol | 1.5 |
| 2,3-heptanediol | 2,3-heptanediol | 0.02 |
| 1,2-octanediol | 1,2-octanediol | 0.4 |
| 2,3-octanediol | 2,3-octanediol | 0.01 |

**Table 78: Clear Beard Gel (not in accordance with the present invention)**

| **Ingredients** | **INCI** | **Amount** |
|---|---|---|
| Aqua/Water | Aqua | ad 100 |
| Edeta^{®} BD | Disodium EDTA | 0.10 |
| Glycerin | Glycerin | 3.00 |
| SymOcide^{®} PH | Phenoxyethanol, Hydroxyacetophenone, Caprylyl glycol, Aqua | 0.50 |
| Keltrol^{®} CG-T | Xanthan gum | 0.10 |
| Aristoflex^{®} AVC | Ammonium, Acryloyoldimethyltaurate/vp Copolymer | 2.00 |
| KeraSym^{™} Shield | Pentylene glycol, Aqua,Glycerin, Triticum vulgare bran extract, 1,2-Hexanediol, Capryₗyl glycol | 1.00 |
| Actipone^{®} Arctic Cloudberry GW | Glycerin, Aqua, Rubus chamaemorus seedextract | 0.50 |
| Hydroviton^{®} PLUS 2290 | Aqua, Pentylene glycol, Glycerin, Fructose, Urea, Citric acid Sodium hydroxide, Maltose Sodium pca, Sodium chloride Sodium lactate, Trehalose Allantoin, Sodium hyaluronate Glucose | 1.00 |
| Propylene Glycol | Propylene glycol | 1.50 |
| Sodium Hydroxide 10% aqueous solution | Aqua, Sodium hydroxide | 0.15 |
| SymSol^{®} PF-3 | Aqua, Pentylene glycol Sodium lauryl sulfoacetate Sodium oleoyl sarcosinate Sodium chloride, Sodium oleate | 3.00 |
| Perfume oil PO1, PO2, PO3, PO4 or PO5 | Perfume | 0.40 |
| Hydrolite^{®} 5 | Pentylene glycol | 3.76 |
| Ethanol 96 % | Alcohol | 18.00 |
| Symlite G8 | Glyceryl Caprylate | 0.2 |
| Frescolat^{®} MGA | Menthone Glycerin Acetal | 0.25 |
| SymCalmin^{®} | Pentylene glycol, Butylene glycol Hydroxyphenyl propamidobenzoic acid | 0.50 |
| SymDeo B125 | 2-Methyl 5-Cyclohexylpentanol | 0.1 |
| 1,2-heptanediol | 1,2-heptanediol | 1.2 |
| 2,3-heptanediol | 2,3-heptanediol | 0.03 |
| 1,2-octanediol | 1,2-octanediol | 0.4 |
| 2,3-octanediol | 2,3-octanediol | 0.01 |

**Table 79: Sprayable Anti Cellulite Gel (not in accordance with the present invention)**

| **Ingredients** | **INCI** | **Amount** |
|---|---|---|
| Aqua/Water | Aqua | ad 100 |
| Stabileze QM | PVM / Ma Decadiene Crosspolymer | 0.25 |
| Sodium Hydroxide 10% aqueous solution | Aqua Sodium hydroxide | 0.4 |
| Coffein pure | Caffeine | 0.5 |
| Extrapone^{®} Horse Chestnut | Propylene Glycol, Water (Aqua), Aesculus Hippocastanum (Horse Chestnut) Seed Extract, Glucose, Lactic Acid | 1.0 |
| Hydrolite^{®} 5 | Pentylene Glycol | 3.0 |
| 1,3 Butylene Glycol | Butylene Glycol | 5.0 |
| Biotive^{®} Esculin Sesquihydrate | Esculin | 0.3 |
| Preservative | Methylparaben, | 0.3 |
| Ethanol 96 % | Alcohol Denat, | 10.0 |
| Solubilizer | PEG-40 Hydrogenated Castor Oil, Trideceth-9, Water (Aqua) | 0.5 |
| Perfume oil PO1, PO2, PO3, PO4 or PO5 | Perfume | 0.2 |
| Benzyl Salicylate | Benzyl Salicylate | 0.1 |
| Chlorphenesin | Chlorphenesin | 0.1 |
| 1,2-heptanediol | 1,2-heptanediol | 1.0 |
| 2,3-heptanediol | 2,3-heptanediol | 0.03 |
| 1,2-octanediol | 1,2-octanediol | 0.5 |
| 2,3-octanediol | 2,3-octanediol | 0.01 |

**Table 80: Aloe Vera Gel (not in accordance with the present invention)**

| **Ingredients** | **INCI** | **Amount** |
|---|---|---|
| Aqua/Water | Aqua | ad 100 |
| Carbopol^{®} Ultrez 10 Polymer | Carbomer | 0.45 |
| Glycerin 99,5 % | Glycerin | 5.0 |
| Sodium Hydroxide 10% aqueous solution | Aqua Sodium hydroxide | 0.9 |
| Extrapone^{®} Aloe Vera P | Propylene glycol Aqua, Aloe barbadensis leaf juice powder | 2.0 |
| SymGlucan^{®} | Aqua, Glycerin, 1,2-Hexanediol Caprylyl glycol, Beta-glucan | 1.0 |
| SymOcide^{®} PS | Phenoxyethanol, Decylene glycol 1,2-Hexanediol | 0.8 |
| Hydrolite^{®} 5 | Pentylene glycol | 3.0 |
| Perfume oil PO1, PO2, PO3, PO4 or PO5 | Perfume | 0.2 |
| Solubilizer | Peg-40 hydrogenated castor oil Trideceth-9, Propylene glycol Aqua | 2.5 |
| Butylene Glycol | Butylene glycol | 1.0 |
| Chlorhexidine Digluconate | Chlorhexidine Digluconate | 0.1 |
| Glyceryl Laurate | Glyceryl Laurate | 0.1 |
| 1,2-heptanediol | 1,2-heptanediol | 1.5 |
| 2,3-heptanediol | 2,3-heptanediol | 0.1 |
| 1,2-octanediol | 1,2-octanediol | 0.2 |
| 2,3-octanediol | 2,3-octanediol | 0.005 |

**Table 81: Antidandruff Shampoo (not in accordance with the present invention)**

| **Ingredients** | **INCI** | **Amount** |
|---|---|---|
| Water, distilled | Water (Aqua) | ad 100 |
| Polymer JR 400 | Polyquaternium-10 | 0.4 |
| Texapon^{®} NSO IS | Sodium Laureth Sulfate | 40.0 |
| Farnesol | Farnesol | 0.1 |
| Dehyton K | Cocamidopropyl Betaine | 8.0 |
| Plantacare 2000 UP | Decyl Glucoside | 2.0 |
| Sodium Methylparaben | Sodium Methylparaben | 0.3 |
| Citric Acid 10 % solution | Citric Acid | 0.8 |
| Frescolat^{®} X-cool | Menthyl Ethylamido Oxalate | 0.5 |
| SymTriol^{®} | Caprylyl Glycol, 1,2-Hexanediol, Methylbenzyl Alcohol | 0.8 |
| Crinipan^{®} AD | Climbazole | 0.5 |
| SymMollient^{®} W/S | Trideceth-9, PEG-5 Isononanoate, Water (Aqua) | 2.0 |
| Tinogard^{®} Q | Tri(tetramethylhydroxypiperodonol) Citrate | 0.02 |
| Sodium Chloride | Sodium Chloride | 0.5 |
| Colour I | Colour | 0.3 |
| Colour II | Colour | 0.1 |
| Perfume oil PO1, PO2, PO3, PO4 or PO5 | Perfume | 0.4 |
| Octopirox | Octopirox | 0.1 |
| 1,2-heptanediol | 1,2-heptanediol | 0.4 |
| 2,3-heptanediol | 2,3-heptanediol | 0.05 |
| 1,2-octanediol | 1,2-octanediol | 0.2 |
| 2,3-octanediol | 2,3-octanediol | 0.01 |

**Table 82: Hair Conditioner, rinse-off (not in accordance with the present invention)**

| **Ingredients** | **INCI** | **Amount** |
|---|---|---|
| Lanette O | Cetearyl Alcohol | 4.0 |
| Mineral Oil | Paraffinum Liquidum | 2.0 |
| Genamin KDM-P | Behentrimonium Chloride | 1.0 |
| Dow Corning 556 Fluid | Phenyl Trimethicone | 1.0 |
| Neo Heliopan^{®} BB | Benzophenone - 3 | 0.1 |
| Crinipan^{®} AD | Climbazole | 0.1 |
| Water, distilled | Water (Aqua) | ad 100 |
| Glycerin 99,5 P. | Glycerin | 6.0 |
| Preservative | Phenoxyethanol, Methylparaben, Ethylparaben, Butylparaben, Propylparaben, Isobutylparaben | 0.8 |
| Extrapone Bamboo P | Propylene Glycol, Water (Aqua), Butylene Glycol, Bambusa Vulgaris Shoot Extract | 0.5 |
| Sodium Hydroxide 10% solution | Aqua, Sodium Hydroxide | 0.4 |
| Colour I | Colour | 0.6 |
| Colour II | Colour | 0.3 |
| Perfume oil PO1, PO2, PO3, PO4 or PO5 | Perfume | 0.6 |
| Methylchloroisothiazolinone | Methylchloroisothiazolinone | 0.1 |
| Glyceryl Laurate | Glyceryl Laurate | 0.1 |
| 1,2-heptanediol | 1,2-heptanediol | 0.9 |
| 2,3-heptanediol | 2,3-heptanediol | 0.02 |
| 1,2-octanediol | 1,2-octanediol | 0.4 |
| 2,3-octanediol | 2,3-octanediol | 0.01 |

**Table 83: Air freshener (A/F spray water based) (not in accordance with the present invention)**

| **No** | **Ingredients** | **Amount** |
|---|---|---|
| 1 | Fragrance | 4 |
| 2 | Ethanol | 8 |
| 3 | PEG-40 Hydrogenated Castor Oil | 5 |
| 4 | 1,2-heptanediol / 2,3-heptanediol blend (ratio 99:1) | 1 |
| 5 | Hydroxyacetophenone | 0.5 |
| 6 | Aqua | add to 100 |

### Colorless liquid, pH neutral

**Table 84: All purpose cleaner (not in accordance with the present invention)**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 1,2-heptanediol / 2,3-heptanediol blend (ratio 98:2) | 1 |
| 6 | Phenoxyethanol | 0.5 |
| 7 | Aqua | add to 100 |

### Colorless liquid, pH 7

**Table 85: All purpose cleaner (not in accordance with the present invention)**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 2,3-octanediol | 0.1 |
| 6 | 1,2-heptanediol | 0.9 |
| 7 | Aqua | add to 100 |

### Colorless liquid, pH 7

**Table 86: APC alkaline (not in accordance with the present invention)**

| **No** | **Ingredients** | **Amount** |
|---|---|---|
| 1 | Coco-Glycoside | 1 |
| 2 | Benzalkonium Chloride | 0.2 |
| 3 | Sodiumbicarbonat | 0.1 |
| 4 | Tri-sodiumcitrate-dihydrate | 0.1 |
| 5 | Methylglycinediacetic acid | 0.1 |
| 6 | 1,2-Benzisothiazol-3(2H)-on | 0.1 |
| 7 | Fragrance | 0.1 |
| 8 | 1,2-heptanediol | 0.5 |
| 9 | Hydroxyacetophenone | 0.3 |
| 10 | Aqua | add to 100 |

### Colorless liquid, pH 9

**Table 87: Cleaner, liquid, citric acid (not in accordance with the present invention)**

| **No** | **Ingredients** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol | 0.5 |
| 5 | Fragrance | 0.3 |
| 6 | Aqua | add 100 |
| 7 | Sodium Benzoate | 0.3 |
| 8 | Potassium Sorbate | 0.2 |

### Liquid of low viscosity, pH 2

**Table 88: Cleaner, liquid, citric acid (not in accordance with the present invention)**

| **No** | **Ingredient** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol/2,3 heptanediol 90 : 10 | 0.5 |
| 5 | Fragrance | 0.3 |
| 6 | Caprylhydroxamic Acid | 0.1 |
| 7 | Aqua | add 100 |

### Liquid of low viscosity, pH 2

**Table 89: Detergent liquid light duty (not in accordance with the present invention)**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-octanediol | 0.1 |
| 9 | 1,2-heptanediol | 0.9 |
| 10 | Fragrance | 0.5 |
| 11 | Aqua | add to 100 |
| 12 | Hydroxyacetophenone | 0.2 |

### Liquid, pH 7.3

**Table 90: Detergent liquid light duty (not in accordance with the present invention)**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-heptanediol | 0.1 |
| 9 | 1,2-heptanediol | 0.9 |
| 10 | Fragrance | 0.5 |
| 11 | Aqua | add to 100 |

### Liquid, pH 7.3

**Table 91: Dishwash liquid manual (not in accordance with the present invention)**

| **No** | **Ingredients** | **Amount** |
|---|---|---|
| 1 | Sodium Laureth Sulfate | add 100 |
| 2 | Ethanol | 2.9 |
| 3 | lauramine oxide | 7.7 |
| 4 | propylene glycol | 1.9 |
| 5 | Phenoxyethanol | 0.1 |
| 6 | Benzisothiazolinone, Methylisothiazolinone, Laurylamine Dipropylenediamine | 0.1 |
| 7 | 1,2-heptanediol | 0.5 |
| 8 | sodium chloride | 3.85 |
| 9 | Fragrance | 0.3 |
| 9 | Benzyl Alcohol | 0.2 |

### Colorless liquid, pH 8.5

**Table 92: Fabric softener (not in accordance with the present invention)**

| **No** | **Ingredients** | **Amount** |
|---|---|---|
| 1 | Di-(Talg Carboxyethyl) Hydroxyethyl methylammonium-methosulfat | 5.5 |
| 2 | Simethicone | 0.3 |
| 3 | 2-octyl-2H-isothiazol-3-one | 0.1 |
| 4 | 1,2-heptanediol | 0.5 |
| 5 | Fragrance | 0.2 |
| 6 | Aqua | add 100 |
| 7 | Phenoxyethanol | 0.2 |

### Liquid, pH 3

**Table 93: Fabric softener concentrate, encapsulated (not in accordance with the present invention)**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 1,2-heptanediol | 0.5 |
| 8 | Fragrance | 0.6 |
| 9 | Aqua | add 100 |
| 10 | Ethylhexylglycerin | 0.2 |

### White liquid, pH 2.5

**Table 94: Fabric softener concentrate, encapsulated (not in accordance with the present invention)**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 2,3-heptanediol | 0.05 |
| 8 | 1,2-heptanediol | 0.5 |
| 9 | Fragrance | 0.6 |
| 10 | Aqua | add 100 |
| 11 | Glyceryl Caprylate | 0.1 |

### White liquid, pH 2.5

**Table 95: Hand soap, liquid (not in accordance with the present invention)**

| **No** | **Ingredients** | **Amount** |
|---|---|---|
| 1 | Sodium Laureth Sulfate | 18 |
| 2 | Cocoamidopropylbetaine | 6 |
| 3 | Cocamide DEA | 3 |
| 4 | citric acid | 0.5 |
| 5 | sodium chloride | 1.9 |
| 6 | Glycerol | 2 |
| 7 | Fragrance | 0.2 |
| 8 | 1,2-heptanediol / 2,3-heptanediol blend (ratio 99:1) | 0.25 |
| 9 | Aqua | add 100 |
| 10 | Propanediol Caprylate | 0.3 |

### Slightly colored liquid, pH 6

**Table 96: Rim block gel (not in accordance with the present invention)**

| **No** | **Ingredients** | **Amount** |
|---|---|---|
| 1 | Sodium Laureth Sulfate | 11 |
| 2 | Trideceth-9 | 2 |
| 3 | Xanthan gum | 2 |
| 4 | Hydroxyethylcellulose | 0.5 |
| 5 | Citric Acid | 0.4 |
| 6 | Ethanol | 6 |
| 7 | Fragrance | 5 |
| 8 | 1,2-heptanediol | 0.5 |
| 9 | Aqua | add 100 |
| 10 | Sodium Benzoate | 0.2 |
| 11 | Potassium Sorbate | 0.2 |

### Colorless paste/gel, pH = 4.5

**Table 97: Scent Lotion with capsules (not in accordance with the present invention)**

| **No** | **Ingredients** | **Amount** |
|---|---|---|
| 1 | microcrystalline cellulose/cellulose gum | 1 |
| 2 | PEG-40 hydrogenated Castor oil/ Trideceth-9 | 0.5 |
| 3 | encapsulated perfume oil | 1 |
| 4 | 2-octyl-2H-isothiazol-3-one | 0.1 |
| 5 | Fragrance | 5 |
| 6 | 1,2-heptanediol | 1 |
| 7 | Aqua | add 100 |
| 8 | 1,2-octanediol / 2,3-octanediol belnd (ratio 99:1) | 0.2 |

### White emulsion, pH neutral

**Table 98: Cleaner, liquid, lactic acid (not in accordance with the present invention)**

| **No** | **Ingredients** | **Amount** |
|---|---|---|
| 1 | Sodium Laureth Sulfate | 1.8 |
| 2 | Trideceth-9 | 2.1 |
| 3 | Xanthan gum | 0.35 |
| 4 | Lactic Acid | 2,8 |
| 5 | 1,2-heptanediol | 1 |
| 6 | Fragrance | 0.3 |
| 7 | Aqua | add 100 |
| 8 | Sodium Benzoate | 0.2 |
| 9 | Potassium Sorbate | 0.4 |

### Colorless liquid, pH 2,0

**Table 99: Cleaner, liquid, citric acid (not in accordance with the present invention)**

| **No** | **Ingredients** | **Amount** |
|---|---|---|
| 1 | Sulfonic acid, C 13-17-sec-Alkyl-, Sodium salt | 1 |
| 2 | Xanthan gum | 0-3 |
| 3 | citric acid | 5 |
| 4 | 1,2heptanediol | 0.25 |
| 5 | Fragrance | 0.3 |
| 6 | Aqua | add 100 |
| 7 | Anisic Acid | 0.1 |

Liquid, pH 2

## Claims

1. A composition, comprising or consisting of
(a1) an effective amount of 2,3-octanediol;
(b1) an effective amount of 4-hydroxyacetophenone; and
(c1) optionally at least one active substance for a food, a cosmetic or pharmaceutical composition or a homecare product and/or additive.

2. A composition, comprising or consisting of
(a2) an effective amount of a mixture comprising 1,2-octanediol and 2,3-octanediol;
(b2) an effective amount of 4-hydroxyacetophenone; and
(c2) optionally at least one active substance for a food, a cosmetic or pharmaceutical composition or a homecare product and/or additive.

3. Cosmetic or pharmaceutical composition or homecare product according to claim 2, wherein the mixture comprising 1,2-octanediol and 2,3-octanediol comprises the 1,2-octanediol and the 2,3-octanediol in a ratio in a range of 50 : 50 to 99.9 : 0.1 (w/w), preferably in a ratio in a range of 75 : 25 to 99 : 1 (w/w), more preferred in a ratio in a range of 80 : 20 to 98 : 2 (w/w) or still more preferred in a ratio of 90 : 10 to 95 : 5 (w/w).

4. Composition according to any one of claims 1 to 3, further comprising at least one antimicrobial component, selected from the group consisting of Caprylhydroxamic Acid, o-Cymen-5-ol, Isopropylparaben, Capryloyl Glycine, Phenylpropanol, Tropolone, PCA Ethyl Cocoyl Arginate, 2-Methyl 5-Cyclohexylpentanol, Phenoxyethanol, Disodium EDTA, Methylparaben and its salts, Sodium Benzoate, Benzyl Alcohol, Potassium Sorbate, Benzyl Salicylate, Propylparaben, Sodium Methylparaben, Methylchloroisothiazolinone, Methylisothiazolinone, Ethylhexylglycerin, Butylparaben, Ethylparaben, Sodium Propylparaben, DMDM Hydantoin, Dehydroacetic Acid, Salicylic Acid, Chlorphenesin, Isobutylparaben, Sodium Ethylparaben, Diazolidinyl Urea, Farnesol, Bisabolol, Glyceryl Caprylate, Sodium Phytate or Phytic Acid, Sodium Levulinate or Levulinic Acid and esters and/or ketals thereof, Chlorhexidine, Glyceryl Laurate, Anisic Acid and its salts, Chlorhexidine Digluconate, TEA-salicylate, Phenethyl Alcohol, Glyceryl Caprate, Sorbitan Caprylate, Tetrasodium Glutamate Diacetate, Trisodium Ethylenediamine Disuccinate, Hydroxyethoxyphenyl Butanone, Hydroxyethoxyphenyl Butanol, Itaconic Acid, Octopirox, Propanediol Caprylate, Climbazole, Undecylenoyl Glycine, Thymol, Lactic Acid, Sodium Lactate, 1,3-propanediol, 1,2-pentanediol, 1,2-hexanediol, 1,2-heptanediol, 1,2-nonanediol, 1,2-decanediol, 2,3-pentanediol, 2,3-hexanediol, 2,3-heptanediol, 2,3-nonanediol, and mixtures of two or more the aforesaid antimicrobial components.

5. Composition according to any one of claims 1 to 4, further comprising at least one antimicrobial agent, which is different to the antimicrobial component as claimed in claim 4, selected from the group consisting of 2-benzylheptanol, alkyl (C 12-22) trimethyl ammonium bromide and chloride, ascorbic acid and salts therof, benzalkonium bromide, benzalkonium chloride, benzalkonium saccharinate, benzethonium chloride, Benzoic Acid, camphor, cetylpyridinium chloride, chlorhexidine diacetate, chlorhexidine dihydrochloride, chlorocresol, chloroxylenol, Cyclohexylglycerin, Dimethyl phenylbutanol, Dimethyl phenylpropanol, ethanol, ethyl lauroyl arginate HCl, Ethyl Lauroyl Arginate Laurate, eucalyptol, gluconic acid and salts thereof, glycerin, hexamidine, hexamidine diisethionate, Hexylglycerin, lodopropynyl Butylcarbamate, jasmol, lauryl alcohol, mannitol, menthol, methyl salicylate, Octenidine HCl, polyarginine, Polyglyceryl-10 Laurate, polyglyceryl-2 caprate, Polyglyceryl-3 caprylate, polylysine, Salvia Officinalis (Sage) Oil, silver chloride, silver citrate, sodium caproyl lactylate, Sodium Caproyl/Lauroyl Lactylate, sodium lauroyl lactylate, Sorbic Acid, sorbitol, Tetraselmis extract, triclocarban, triclosan, Triethyl citrate, Xylityl Sesquicaprylate, zinc citrate, zinc pyrithione, Zinc ricinoleate, and mixtures of two or more of the aforesaid antimicrobial agents.

6. Composition according to any one of claims 1 to 5, wherein the at least one active substance for a food, cosmetic or pharmaceutical composition or homecare product and/or additive is selected from the group consisting of agents against ageing of the skin, antioxidants, chelating agents, emulsifiers, surfactants, green and synthetic polymers, skin-cooling agents, rheology additives, oils, fragrances or perfume oils, polyols, and mixtures of two or more of the afore-mentioned substances.

7. Composition according to any one of claims 1 to 6, comprising the 2,3-octanediol or the mixture comprising 1,2-octanediol and 2,3-octanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, most particularly in an amounf of 0.5 to 1.0 % by weight, based on the total weight of the composition or a final food, cosmetic or pharmaceutical preparation or homecare product;
and/or
cosmetic or pharmaceutical composition or homecare product, comprising the 2,3-octanediol or the 2,3-octanediol of the mixture comprising 1,2-octanediol and 2,3-octanediol in an amount of 0.001 to 15.0 % by weight, particularly in an amount of 0.01 to 10.0 % by weight, more particularly in an amount of 0.1 to 5.0 % by weight, still more preferred in an amount of 0.3 to 3.0 % by weight, and most particularly in an amount of 0.5 to 1.0 % by weight, based on the total weight of the composition or or a final food, cosmetic or pharmaceutical preparation or homecare product;
preferably in an amount of 0.001 to 0.5 % by weight, more preferably in an amount of 0.005 to 0.1 % by weight and most preferably in an amount of 0.01 to 0.075 % by weight, based on the total weight of the composition or a final food, cosmetic or pharmaceutical preparation or homecare product.

8. Composition according to any one of claims 1 to 7, comprising the 4-hydroxyacetophenone in an amount of 0.01 to 25.0 % by weight, particularly in an amount of 0.02 to 15.0 % by weight, most particularly in an amount of 0.05 to 6.0 % by weight, based on the total weight of the composition.

9. Food, cosmetic or pharmaceutical preparation comprising a composition according to any one of claims 1 to 8.

10. The cosmetic or pharmaceutical preparation according to claim 9, wherein the cosmetic or pharmaceutical preparation is
i. in the form of a dispersion comprising the oil component in an amount of ≥ 1% by weight, in particular in an amount of ≥ 3 % by weight, in particular, preferably in the form of an emulsion, in particular a O/W emulsion, a W/O emulsion, a multiple emulsion, a hydrodispersion gel, a balm, a multiple emulsion of the water-in-oil type (W/O/W) or of the oil-in-water type (O/W/O), **PIT** emulsion, Pickering emulsion, a micro-emulsion, a liquid, a lotion, a suspension, a milk, an ointment, a paste, a gel, a cream based, an oil based or a liposome-based formulation;
ii. in the form of a liquid surfactant formulation;
iii. in the form of a solid surfactant formulation;
iv. in the form of an aqueous or aqueous/alcoholic, in particular aqueous/ethanolic solution, or an aqueous/glycolic solution; in particular, wherein the cosmetic or pharmaceutical preparation is a cold formulation composition.

11. Use of the composition according to any one of claims 1 to 8 for suppressing or inhibiting microorganism growth in food, a cosmetic or a pharmaceutical preparation or homecare product.

12. The use of the composition according to claim 11 wherein the microorganisms are selected from the group consisting of the genus Pseudomonas, Staphylococcus, Echerichia, Candida, Aspergillus, and combinations thereof, in particular selected from the group consisting of *Pseudomonas aeruginosa, Staphylococcus aureus, Escherichia coli, Candida albicans* und *Aspergillus brasiliensis,* and combinations thereof, more particular *Candida albicans* und *Aspergillus brasiliensis.*

13. Use of 2,3-octanediol or a mixture comprising 1,2-octanediol and 2,3-octanediol for boosting the efficacy of 4-hydroxyacetophenone.

14. Method for suppressing or inhibiting microorganism growth in food, a cosmetic, or a pharmaceutical preparation or a homecare product, comprising the steps of:
- providing a food, a cosmetic or pharmaceutical preparation or homecare product; and
- incorporating therein an effective amount of the composition as defined in any one of claims 1 to 8.

## Patentansprüche

1. Zusammensetzung, die aufweist oder besteht aus:
(a1) einer wirksamen Menge an 2,3-Octandiol;
(b1) einer wirksamen Menge an 4-Hydroxyacetophenon; und
(c1) optional mindestens einem Wirkstoff für ein Lebensmittel, eine kosmetische oder pharmazeutische Zusammensetzung oder ein Homecare-Produkt und/oder einem Zusatzstoff.

2. Zusammensetzung, die aufweist oder besteht aus:
(a2) einer wirksamen Menge einer Mischung, die 1,2-Octandiol und 2,3-Octandiol aufweist;
(b2) einer wirksamen Menge an 4-Hydroxyacetophenon; und
(c2) optional mindestens einem Wirkstoff für ein Lebensmittel, eine kosmetische oder pharmazeutische Zusammensetzung oder ein Homecare-Produkt und/oder einem Zusatzstoff.

3. Kosmetische oder pharmazeutische Zusammensetzung oder Homecare-Produkt nach Anspruch 2, wobei die Mischung, die 1,2-Octandiol und 2,3-Octandiol aufweist, das 1,2-Octandiol und das 2,3-Octandiol in einem Verhältnis im Bereich von 50 : 50 bis 99,9 : 0,1 (Gew./Gew.) enthält, vorzugsweise in einem Verhältnis im Bereich von 75 : 25 bis 99 : 1 (Gew./Gew.), noch bevorzugter in einem Verhältnis im Bereich von 80 : 20 bis 98 : 2 (Gew./Gew.) oder am meisten bevorzugt in einem Verhältnis von 90 : 10 bis 95 : 5 (Gew./Gew.).

4. Zusammensetzung nach einem der Ansprüche 1 bis 3, die ferner mindestens eine antimikrobielle Komponente enthält, die aus der Gruppe ausgewählt ist, die aus Caprylhydroxamsäure, o-Cymen-5-ol, Isopropylparaben, Capryloylglycin, Phenylpropanol, Tropolon, PCA-Ethylcocoylarginat, 2-Methyl-5-cyclohexylpentanol, Phenoxyethanol, Dinatrium-EDTA, Methylparaben und dessen Salze, Natriumbenzoat, Benzylalkohol, Kaliumsorbat, Benzylsalicylat, Propylparaben, Natriummethylparaben, Methylchloroisothiazolinon, Methylisothiazolinon, Ethylhexylglycerin, Butylparaben, Ethylparaben, Natriumpropylparaben, DMDM-Hydantoin, Dehydroessigsäure, Salicylsäure, Chlorphenesin, Isobutylparaben, Natriumethylparaben, Diazolidinylharnstoff, Farnesol, Bisabolol, Glycerylcaprylat, Natriumphytat oder Phytinsäure, Natriumlevulinat oder Levulinsäure und deren Ester und/oder Ketale, Chlorhexidin, Glyceryllaurat, Anissäure und deren Salze, Chlorhexidindigluconat, TEA-Salicylat, Phenethylalkohol, Glycerylcaprat, Sorbitancaprylat, Tetranatriumglutamatdiacetat, Trinatriumethylendiamindisuccinat, Hydroxyethoxyphenylbutanon, Hydroxyethoxyphenylbutanol, Itaconsäure, Octopirox, Propandiolcaprylat, Climbazol, Undecylenoylglycin, Thymol, Milchsäure, Natriumlactat, 1,3-Propandiol, 1,2-Pentandiol, 1,2-Hexandiol, 1,2-Heptandiol, 1,2-Nonandiol, 1,2-Decandiol, 2,3-Pentandiol, 2,3-Hexandiol, 2,3-Heptandiol, 2,3-Nonandiol und Mischungen aus zwei oder mehr der vorgenannten antimikrobiellen Komponenten besteht.

5. Zusammensetzung nach einem der Ansprüche 1 bis 4, die ferner mindestens einen antimikrobiellen Wirkstoff aufweist, der sich von der in Anspruch 4 beanspruchten antimikrobiellen Komponente unterscheidet und aus der Gruppe ausgewählt ist, die aus 2-Benzylheptanol, Alkyl(C 12-22)-trimethylammoniumbromid und -chlorid, Ascorbinsäure und deren Salzen, Benzalkoniumbromid, Benzalkoniumchlorid, Benzalkonium-Saccharinat, Benzethoniumchlorid, Benzoesäure, Kampfer, Cetylpyridiniumchlorid, Chlorhexidin-Diacetat, Chlorhexidin-Dihydrochlorid, Chlorkresol, Chloroxylenol, Cyclohexylglycerin, Dimethylphenylbutanol, Dimethylphenylpropanol, Ethanol, Ethyllauroylarginat-HCl, Ethyllauroylarginatlaurat, Eukalyptol, Gluconsäure und deren Salze, Glycerin, Hexamidin, Hexamidin-Diisethionat, Hexylglycerin, Iodopropynylbutylcarbamat, Jasmol, Laurylalkohol, Mannitol, Menthol, Methylsalicylat, Octenidin-HCl, Polyarginin, Polyglyceryl-10-Laurat, Polyglyceryl-2-Caprat, Polyglyceryl-3-Caprylat, Polylysin, Salvia officinalis (Salbei)-Öl, Silberchlorid, Silbercitrat, Natriumcaproyl-Lactylat, Natriumcaproyl-/Lauroyl-Lactylat, Natriumlauroyl-Lactylat, Sorbinsäure, Sorbit, Tetraselmis-Extrakt, Triclocarban, Triclosan, Triethylcitrat, Xylityl-Sesquicaprylat, Zinkcitrat, Zinkpyrithion, Zinkricinoleat und Mischungen aus zwei oder mehr der vorgenannten antimikrobiellen Wirkstoffe besteht.

6. Zusammensetzung nach einem der Ansprüche 1 bis 5, wobei der mindestens eine Wirkstoff für ein Lebensmittel, eine kosmetische oder pharmazeutische Zusammensetzung oder ein Homecare-Produkt und/oder Zusatzstoff aus der Gruppe ausgewählt ist, die aus Mitteln gegen Hautalterung, Antioxidantien, Chelatbildnern, Emulgatoren, Tensiden, natürlichen und synthetischen Polymeren, hautkühlenden Mitteln, Rheologieadditiven, Ölen, Duftstoffen oder Parfümölen, Polyolen und Mischungen aus zwei oder mehr der vorgenannten Substanzen besteht.

7. Zusammensetzung nach einem der Ansprüche 1 bis 6, die das 2,3-Octandiol oder die Mischung, die 1,2-Octandiol und 2,3-Octandiol aufweist, in einer Menge von 0,001 bis 15,0 Gew.-%, vorzugsweise in einer Menge von 0,01 bis 10,0 Gew.-%, noch bevorzugter in einer Menge von 0,1 bis 5,0 Gew.-%, am meisten bevorzugt in einer Menge von 0,3 bis 3,0 Gew.-%, ganz besonders in einer Menge von 0,5 bis 1,0 Gew.-%, bezogen auf das Gesamtgewicht der Zusammensetzung oder eines fertigen Lebensmittels, eines kosmetischen oder pharmazeutischen Präparats oder eines Homecare-Produkts aufweist; und/oder
kosmetische oder pharmazeutische Zusammensetzung oder ein Homecare-Produkt, die das 2,3-Octandiol oder das 2,3-Octandiol der Mischung, die 1,2-Octandiol und 2,3-Octandiol aufweist, in einer Menge von 0,001 bis 15,0 Gew.- %, insbesondere in einer Menge von 0,01 bis 10,0 Gew.-%, ganz besonders in einer Menge von 0,1 bis 5,0 Gew.-%, noch bevorzugter in einer Menge von 0,3 bis 3,0 Gew.-% und am meisten bevorzugt in einer Menge von 0,5 bis 1,0 Gew.- %, bezogen auf das Gesamtgewicht der Zusammensetzung oder eines fertigen Lebensmittels, eines kosmetischen oder pharmazeutischen Präparats oder eines Homecare-Produkts aufweist;
vorzugsweise in einer Menge von 0,001 bis 0,5 Gew.-%, noch bevorzugter in einer Menge von 0,005 bis 0,1 Gew.-% und am meisten bevorzugt in einer Menge von 0,01 bis 0,075 Gew.-%, bezogen auf das Gesamtgewicht der Zusammensetzung oder eines fertigen Lebensmittels, eines kosmetischen oder pharmazeutischen Präparats oder eines Homecare-Produkts.

8. Zusammensetzung nach einem der Ansprüche 1 bis 7, die das 4-Hydroxyacetophenon in einer Menge von 0,01 bis 25,0 Gew.-%, insbesondere in einer Menge von 0,02 bis 15,0 Gew.-%, am meisten insbesondere in einer Menge von 0,05 bis 6,0 Gew.-%, bezogen auf das Gesamtgewicht der Zusammensetzung aufweist.

9. Lebensmittel, kosmetisches oder pharmazeutisches Präparat, das eine Zusammensetzung nach einem der Ansprüche 1 bis 8 aufweist.

10. Kosmetisches oder pharmazeutisches Präparat nach Anspruch 9, wobei das kosmetische oder pharmazeutische Präparat vorliegt
i. in der Form einer Dispersion, die die Ölkomponente in einer Menge von ≥ 1 Gew.-%, insbesondere in einer Menge von ≥ 3 Gew.-%, aufweist, insbesondere vorzugsweise in Form einer Emulsion, insbesondere einer O/W-Emulsion, einer W/O-Emulsion, einer Mehrfachemulsion, eines Hydrodispersionsgels, eines Balsams, einer Mehrfachemulsion vom Typ Wasser-in-Öl (W/O/W) oder vom Typ Öl-in-Wasser (O/W/O), einer PIT-Emulsion, einer Pickering-Emulsion, einer Mikroemulsion, einer Flüssigkeit, einer Lotion, einer Suspension, einer Milch, einer Salbe, einer Paste, eines Gels, einer Formulierung auf Cremebasis, auf Ölbasis oder auf Liposomenbasis vorliegt;
ii. in der Form einer flüssigen Tensidformulierung;
iii. in der Form einer festen Tensidformulierung;
iv. in der Form einer wässrigen oder wässrig-alkoholischen, insbesondere wässrig-ethanolischen Lösung oder einer wässrig-glykolischen Lösung; wobei das kosmetische oder pharmazeutische Präparat insbesondere eine Kaltformulierungszusammensetzung ist.

11. Verwendung der Zusammensetzung nach einem der Ansprüche 1 bis 8 zur Unterdrückung oder Hemmung des Wachstums von Mikroorganismen in einem Lebensmittel, einem kosmetischen oder pharmazeutischen Präparat oder einem Homecare-Produkt.

12. Verwendung der Zusammensetzung nach Anspruch 11, wobei die Mikroorganismen aus der Gruppe ausgewählt sind, die aus der Gattung Pseudomonas, Staphylococcus, Escherichia, Candida, Aspergillus und Kombinationen davon besteht, insbesondere aus der Gruppe ausgewählt sind, die aus Pseudomonas aeruginosa, Staphylococcus aureus, Escherichia coli, Candida albicans und Aspergillus brasiliensis und Kombinationen davon, ganz besonders Candida albicans und Aspergillus brasiliensis besteht.

13. Verwendung von 2,3-Octandiol oder einer Mischung, die 1,2-Octandiol und 2,3-Octandiol aufweist, zur Steigerung der Wirksamkeit von 4-Hydroxyacetophenon.

14. Verfahren zur Unterdrückung oder Hemmung des Wachstums von Mikroorganismen in einem Lebensmittel, einem Kosmetikum, einem pharmazeutischen Präparat oder einem Homecare-Produkt, das die Schritte aufweist:
- Bereitstellen eines Lebensmittels, eines Kosmetikums oder einea pharmazeutischen Präparat oder eines Homecare-Produkts; und
- Einbringen darin einer wirksamen Menge der in einem der Ansprüche 1 bis 8 definierten Zusammensetzung.

## Revendications

1. Composition comprenant ou constituée de
(a1) une quantité efficace de 2,3-octanediol ;
(b1) une quantité efficace de 4-hydroxyacétophénone ; et
(c1) éventuellement au moins une substance active destinée à une composition alimentaire, cosmétique ou pharmaceutique ou à un produit d'entretien ménager et/ou un additif.

2. Composition comprenant ou constituée de
(a2) une quantité efficace d'un mélange comprenant du 1,2-octanediol et du 2,3-octanediol ;
(b2) une quantité efficace de 4-hydroxyacétophénone ; et
(c2) éventuellement au moins une substance active destinée à une composition alimentaire, cosmétique ou pharmaceutique ou à un produit d'entretien ménager et/ou un additif.

3. Composition cosmétique ou pharmaceutique ou produit d'entretien ménager selon la revendication 2, dans laquelle le mélange comprenant du 1,2-octanediol et du 2,3-octanediol comprend le 1,2-octanediol et le 2,3-octanediol dans un rapport compris dans une plage de 50 : 50 à 99,9 : 0,1 (p/p), de préférence dans un rapport compris entre 75 : 25 et 99 : 1 (p/p), plus préférablement dans un rapport compris entre 80 : 20 et 98 : 2 (p/p) ou, de préférence encore, dans un rapport compris entre 90 : 10 et 95 : 5 (p/p).

4. Composition selon l'une quelconque des revendications 1 à 3, comprenant en outre au moins un composant antimicrobien, choisi dans le groupe constitué par l'acide caprylhydroxamique, l'o-cymène-5-ol, l'isopropylparabène, la capryloylglycine, le phénylpropanol, la tropolone, l'arginate d'éthylcocoyle de PCA, le 2-méthyl-5-cyclohexylpentanol, le phénoxyéthanol, l'EDTA disodique, le méthylparabène et ses sels, le benzoate de sodium, l'alcool benzylique, le sorbate de potassium, le salicylate de benzyle, le propylparabène, le méthylparabène de sodium, la méthylchloroisothiazolinone, la méthylisothiazolinone, l'éthylhexylglycérine, le butylparabène, l'éthylparabène, le propylparabène de sodium, l'hydantoïne DMDM, l'acide déhydroacétique, l'acide salicylique, la chlorphénésine, l'isobutylparabène, l'éthylparabène de sodium, la diazolidinylurée, le farnésol, le bisabolol, le caprylate de glycéryle, le phytate de sodium ou l'acide phytique, le lévulinate de sodium ou l'acide lévulinique et leurs esters et/ou cétals, la chlorhexidine, le laurate de glycéryle, l'acide anisique et ses sels, le digluconate de chlorhexidine, le salicylate de TEA, l'alcool phénéthylique, le caprate de glycéryle, le caprylate de sorbitane, le diacétate de glutamate tétrasodique, le disuccinate d'éthylènediamine trisodique, l'hydroxyéthoxyphénylbutanone, l'hydroxyéthoxyphénylbutanol, l'acide itaconique, l'octopirox, le caprylate de propanediol, le climbazole, l'undécylénoylglycine, le thymol, l'acide lactique, le lactate de sodium, le 1,3-propanediol, le 1,2-pentanediol, le 1,2-hexanediol, le 1,2-heptanediol, le 1,2-nonanediol, le 1,2-décanediol, le 2,3-pentanediol, le 2,3-hexanediol, le 2,3-heptanediol, le 2,3-nonanediol, et des mélanges de deux ou plusieurs des composants antimicrobiens susmentionnés.

5. Composition selon l'une quelconque des revendications 1 à 4, comprenant en outre au moins un agent antimicrobien, différent du composant antimicrobien revendiqué dans la revendication 4, choisi dans le groupe constitué par le 2-benzylheptanol, le bromure et le chlorure d'alkyl (C 12-22) triméthylammonium, l'acide ascorbique et ses sels, le bromure de benzalkonium, le chlorure de benzalkonium, le saccharinate de benzalkonium, le chlorure de benzéthonium, l'acide benzoïque, le camphre, le chlorure de cétylpyridinium, le diacétate de chlorhexidine, le dichlorhydrate de chlorhexidine, le chlorocrésol, le chloroxylénol, la cyclohexylglycérine, le diméthylphénylbutanol, le diméthylphénylpropanol, l'éthanol, le chlorhydrate d'arginate de lauroyle d'éthyle, le laurate d'arginate de lauroyle d'éthyle, l'eucalyptol, l'acide gluconique et ses sels, la glycérine, l'hexamidine, le diiséthionate d'hexamidine, l'hexylglycérine, le butylcarbamate d'iodopropynyle, le jasmol, l'alcool laurylique, le mannitol, le menthol, le salicylate de méthyle, le chlorhydrate d'octénidine, le polyarginine, le laurate de polyglycéryle-10, le caprate de polyglycéryle-2, le caprylate de polyglycéryle-3, la polylysine, l'huile de Salvia officinalis (sauge), le chlorure d'argent, le citrate d'argent, le lactylate de caproyle de sodium, le lactylate de caproyle/lauroyle de sodium, le lactylate de lauroyle de sodium, l'acide sorbique, le sorbitol, l'extrait de Tetraselmis, le triclocarban, le triclosan, le citrate de triéthyle, le sesquicaprylate de xylityle, le citrate de zinc, le pyrithione de zinc, le ricinoléate de zinc, et des mélanges de deux ou plusieurs des agents antimicrobiens susmentionnés.

6. Composition selon l'une quelconque des revendications 1 à 5, dans laquelle l'au moins une substance active destinée à une composition alimentaire, cosmétique ou pharmaceutique ou à un produit d'entretien ménager et/ou à un additif est choisie parmi le groupe constitué d'agents anti-vieillissement cutané, d'antioxydants, d'agents chélatants, d'émulsifiants, de tensioactifs, de polymères naturels et synthétiques, d'agents rafraîchissants pour la peau, d'additifs rhéologiques, d'huiles, de parfums ou d'huiles parfumées, des polyols, et des mélanges de deux ou plusieurs des substances susmentionnées.

7. Composition selon l'une quelconque des revendications 1 à 6, comprenant le 2,3-octanediol ou le mélange comprenant du 1,2-octanediol et du 2,3-octanediol en une quantité de 0,001 à 15,0 % en poids, de préférence en une quantité de 0,01 à 10,0 % en poids, de préférence en une quantité de 0,1 à 5,0 % en poids, encore de préférence en une quantité de 0,3 à 3,0 % en poids, et plus particulièrement en une quantité de 0,5 à 1,0 % en poids, par rapport au poids total de la composition ou d'une préparation alimentaire, cosmétique ou pharmaceutique finale ou d'un produit d'entretien ménager final ;
et/ou
une composition cosmétique ou pharmaceutique ou un produit d'entretien ménager, comprenant le 2,3-octanediol ou le 2,3-octanediol du mélange de 1,2-octanediol et de 2,3-octanediol, en une quantité comprise entre 0,001 et 15,0 % en poids, en particulier en une quantité comprise entre 0,01 et 10,0 % en poids, plus particulièrement en une quantité comprise entre 0,1 et 5,0 % en poids, encore davantage de préférence en une quantité comprise entre 0,3 et 3,0 % en poids, et tout particulièrement en une quantité comprise entre 0,5 et 1,0 % en poids, par rapport au poids total de la composition ou d'une préparation alimentaire, cosmétique ou pharmaceutique finale ou d'un produit d'entretien ménager final ;
de préférence en une quantité comprise entre 0,001 et 0,5 % en poids, plus préférablement en une quantité comprise entre 0,005 et 0,1 % en poids et le plus préférablement en une quantité comprise entre 0,01 et 0,075 % en poids, par rapport au poids total de la composition ou d'une préparation alimentaire, cosmétique ou pharmaceutique finale ou d'un produit d'entretien ménager final.

8. Composition selon l'une quelconque des revendications 1 à 7, comprenant de la 4-hydroxyacétophénone en une quantité comprise entre 0,01 et 25,0 % en poids, en particulier en une quantité comprise entre 0,02 et 15,0 % en poids, et plus particulièrement en une quantité comprise entre 0,05 et 6,0 % en poids, par rapport au poids total de la composition.

9. Préparation alimentaire, cosmétique ou pharmaceutique comprenant une composition selon l'une quelconque des revendications 1 à 8.

10. Préparation cosmétique ou pharmaceutique selon la revendication 9, dans laquelle la préparation cosmétique ou pharmaceutique est
i. sous la forme d'une dispersion comprenant le composant huileux en une quantité > 1 % en poids, en particulier en une quantité > 3 % en poids, en particulier, de préférence sous la forme d'une émulsion, en particulier d'une émulsion E/H, d'une émulsion H/E, d'une émulsion multiple, d'un gel hydrodispersé, d'un baume, d'une émulsion multiple de type eau-dans-huile (E/H/E) ou de type huile-dans-eau (H/E/H), d'une émulsion PIT, d'une émulsion de Pickering, d'une micro-émulsion, d'un liquide, d'une lotion, d'une suspension, d'un lait, d'une pommade, d'une pâte, d'un gel, d'une formulation à base de crème, à base d'huile ou à base de liposomes ;
ii. sous la forme d'une formulation tensioactive liquide ;
iii. sous la forme d'une formulation tensioactive solide ;
iv. sous la forme d'une solution aqueuse ou aqueuse/alcoolique, en particulier une solution aqueuse/éthanolique ou une solution aqueuse/glycolique ; en particulier, dans laquelle la préparation cosmétique ou pharmaceutique est une composition de formulation à froid.

11. Utilisation de la composition selon l'une quelconque des revendications 1 à 8 pour supprimer ou inhiber la croissance de micro-organismes dans un aliment, une préparation cosmétique ou pharmaceutique ou un produit d'entretien ménager.

12. Utilisation de la composition selon la revendication 11, dans laquelle les micro-organismes sont choisis parmi le groupe constitué du genre Pseudomonas, Staphylococcus, Escherichia, Candida, Aspergillus, et de leurs combinaisons, en particulier choisis parmi le groupe constitué de Pseudomonas aeruginosa, Staphylococcus aureus, Escherichia coli, Candida albicans et Aspergillus brasiliensis, et de leurs combinaisons, plus particulièrement Candida albicans et Aspergillus brasiliensis.

13. Utilisation du 2,3-octanediol ou d'un mélange comprenant du 1,2-octanediol et du 2,3-octanediol pour renforcer l'efficacité de la 4-hydroxyacétophénone.

14. Procédé destiné à supprimer ou à inhiber la croissance de micro-organismes dans un aliment, un produit cosmétique, une préparation pharmaceutique ou un produit d'entretien ménager, comprenant les étapes suivantes :
- fournir un produit alimentaire, un produit cosmétique, une préparation pharmaceutique ou un produit d'entretien ménager ; et
- y incorporer une quantité efficace de la composition telle que définie dans l'une quelconque des revendications 1 à 8.
